(19) Europäisches Patentamt
European Patent Office
Office européen des brevets

(11) Veröffentlichungsnummer: **0 376 072 B1**

(12) **EUROPÄISCHE PATENTSCHRIFT**

(45) Veröffentlichungstag der Patentschrift: **16.02.94**

(21) Anmeldenummer: **89123112.8**

(22) Anmeldetag: **14.12.89**

(51) Int. Cl.$^5$: **C07C 229/48**, A01N 37/10, C07C 229/50, C07C 255/46, A01N 37/14, C07C 237/30, A01N 37/18, C07D 209/48, A01N 37/32, C07C 237/40, C07C 215/44

(54) **Fungizide und herbizide Mittel sowie substituierte 2-Cyclohexen-1-yl-amin-Derivate und deren Herstellung.**

(30) Priorität: **27.12.88 DE 3843978**
**17.08.89 DE 3927115**

(43) Veröffentlichungstag der Anmeldung:
**04.07.90 Patentblatt 90/27**

(45) Bekanntmachung des Hinweises auf die Patenterteilung:
**16.02.94 Patentblatt 94/07**

(84) Benannte Vertragsstaaten:
**BE CH DE ES FR GB IT LI NL**

(56) Entgegenhaltungen:
**EP-A- 0 128 006**

(73) Patentinhaber: **BAYER AG**

**D-51368 Leverkusen(DE)**

(72) Erfinder: **Kunisch, Franz, Dr.**
**Zum Hahnenberg 20**
**D-5068 Odenthal-Gloebusch(DE)**
Erfinder: **Babczinski, Peter, Dr.**
**In der Lohrenbeck 11**
**D-5600 Wuppertal 1(DE)**
Erfinder: **Arlt, Prof. Dr.**
**Rybnikerstrasse 2**
**D-5000 Köln 80(DE)**
Erfinder: **Santel, Hans-Joachim, Dr.**
**Grünstrasse 9a**
**D-5090 Leverkusen(DE)**
Erfinder: **Schmidt, Robert R., Dr.**
**Im Waldwinkel 110**
**D-5060 Bergisch-Gladbach 2(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

**JOURNAL OF THE AMERICAN CHEMICAL SOCIETY, Band 100, Nr. 10, 10. Mai 1978, seiten 3182-3189, American Chemical Society; L.E. OVERMAN et al.: "Diels-Alder reactions between trans-1-N-acylamino-1,3-dienes and methyl acrylate. A correlelation between diene photoelectron ionization potentials and reactivity, stereoselectivity, and regioselectivity"**

**J. ORG. CHEM., Band 43, Nr. 7, 1978, Seiten 1448-1455, American Chemical Society; S.P. SINGER et al.: "Synthesis of dl-gabaculine utilizing direct allylic amination as the key step"**

**Gazz.Chim.Ital.Band 57,(1927),Seiten 292-299**

Erfinder: **Brandes, Wilhelm, Dr.**
**Eichendorffstrasse 3**
**D-5653 Leichlingen(DE)**
Erfinder: **Strang, Harry, Dr.**
**Heiderweg 53**
**D-4000 Düsseldorf 31(DE)**

## Beschreibung

Die vorliegende Erfindung betrifft die Verwendung von teilweise bekannten substituierten 2-Cyclohexen-1-yl-amin-Derivaten in Schädlingsbekämpfungsmitteln, vor allem als Fungizide und Herbizide sowie neue substituierte 2-Cyclohexen-1-yl-amin-Derivate und mehrere Verfahren zu ihrer Herstellung.

Es ist bereits bekannt, daß bestimmte Tetrahydrophthalimide, wie beispielsweise cis-N-[-(Trichlormethyl)thio]-4-cyclohexen-1,2-dicarboimid, fungizide Eigenschaften aufweisen (vgl. z.B. Science, (Washington) 115, 84 (1952); US 2 553 770).

Die Wirkung dieser Verbindungen ist jedoch insbesondere bei niedrigen Aufwandmengen und -konzentrationen nicht immer in allen Anwendungsbereichen völlig zufriedenstellend.

Es ist weiter bereits bekannt, daß 2-Cycloalkenylamin-Derivate und ihre Salze, wie beispielsweise N-2-Cyclohexen-1-yl-2,2-dimethyl-propionamid fungizide Eigenschaften besitzen (vgl. EP-OS 0 128 006).

Außerdem sind N-Benzyl-2-cyclohexen-1-yl-amin-Derivate, wie beispielsweise N-4-Methylbenzyl-2-cyclohexenylamin als Zwischenprodukte für die Herstellung von substituierten Benzylcycloalkenylharnstoff-Derivaten beschrieben (vgl. EP-OS 0 113 028).

Weiterhin sind substituierte 2-Cyclohexen-1-yl-amin-Derivate, wie beispielsweise 6-Carbomethoxy-2-cyclohexen-1-yl-amin als Zusatzstoffe für Schmieröle bekannt (vgl. US-PS 4 582 618).

Ferner sind substituierte 2-Cyclohexen-1-yl-amin-Derivate, wie beispielsweise 5-Carbomethoxy-2-cyclohexen-1-yl-(4,4′-dimethoxybenzhydrylamin) als Zwischenprodukte für die Synthese des Naturstoffes (±)-Gabaculin beschrieben (vgl. J. Org. Chem. 44, 3451-3457, 1979).

Außerdem wird die Diels-Alder Reaktion verschiedener N-Acylamino-1,3-butadiene mit Acrylsäuremethylester zu den entsprechenden N-Acylamino-2-cyclohexen-Derivaten, wie beispielsweise 6-Carbomethoxy-2-cyclohexen-1-yl-ethylcarbamat, quantitativ untersucht (vgl. J. Am. Chem. Soc., 100, 3182-9, 1978).

Weiterhin ist die Synthese von zahlreichen 2-Cyclohexen-1-yl-amin-Derivatenbeschrieben (vgl. J. Med. Chem., 29, 1 - 8, 1986; J. Med. Chem., 24, 788-94, 1981, Gazz. Chim. Ital. 57, 295-7 (1927), J. Am. Chem. Soc., 103, 2816-22, 1981; J. Am. Chem. Soc., 100, 3182-9, 1978; J. Am. Chem. Soc., 100, 5179-85, 1978 und Tetrahedron Lett., 25, 2183-6, 1984, J. Org. Chem., 46, 2833-5, 1981, J. Am. Chem. Soc., 105, 5373-9, 1983), über deren Wirksamkeit im Pflanzenschutz ist jedoch nichts bekannt.

Es wurde nun gefunden, daß die teilweise bekannten substituierten 2-Cyclohexen-1-yl-amin-Derivate der Formel (I)

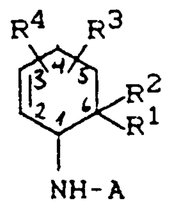

(I)

in welcher

$R^1$ für Wasserstoff, Alkyl oder Halogen steht,

$R^2$ für Formyl, Hydroxyalkyl, Cyano, Nitro oder für einen der Reste $-NHR^5$, $-NR^6R^7$,

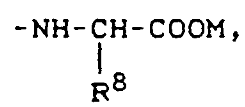

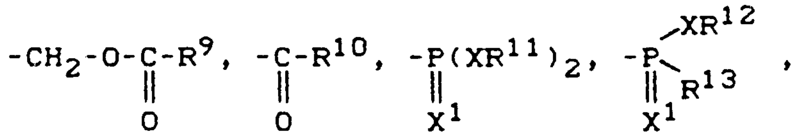

$-S(O)_nR^{14}$ oder $-CH=CH-R^{15}$ steht,

$R^3$ und $R^4$ gleich oder verschieden sind und jeweils für Wasserstoff, Alkyl, Alkenyl, Alkinyl, Alkoxy, Alkenyloxy, Alkinyloxy, für unsubstituiertes oder substituiertes Aryl, für unsubstituiertes oder substituiertes Aralkyl, für unsubstituiertes oder substituiertes Heteroaryl, für unsubstituiertes oder substituiertes Heterocyclylalkyl, Alkoxyalkyloxy, Halogen oder für einen der Reste
-NH-$R^5$, -$NR^6R^7$ oder $S(O)_n$-$R^{14}$ stehen

oder

$R^2$ und $R^3$ gemeinsam für einen der Reste

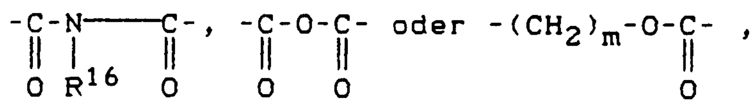

verbrückt über die Positionen 6 und 5, stehen,

oder

$R^3$ und $R^4$ gemeinsam für eine Alkylkette mit 3- oder 4-Kohlenstoffatomen stehen, die über die Positionen 4 und 3 verbunden ist,

$R^5$ für Wasserstoff, Alkyl oder unsubstituiertes oder substituiertes Aryl steht,

$R^6$ für Alkyl oder unsubstituiertes oder substituiertes Aryl steht,

$R^7$ für Alkyl oder unsubstituiertes oder substituiertes Aryl steht,

$R^8$ für Wasserstoff, Alkyl, unsubstituiertes oder substituiertes Aryl oder unsubstituiertes oder substituiertes Aralkyl steht,

$R^9$ für Alkyl oder Alkoxy steht,

$R^{10}$ für Hydroxy, Hydroxyalkyloxy, Halogenalkyloxy, Alkoxy, Alkoxyalkyloxy, unsubstituiertes oder substituiertes Cycloalkyloxy, unsubstituiertes oder substituiertes Aralkyloxy, unsubstituiertes oder substituiertes Aryloxy, unsubstituiertes oder substituiertes Aralkyl, Alkylthio, unsubstituiertes oder substituiertes Arylthio oder für eine Gruppe -OM, -$NHR^5$, -$NR^6R^7$ oder -O-Z-$NR^5R^6$ steht,

$R^{11}$ für Wasserstoff oder Alkyl steht,

$R^{12}$ für Wasserstoff oder Alkyl steht,

$R^{13}$ für Alkyl steht,

$R^{14}$ für Alkyl, Alkoxy, unsubstituiertes oder substituiertes Aryl oder für die Gruppe -OM steht,

$R^{15}$ für Formyl, Cyano oder für die Gruppierung

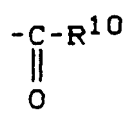

steht,

$R^{16}$ für Wasserstoff, Alkyl oder unsubstituiertes oder substituiertes Aryl steht,

M für Wasserstoff oder für ein Äquivalent eines entsprechenden Alkalimetall-, Erdalkalimetall- oder Ammoniumkations steht,

n für eine Zahl 0, 1 oder 2 steht,

X und $X^1$ gleich oder verschieden sind und für Sauerstoff oder Schwefel stehen,

m für eine Zahl 1 oder 2 steht,

A für Wasserstoff oder eine Aminoschutzgruppe steht und

Z für eine geradkettige oder verzweigte Alkylkette steht,

sowie deren Säureadditions-Salze und Metallsalz-Komplexe starke biologische Eigenschaften aufweisen.

Die Verbindungen der Formel (I) können als geometrische Isomere (E/Z-Isomere) oder Isomerengemische unterschiedlicher Zusammensetzung vorliegen. Die Verwendung sowohl der reinen Isomeren als auch der Isomerengemische werden erfindungsgemäß beansprucht.
Die Verbindungen der Formel (I) enthalten außerdem 1 bis 4 Chiralitätszentren und können somit in verschiedenen Enantiomeren- und Diastereomerengemischen vorliegen, die gegebenenfalls in üblicher Art und Weise getrennt werden können. Die Verwendung sowohl der reinen Enantiomeren und Diastereomeren, als auch die der Gemische werden ebenfalls erfindungsgemäß beansprucht.

Im folgenden wird der Einfachheit halber stets von der Verwendung von Verbindungen der Formel (I) gesprochen, obwohl sowohl die reinen Verbindungen, als auch die Gemische mit unterschiedlichen Anteilen

an isomeren, enantiomeren und diastereomeren Verbindungen gemeint sind.

Überraschenderweise zeigen die teilweise bekannten substituierten 2-Cyclohexen-1-yl-amin-Derivate der Formel (I) sowie deren Säureadditions-Salze und Metallsalz-Komplexe bei entsprechenden Anwendungskonzentrationen bessere fungizide Eigenschaften, als das aus dem Stand der Technik bekannte cis-N-[-(Trichlormethyl)-thio]-4-cyclohexen-1,2-dicarboimid, welches ein konstitutionell ähnlicher Wirkstoff gleicher Wirkungsart ist. Zusätzlich zeigen die teilweise bekannten substituierten 2-Cyclohexen-1-yl-amin-Derivate der Formel (I) bei entsprechenden Anwendungskonzentrationen auch sehr gute herbizide Eigenschaften.

Die erfindungsgemäß zu verwendenden substituierten 2-Cyclohexen-1-yl-amin-Derivate sind durch die Formel (I) allgemein definiert.

Im folgenden bedeutet in den allgemeinen Formeln, falls nicht anders definiert:

Alkyl - geradkettiges oder verzweigtes Alkyl mit 1 bis 8, vorzugsweise 1 bis 6, insbesondere 1 bis 4 Kohlenstoffatomen. Beispielhaft und vorzugsweise seien gegebenenfalls substituiertes Methyl, Ethyl, n.- und i.-Propyl, n-, i-, s- und t-Butyl, genannt.

Alkenyl sowie der Alkenylteil von gegebenenfalls substituiertem Alkenyloxy - geradkettiges oder verzweigtes Alkenyl mit 2 bis 8, vorzugsweise 2 bis 6, insbesondere 2 bis 4 Kohlenstoffatomen. Beispielhaft und vorzugsweise seien gegebenenfalls substituiertes Ethenyl, Propenyl-(1), Propenyl-(2) und Butenyl-(3) genannt.

Alkinyl sowie der Alkinylteil von gegebenenfalls substituiertem Alkinyloxy - geradkettiges oder verzweigtes Alkinyl mit 2 bis 8, vorzugsweise 2 bis 6, insbesondere 2 bis 4 Kohlenstoffatomen. Beispielhaft und vorzugsweise seien gegebenenfalls substituiertes Ethinyl, Propinyl-(1), Propinyl-(2) und Butinyl-(3) genannt.

Alkoxy - unsubstituiertes oder substituiertes, geradkettiges oder verzweigtes Alkoxy mit 1 bis 8, vorzugsweise 1 bis 6, insbesondere 1 bis 4 Kohlenstoffatomen. Beispielhaft und vorzugsweise seien gegebenenfalls substituiertes Methoxy, Ethoxy, n.- und i.-Propoxy und n-, i-, s- und t-Butoxy genannt.

Aryl - vorzugsweise unsubstituiertes oder substituiertes Phenyl oder Naphtyl, insbesondere Phenyl.

Aralkyl und Aralkoxy - unsubstituiertes oder im Arylteil und/oder Alkylteil substituiertes Aralkyl bzw. Aralkoxy mit vorzugsweise 6 oder 10, insbesondere 6 Kohlenstoffatomen im Arylteil (vorzugsweise Phenyl oder Naphtyl, insbesondere Phenyl) und vorzugsweise 1 bis 8, insbesondere 1 bis 6 Kohlenstoffatomen im Alkylteil, wobei der Alkylteil geradkettig oder verzweigt sein kann. Beispielhaft und vorzugsweise seien gegebenenfalls substituiertes Benzyl und Phenylethyl bzw. Benzyloxy und Phenylethyloxy genannt.

Unsubstituierte oder substituierte heterocyclische Reste bedeuten in den allgemeinen Formeln heteroparaffinische, heteroaromatische und heteroolefinische 5-6-gliedrige Ringe mit vorzugsweise 1 bis 3, insbesondere 1 oder 2 gleichen oder verschiedenen Heteroatomen. Als Heteroatome stehen Sauerstoff, Schwefel oder Stickstoff. Beispielhaft und vorzugsweise seien Pyrrolidinyl, Piperidinyl, Furyl, Thienyl, Pyrazolyl, Imidazolyl, 1,2,3- und 1,2,4-Triazolyl, Oxazolyl, Isoxazolyl, Thiazolyl, Isothiazolyl, 1,3,4- und 1,2,4-Oxadiazolyl, Azepinyl, Pyrrolyl, Isopyrrolyl, Pyridyl, Pyridazinyl, Pyrimidinyl, Pyrazinyl und 1,2,3-, 1,2,4-, 1,2,5- und 1,3,4-Thiadiazolyl genannt.

Halogen bedeutet in den allgemeinen Formeln vorzugsweise Fluor, Chlor, Brom und Jod, insbesondere Fluor, Chlor und Brom und besonders bevorzugt Fluor und Chlor.

Die gegebenenfalls substituierten Reste der allgemeinen Formeln können einen oder mehrere, vorzugsweise 1 bis 3, insbesondere 1 oder 2 gleiche oder verschiedene Substituenten tragen. Als Substituenten seien beispielhaft und vorzugsweise aufgeführt:

Alkyl mit vorzugsweise 1 bis 4, insbesondere 1 oder 2 Kohlenstoffatomen, wie Methyl, Ethyl, n.- und i.-Propyl und n.-, i.- und t.-Butyl; Alkoxy mit vorzugsweise 1 bis 4, insbesondere 1 oder 2 Kohlenstoffatomen, wie Methoxy, Ethoxy, n.- und i.-Propyloxy und n.-, i.-, sec.- und t.-Butyloxy; Alkylthio mit vorzugsweise 1 bis 4, insbesondere 1 oder 2 Kohlenstoffatomen, wie Methylthio, Ethylthio, n.- und i.-Propylthio und n.-, i.-, sec.- und t.-Butylthio; Halogenalkyl, Halogenalkoxy und Halogenalkylthio mit vorzugsweise 1 bis 4, insbesondere 1 oder 2 Kohlenstoffatomen und vorzugsweise 1 bis 9, insbesondere 1 bis 5 Halogenatomen, wobei die Halogenatome gleich oder verschieden sind und als Halogenatome, vorzugsweise Fluor, Chlor oder Brom, insbesondere Fluor stehen, wie Trifluormethyl, Trifluormethoxy und Trifluormethylthio; Hydroxy; Halogen, vorzugsweise Fluor, Chlor, Brom und Jod, insbesondere Fluor, Chlor und Brom; Cyano; Nitro; Amino; Dialkylamino mit vorzugsweise 1 bis 4, insbesondere 1 oder 2 Kohlenstoffatomen je Alkylgruppe, wie Methyl-ethyl-amino, und Methyl-n.-butylamino; Carboxyl.

Bevorzugt werden die Verbindungen der Formel (I) verwendet, in welcher

$R^1$ für Wasserstoff, geradkettiges oder verzweigtes Alkyl mit 1 bis 6 Kohlenstoffatomen oder Fluor, Chlor oder Brom steht,

$R^2$ für Formyl, geradkettiges oder verzweigtes Hydroxyalkyl mit 1 bis 8 Kohlenstoffatomen im Alkylteil, Cyano, Nitro oder für einen der Reste $-NHR^5$, $NR^6R^7$,

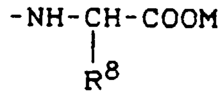, 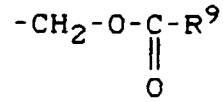,

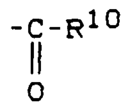, 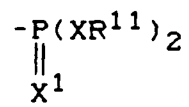, 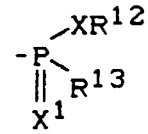,

$-S(O)_nR^{14}$ oder $-CH=CH-R^{15}$ steht,

$R^3$ und $R^4$ gleich oder verschieden sind und jeweils für Wasserstoff, jeweils geradkettiges oder verzweigtes Alkyl oder Alkoxy mit 1 bis 8 Kohlenstoffatomen, jeweils geradkettiges oder verzweigtes Alkenyl, Alkinyl, Alkenyloxy oder Alkinyloxy mit jeweils 2 bis 8 Kohlenstoffatomen, Alkoxyalkyloxy mit jeweils 1 bis 8 Kohlenstoffatomen in den einzelnen Alkylteilen, für jeweils im Arylteil unsubstituiertes oder einfach bis fünffach, gleich oder verschieden substituiertes Aryl oder Aralkyl mit jeweils 6 bis 10 Kohlenstoffatomen im Arylteil und gegebenenfalls 1 bis 4 Kohlenstoffatomen im Alkylteil stehen, wobei als Arylsubstituenten in Frage kommen: Halogen, Nitro, Cyano, Amino, $C_1$-$C_4$-Alkyl, $C_1$-$C_4$-Alkoxy oder $C_1$-$C_4$-Alkylthio, Halogen-($C_1$-$C_4$)-alkyl, Halogen-($C_1$-$C_4$)-alkoxy, Halogen-($C_1$-$C_4$)-alkylthio mit jeweils 1 bis 9 gleichen oder verschiedenen Halogenatomen und Di-($C_1$-$C_4$)-alkylamino, weiterhin für einen unsubstituierten oder einfach bis fünffach, gleich oder verschieden substituierten heterocyclischen 5- oder 6-gliedrigen Ring, der 1 bis 3 Sauerstoff-, Schwefel- und/oder Stickstoffatome als weitere Heteroatome enthalten kann oder für unsubstituiertes oder einfach bis fünffach, gleich oder verschieden substituiertes Heterocyclylalkyl mit einem 5- oder 6-gliedrigen Ring, der 1 bis 3 Sauerstoff-, Schwefel- und/oder Stickstoffatome als weitere Heteroatome und 1 oder 2 Kohlenstoffatome im Alkylteil enthalten kann, steht, wobei als Substituenten für den Heterocyclus jeweils infrage kommen: Halogen, Nitro, Cyano, Amino, $C_1$-$C_4$-Alkyl, $C_1$-$C_4$-Alkoxy, $C_1$-$C_4$-Alkylthio, Halogen-($C_1$-$C_4$)-alkyl, Halogen-($C_1$-$C_4$)-alkoxy, Halogen-($C_1$-$C_4$)-alkylthio mit jeweils 1 bis 9 gleichen oder verschiedenen Halogenatomen und Di-($C_1$-$C_4$)-alkylamino,
weiterhin für Fluor, Chlor, Brom oder für einen Rest
$-NH-R^5$, $-NR^6R^7$ oder $-S(O)_n-R^{14}$ stehen oder

$R^2$ und $R^3$ gemeinsam für einen der Reste

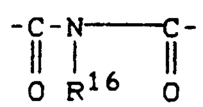, 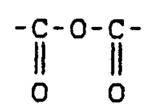 oder 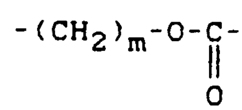,

verbrückt über die Positionen 6 und 5, stehen,

oder

$R^3$ und $R^4$ gemeinsam für eine Alkylkette mit 3- oder 4-Kohlenstoffatomen stehen, die über die Positionen 4 und 3 verbunden ist,

$R^5$ für Wasserstoff, geradkettiges oder verzweigtes Alkyl mit 1 bis 6 Kohlenstoffatomen oder unsubstituiertes oder einfach bis fünffach, gleich oder verschieden substituiertes Aryl mit 6 bis 10 Kohlenstoffatomen steht, wobei als Arylsubstituenten die unter $R^3$ aufgeführten Arylsubstituenten infrage kommen,

$R^6$ für geradkettiges oder verzweigtes Alkyl mit 1 bis 6 Kohlenstoffatomen oder unsubstituiertes oder einfach bis fünffach, gleich oder verschieden substituiertes Aryl mit 6 bis 10 Kohlenstoffatomen steht, wobei als Arylsubstituenten die unter $R^3$ aufgeführten Arylsubstituenten in Frage kommen,

$R^7$ für geradkettiges oder verzweigtes Alkyl mit 1 bis 6 Kohlenstoffatomen oder unsubstituiertes oder einfach bis fünffach, gleich oder verschieden substituiertes Aryl mit 6 bis 10 Kohlenstoffatomen steht, wobei als Arylsubstituenten die unter $R^3$ aufgeführten Arylsubstituenten in Frage kommen

$R^8$ für Wasserstoff, geradkettiges oder verzweigtes Alkyl mit 1 bis 6 Kohlenstoffatomen, für

jeweils im Arylteil unsubstituiertes oder einfach bis fünffach, gleich oder verschieden substituiertes Aryl oder Aralkyl mit jeweils 6 bis 10 Kohlenstoffatomen im Arylteil und gegebenenfalls 1 bis 4 Kohlenstoffatomen im Alkylteil steht, wobei als Arylsubstituenten die unter $R^3$ aufgeführten Arylsubstituenten in Frage kommen,

$R^9$ für jeweils geradkettiges oder verzweigtes Alkyl oder Alkoxy mit 1 bis 6 Kohlenstoffatomen steht,

$R^{10}$ für Hydroxy, geradkettiges oder verzweigtes Hydroxyalkyloxy mit 1 bis 8 Kohlenstoffatomen, geradkettiges oder verzweigtes Halogenalkyloxy mit 1 bis 8 Kohlenstoffatomen und 1 bis 17 gleichen oder verschiedenen Halogenatomen, unsubstituiertes oder einfach bis mehrfach, gleich oder verschieden durch Halogen substituiertes Cycloalkyloxy mit 3 bis 6 Kohlenstoffatomen, jeweils geradkettiges oder verzweigtes Alkoxy oder Alkylthio mit 1 bis 6 Kohlenstoffatomen, geradkettiges oder verzweigtes Alkoxyalkyloxy mit jeweils 1 bis 6 Kohlenstoffatomen im Alkoxy- und Alkylteil, jeweils im Arylteil unsubstituiertes oder einfach bis fünffach, gleich oder verschieden substituiertes Aryloxy, Arylthio, Aralkyl oder Aralkyloxy mit jeweils 6 bis 10 Kohlenstoffatomen im Arylteil und gegebenenfalls 1 bis 8 Kohlenstoffatomen im Alkylteil steht, wobei als Arylsubstituenten die unter $R^3$ aufgeführten Arylsubstituenten infrage kommen oder für eine Gruppe -OM, $-NHR^5$, $-NR^6R^7$ oder $-O-Z-NR^5R^6$ steht,

$R^{11}$ für Wasserstoff oder geradkettiges oder verzweigtes Alkyl mit 1 bis 6 Kohlenstoffatomen steht,

$R^{12}$ für Wasserstoff oder geradkettiges oder verzweigtes Alkyl mit 1 bis 6 Kohlenstoffatomen steht,

$R^{13}$ für geradkettiges oder verzweigtes Alkyl mit 1 bis 6 Kohlenstoffatomen steht,

$R^{14}$ für jeweils geradkettiges oder verzweigtes Alkyl oder Alkoxy mit 1 bis 6 Kohlenstoffatomen, unsubstituiertes oder einfach bis fünffach, gleich oder verschieden substituiertes Aryl mit 6 bis 10 Kohlenstoffatomen steht, wobei als Arylsubstituenten die unter $R^3$ aufgeführten Arylsubstituenten in Frage kommen oder für die Gruppe -OM steht,

$R^{15}$ für Formyl, Cyano oder für die Gruppierung

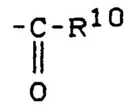

steht,

$R^{16}$ für Wasserstoff, geradkettiges oder verzweigtes Alkyl mit 1 bis 6 Kohlenstoffatomen oder unsubstituiertes oder einfach bis fünffach, gleich oder verschieden substituiertes Aryl mit 6 bis 10 Kohlenstoffatomen steht, wobei als Arylsubstituenten die oben unter $R^3$ aufgeführten Arylsubstituenten in Frage kommen,

m für eine Zahl 1 oder 2 steht und

M für Wasserstoff oder für ein Äquivalent eines entsprechenden Alkalimetall-, Erdalkalimetall- oder Ammoniumkations steht,

n für eine Zahl 0, 1 oder 2 steht,

X und $X^1$ gleich oder verschieden sind und für Sauerstoff oder Schwefel stehen,

A für Wasserstoff oder eine Aminoschutzgruppe steht und

Z für eine geradkettige oder verzweigte Alkylkette mit 1 bis 8 Kohlenstoffatomen steht.

Bevorzugt erfindungsgemäß zu verwendende Verbindungen sind auch Additionsprodukte aus Säuren und denjenigen substituierten 2-Cyclohexen-1-yl-amin-Derivaten der Formel (I) in denen $R^1$, $R^2$, $R^3$ und $R^4$ diejenigen Bedeutungen haben, die im Zusammenhang mit der Beschreibung der erfindungsgemäß zu verwendenden Stoffe bereits vorzugsweise für diese Substituenten genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z.B. Essigsäure, Trifluoressigsäure, Maleinsäure, Bernsteinsäure, Fumarsäure, Weinsäure, Zitronensäure, Salicylsäure, Sorbinsäure und Milchsäure, Ölsäure, Stearinsäure, gegebenenfalls einfach bis mehrfach durch Nitro oder Halogen substituierte Benzoesäure, Gluconsäure, Ascorbinsäure, Äpfelsäure, Sulfamidsäure, Sulfonsäuren, wie z.B. p-Toluolsulfonsäure, 1,5-Naphthalindisulfonsäure und Methansulfonsäure, sowie

Imide, wie z.B. Phthalimid, Saccharin und Thiosaccharin.

Außerdem bevorzugt erfindungsgemäß zu verwendende Verbindungen sind auch Additionsprodukte aus Salzen von Metallen der I., II. und III. Hauptgruppe sowie des Zinns, sowie ferner Salze von Metallen der I., II., VII. und VIII. Nebengruppe des Periodensystems der Elemente und denjenigen substituierten 2-Cyclohexen-1-yl-amin-Derivaten der Formel (I), in denen $R^1$, $R^2$, $R^3$ und $R^4$ die Bedeutungen haben, die bereits im Zusammenhang mit der Beschreibung der erfindungsgemäß zu verwendenden Stoffe der Formel (I) vorzugsweise für diese Substituenten genannt wurden.

Hierbei sind Salze des Kupfers, Zinks, Mangans, Magnesiums, Calciums, Zinns, Eisens, Cobalts und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Besonders bevorzugt werden die Verbindungen der Formel (I) verwendet, in welcher

$R^1$ für Wasserstoff, geradkettiges oder verzweigtes Alkyl mit 1 bis 4 Kohlenstoffatomen oder Fluor, Chlor oder Brom steht,

$R^2$ für Formyl, geradkettiges oder verzweigtes Hydroxyalkyl mit 1 bis 4 Kohlenstoffatomen im Alkylteil, Cyano, Nitro oder für einen der Reste -$NHR^5$, $NR^6R^7$,

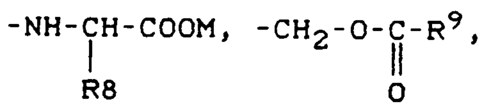

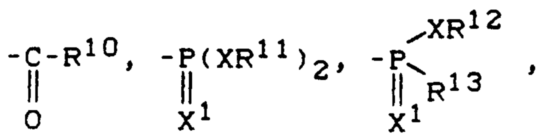

$-S(O)_nR^{14}$ oder $-CH=CH-R^{15}$ steht,

$R^3$ und $R^4$ gleich oder verschieden sind und jeweils für Wasserstoff, jeweils geradkettiges oder verzweigtes Alkyl oder Alkoxy mit 1 bis 6 Kohlenstoffatomen, jeweils geradkettiges oder verzweigtes Alkenyl, Alkinyl, Alkenyloxy oder Alkinyloxy mit jeweils 2 bis 6 Kohlenstoffatomen, Alkoxyalkyloxy mit jeweils 1 bis 6 Kohlenstoffatomen in den einzelnen Alkylteilen, für jeweils im Phenylteil unsubstituiertes oder einfach bis fünffach, gleich oder verschieden substituiertes Phenyl oder Phenylalkyl mit gegebenenfalls 1 oder 2 Kohlenstoffatomen im Alkylteil stehen, wobei als Phenylsubstituenten un Frage kommen: Fluor, Chlor, Brom, Nitro, Cyano, Amino, $C_1$-$C_2$-Alkyl, $C_1$-$C_3$-Alkoxy oder $C_1$-$C_2$-Alkylthio, Halogen-($C_1$-$C_2$)-alkyl, Halogen-($C_1$-$C_2$)-alkoxy und Halogen-($C_1$-$C_2$)-alkylthio mit jeweils 1 bis 5 gleichen oder verschiedenen Fluor- und/oder Chloratomen,

weiterhin für eine unsubstituierte oder einfach bis dreifach, gleich oder verschieden substituierte und gegebenenfalls über eine Methylengruppe gebundene heterocyclische fünf- oder sechsgliedrige Gruppierung aus der Reihe Furyl, Thienyl, Pyrrolyl, Pyrazolyl, Imidazolyl, 1,2,3- oder 1,2,4-Triazolyl, Oxazolyl, Isoxazolyl, 1,2,4- oder 1,3,4-Oxadiazolyl, Thiazolyl, Isothiazolyl, 1,2,3-, 1,2,4-, 1,2,5- oder 1,3,4-Thiadiazolyl, Pyridyl, Pyridazinyl, Pyrimidinyl und Pyrazinyl stehen, wobei als Substituenten für den Heterocyclus jeweils infrage kommen: Fluor, Chlor, Brom, Nitro, Cyano, Amino, $C_1$-$C_2$-Alkyl, $C_1$-$C_2$-Alkoxy, $C_1$-$C_2$-Alkylthio, Halogen-($C_1$-$C_2$)-alkyl, Halogen-($C_1$-$C_2$)-alkoxy, Halogen-($C_1$-$C_2$)-alkylthio mit jeweils 1 bis 5 gleichen oder verschiedenen Fluor- und/oder Chloratomen und Di-($C_1$-$C_2$)-alkylamino,

weiterhin für Fluor, Chlor, Brom oder für einen der Reste

$-NH-R^5$, $-NR^6R^7$ oder $-S(O)_n-R^{14}$ stehen oder

$R^2$ und $R^3$ gemeinsam für einen der Reste

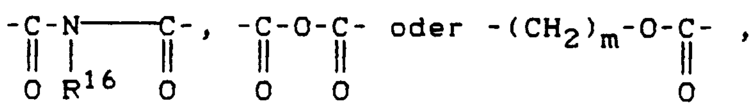

verbrückt über die Positionen 6 und 5, stehen,

oder

$R^3$ und $R^4$ gemeinsam für eine Alkylkette mit 3- oder 4-Kohlenstoffatomen stehen, die über die Positionen 4 und 3 verbunden ist,

$R^5$ für Wasserstoff, geradkettiges oder verzweigtes Alkyl mit 1 bis 4 Kohlenstoffatomen oder unsubstituiertes oder einfach bis fünffach, gleich oder verschieden substituiertes Phenyl steht, wobei als Phenylsubstituenten die unter $R^3$ aufgeführten Phenylsubstituenten infrage kommen,

$R^6$ für geradkettiges oder verzweigtes Alkyl mit 1 bis 4 Kohlenstoffatomen oder unsubstituiertes oder einfach bis fünffach, gleich oder verschieden substituiertes Phenyl steht, wobei als Phenylsubstituenten die unter $R^3$ aufgeführten Phenylsubstituenten infrage kommen,

$R^7$ für geradkettiges oder verzweigtes Alkyl mit 1 bis 4 Kohlenstoffatomen oder unsubstituiertes oder einfach bis fünffach, gleich oder verschieden substituiertes Phenyl steht, wobei als Phenylsubstituenten die unter $R^3$ aufgeführten Phenylsubstituenten infrage kommen,

$R^8$ für Wasserstoff, geradkettiges oder verzweigtes Alkyl mit 1 bis 4 Kohlenstoffatomen, für jeweils im Phenylteil unsubstituiertes oder einfach bis fünffach, gleich oder verschieden substituiertes Phenyl oder Phenylalkyl mit gegebenenfalls 1 oder 2 Kohlenstoffatomen im Alkylteil steht, wobei als Phenylsubstituenten die unter $R^3$ aufgeführten Phenylsubstituenten infrage kommen,

$R^9$ für jeweils geradkettiges oder verzweigtes Alkyl oder Alkoxy mit 1 bis 4 Kohlenstoffatomen steht,

$R^{10}$ für Hydroxy, geradkettiges oder verzweigtes Hydroxyalkyloxy mit 1 bis 6 Kohlenstoffatomen, geradkettiges oder verzweigtes Halogenalkyloxy mit 1 bis 6 Kohlenstoffatomen und 1 bis 13 gleichen oder verschiedenen Halogenatomen, unsubstituiertes oder einfach bis dreifach, gleich oder verschieden durch Fluor, Chlor, Brom substituiertes Cycloalkyloxy mit 3 bis 6 Kohlenstoffatomen, jeweils geradkettiges oder verzweigtes Alkoxy oder Alkylthio mit 1 bis 4 Kohlenstoffatomen, geradkettiges oder verzweigtes Alkoxyalkyloxy mit jeweils 1 bis 4 Kohlenstoffatomen im Alkoxy- oder Alkylteil, jeweils im Phenylteil unsubstituiertes oder einfach bis fünffach, gleich oder verschieden substituiertes Phenyloxy, Phenylthio, Phenylalkyl oder Phenylalkyloxy mit jeweils gegebenenfalls 1 bis 6 Kohlenstoffatomen im Alkylteil steht, wobei als Phenylsubstituenten die unter $R^3$ aufgeführten Phenylsubstituenten in Frage kommen oder für eine Gruppe -OM, -NHR$^5$, -NR$^6$R$^7$ oder -O-Z-NR$^5$R$^6$ steht,

$R^{11}$ für Wasserstoff oder geradkettiges oder verzweigtes Alkyl mit 1 bis 4 Kohlenstoffatomen steht,

$R^{12}$ für Wasserstoff oder geradkettiges oder verzweigtes Alkyl mit 1 bis 4 Kohlenstoffatomen steht,

$R^{13}$ für geradkettiges oder verzweigtes Alkyl mit 1 bis 4 Kohlenstoffatomen steht,

$R^{14}$ für jeweils geradkettiges oder verzweigtes Alkyl oder Alkoxy mit 1 bis 4 Kohlenstoffatomen, unsubstituiertes oder einfach bis fünffach, gleich oder verschieden substituiertes Phenyl steht, wobei als Phenylsubstituenten die unter $R^3$ aufgeführten Phenylsubstituenten infrage kommen oder für die Gruppe -OM steht,

$R^{15}$ für Formyl, Cyano oder für die Gruppierung

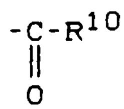

steht,

$R^{16}$ für Wasserstoff, geradkettiges oder verzweigtes Alkyl mit 1 bis 4 Kohlenstoffatomen oder unsubstituiertes oder einfach bis fünffach, gleich oder verschieden substituiertes Phenyl steht, wobei als Phenylsubstituenten die oben unter $R^3$ aufgeführten Phenylsubstituenten in Frage kommen,

m für eine Zahl 1 oder 2 steht und

M für Wasserstoff oder für ein Äquivalent eines entsprechenden Natrium-, Kalium- oder Ammoniumkations steht,

n für eine Zahl 0, 1 oder 2 steht,

X und $X^1$ gleich oder verschieden sind und für Sauerstoff oder Schwefel stehen,
A für Wasserstoff oder eine Aminoschutzgruppe steht und
Z für eine geradkettige oder verzweigte Alkylkette mit 1 bis 6 Kohlenstoffatomen steht.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte Reaktion an anderen Stellen des Moleküls durchgeführt worden ist. Typisch für solche Gruppen sind insbesondere unsubstituierte oder substituierte Acyl-, Aryl- z.B. DNP (2,4-Dinitrophenyl), Aralkoxymethyl- z.B. BOM (N-(benzyloxy)methyl) oder Aralkylgruppen (z.B. Benzyl, 4-Nitrobenzyl, Triphenylmethyl). Da die Aminoschutzgruppen nach der gewünschten Reaktion (oder Reaktionsfolge) entfernt werden, ist ihre Art und Größe im übrigen nicht kritisch; bevorzugt werden jedoch solche mit 1 - 20, insbesondere 1 - 8 Kohlenstoffatomen. Der Ausdruck "Acylgruppe" ist im Zusammenhang mit der vorliegenden Erfindung in weitestem Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie POA (Phenoxyacetyl); Alkoxycarbonyl wie Methoxycarbonyl, Ethoxycarbonyl, 2,2,2-Trichlorethoxycarbonyl, BOC (tert.-Butoxycarbonyl), 2-Iodethoxycarbonyl; Aralkyloxycarbonyl wie CBZ ("Carbobenzoxy") und 4-Methoxybenzyloxycarbonyl. Bevorzugte Aminoschutzgruppen sind Benzyl, Acetyl, Methoxycarbonyl, Allyloxycarbonyl, Trichlorethyloxycarbonyl, (±)-Menthyloxycarbonyl, tert-Butoxycarbonyl und Benzyloxycarbonyl.

Ein weiterer Gegenstand der Anmeldung sind neue substituierte 2-Cyclohexen-1-yl-amin-Derivate der Formel (Ia)

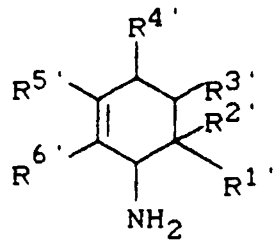

(Ia)

in welcher
$R^{1'}$ für Wasserstoff, Alkyl oder Halogen steht,
$R^{2'}$ für Formyl, Hydroxyalkyl, Cyano, Nitro oder für einen der Reste

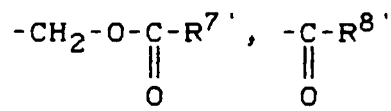

oder $-CH=CH-R^{9'}$ steht,
$R^{3'}$, $R^{4'}$, $R^{5'}$ und $R^{6'}$ gleich oder verschieden sind und jeweils für Wasserstoff, Alkyl, Alkenyl, Alkinyl, Alkoxy, Alkenyloxy, Alkinyloxy, für unsubstituiertes oder substituiertes Aryl, für unsubstituiertes oder substituiertes Aralkyl, für unsubstituiertes oder substituiertes Heteroaryl, für unsubstituiertes oder substituiertes Heterocyclylalkyl, Alkoxyalkyloxy oder für Halogen steht,
wobei mindestens zwei der Reste $R^{3'}$, $R^{4'}$, $R^{5'}$ oder $R^{6'}$ für Wasserstoff stehen,
$R^{7'}$ für Alkyl oder Alkoxy steht,
$R^{8'}$ für Hydroxy, Hydroxyalkyloxy, Halogenalkyloxy, Alkoxy, Alkoxyalkyloxy, unsubstituiertes oder substituiertes Cycloalkyloxy, unsubstituiertes oder substituiertes Aralkyloxy, unsubstituiertes oder substituiertes Aryloxy, unsubstituiertes oder substituiertes Aralkyl, Alkylthio, unsubstituiertes oder substituiertes Arylthio oder für eine Gruppe $-O-Z-NR^{11'}R^{12'}$, $-NHR^{10'}$, $-NR^{11'}R^{12'}$ oder -OM steht,
$R^{9'}$ für Formyl, Cyano oder für die Gruppe

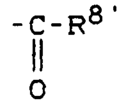

steht,

$R^{10'}$ für Wasserstoff, Alkyl oder unsubstituiertes oder substituiertes Aryl steht,

$R^{11'}$ und $R^{12'}$ gleich oder verschieden sind und jeweils für Alkyl oder unsubstituiertes oder substituiertes Aryl stehen,

Z für eine geradkettige oder verzweigte Alkylkette steht und

M für Wasserstoff oder für ein Äquivalent eines entsprechenden Alkalimetall-, Erdalkalimetall- oder Ammoniumkations steht

oder

$R^{2'}$ und $R^{3'}$ gemeinsam für einen der Reste

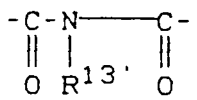 , 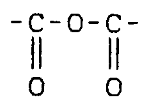 oder 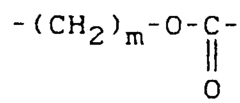 ,

verbrückt über die Positionen 6 und 5, stehen,

worin

$R^{13'}$ für Wasserstoff, Alkyl oder unsubstituiertes oder substituiertes Aryl steht und

m für eine Zahl 1 oder 2 steht,

oder

$R^{4'}$ und $R^{5'}$ gemeinsam für eine Alkylkette mit 3- oder 4-Kohlenstoffatomen stehen, die über die Positionen 4 und 3 verbunden ist,

sowie deren Säureadditions-Salze und Metallsalz-Komplexe ausgenommen die Verbindungen 2-Amino-cyclohex-3-en-carbonsäure und 2-Amino-cyclohex-3-en-carbonsaureethylester.

Die noch nicht bekannten substituierten 2-Cyclohexen-1-yl-amin-Derivate sind durch die Formel (Ia) allgemein definiert.

Bevorzugt werden die Verbindungen der Formel (Ia) in welcher

$R^{1'}$ für Wasserstoff, geradkettiges oder verzweigtes Alkyl mit 1 bis 6 Kohlenstoffatomen oder Fluor, Chlor oder Brom steht,

$R^{2'}$ für Formyl, geradkettiges oder verzweigtes Hydroxyalkyl mit 1 bis 8 Kohlenstoffatomen im Alkylteil, Cyano, Nitro oder für einen der Reste

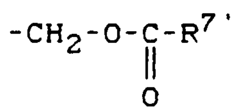 , 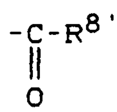

oder $-CH=CH-R^{9'}$ steht,

$R^{3'}$, $R^{4'}$, $R^{5'}$ und $R^{6'}$ gleich oder verschieden sind und jeweils für Wasserstoff, jeweils geradkettiges oder verzweigtes Alkyl oder Alkoxy mit 1 bis 8 Kohlenstoffatomen, jeweils geradkettiges oder verzweigtes Alkenyl, Alkinyl, Alkenyloxy oder Alkinyloxy mit jeweils 2 bis 8 Kohlenstoffatomen, Alkoxyalkyloxy mit jeweils 1 bis 8 Kohlenstoffatomen in den einzelnen Alkylteilen, für jeweils im Arylteil unsubstituiertes oder einfach bis fünffach, gleich oder verschieden substituiertes Aryl oder Aralkyl mit jeweils 6 bis 10 Kohlenstoffatomen im Arylteil und gegebenenfalls 1 bis 4 Kohlenstoffatomen im Alkylteil stehen, wobei als Arylsubstituenten in Frage kommen: Halogen, Nitro, Cyano, Amino, $C_1$-$C_4$-Alkyl, $C_1$-$C_4$-Alkoxy, $C_1$-$C_4$-Alkylthio, Halogen-($C_1$-$C_4$)-alkyl, Halogen-($C_1$-$C_4$)-alkoxy, Halogen-($C_1$-$C_4$)-alkylthio mit jeweils 1 bis 9 gleichen oder verschiedenen Halogenatomen und Di-($C_1$-$C_4$)-alkylamino, weiterhin für eine unsubstituierte oder einfach bis dreifach,

gleich oder verschieden substituierte und gegebenenfalls über eine Methylengruppe gebundene heterocyclische 5- oder 6-gliedrige Gruppierung aus der Reihe Furyl, Thienyl, Pyrrolyl, Pyrazolyl, Imidazolyl, 1,2,3- oder 1,2,4-Triazolyl, Oxazolyl, Isoxazolyl, 1,2,4- oder 1,3,4-Oxadiazolyl, Thiazolyl, Isothiazolyl, 1,2,3-, 1,2,4-, 1,2,5- oder 1,3,4-Thiadiazolyl, Pyridyl, Pyridazinyl, Pyrimidinyl und Pyrazinyl stehen, wobei als Substituenten für den Heterocyclus jeweils infrage kommen: Halogen, Nitro, Cyano, Amino, $C_1$-$C_4$-Alkyl, $C_1$-$C_4$-Alkoxy oder $C_1$-$C_4$-Alkylthio, Halogen-($C_1$-$C_4$)-alkyl, Halogen-($C_1$-$C_4$)-alkoxy oder Halogen-($C_1$-$C_4$)-alkylthio mit jeweils 1 bis 9 gleichen oder verschiedenen Halogenatomen und Di-($C_1$-$C_4$)-alkylamino,

weiterhin für Fluor, Chlor oder Brom stehen,

wobei mindestens zwei der Reste $R^{3'}$, $R^{4'}$, $R^{5'}$ oder $R^{6'}$ für Wasserstoff stehen,

$R^{7'}$ für jeweils geradkettiges oder verzweigtes Alkyl oder Alkoxy mit 1 bis 6 Kohlenstoffatomen steht,

$R^{8'}$ für Hydroxy, geradkettiges oder verzweigtes Hydroxyalkyloxy mit 1 bis 8 Kohlenstoffatomen, geradkettiges oder verzweigtes Halogenalkyloxy mit 1 bis 8 Kohlenstoffatomen und 1 bis 17 gleichen oder verschiedenen Halogenatomen, unsubstituiertes oder einfach bis mehrfach, gleich oder verschieden durch Halogen substituiertes Cycloalkyloxy mit 3 bis 6 Kohlenstoffatomen, jeweils geradkettiges oder verzweigtes Alkoxy oder Alkylthio mit 1 bis 6 Kohlenstoffatomen, geradkettiges oder verzweigtes Alkoxyalkyloxy mit jeweils 1 bis 6 Kohlenstoffatomen im Alkoxy- oder Alkylteil, jeweils im Arylteil unsubstituiertes oder einfach bis fünffach, gleich oder verschieden substituiertes Aryloxy, Arylthio, Aralkyl oder Aralkyloxy mit jeweils 6 bis 10 Kohlenstoffatomen im Arylteil und gegebenenfalls 1 bis 8 Kohlenstoffatomen im Alkylteil steht, wobei als Arylsubstituenten die oben aufgeführten Arylsubstituenten in Frage kommen oder für eine Gruppe -O-Z-$NR^{11'}R^{12'}$, -$NHR^{10'}$, -$NR^{11'}R^{12'}$ oder -OM steht,

$R^{9'}$ für Formyl, Cyano oder für die Gruppe

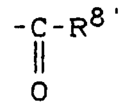

steht,

$R^{10'}$ für Wasserstoff, geradkettiges oder verzweigtes Alkyl mit 1 bis 6 Kohlenstoffatomen oder unsubstituiertes oder einfach bis fünffach, gleich oder verschieden substituiertes Aryl mit 6 bis 10 Kohlenstoffatomen steht, wobei als Arylsubstituenten die oben aufgeführten Arylsubstituenten in Frage kommen,

$R^{11'}$ für geradkettiges oder verzweigtes Alkyl mit 1 bis 6 Kohlenstoffatomen oder unsubstituiertes oder einfach bis fünffach, gleich oder verschieden substituiertes Aryl mit 6 bis 10 Kohlenstoffatomen steht, wobei als Arylsubstituenten die oben aufgeführten Arylsubstituenten in Frage kommen,

$R^{12'}$ für geradkettiges oder verzweigtes Alkyl mit 1 bis 6 Kohlenstoffatomen oder unsubstituiertes oder einfach bis fünffach, gleich oder verschieden substituiertes Aryl mit 6 bis 10 Kohlenstoffatomen steht, wobei als Arylsubstituenten die oben aufgeführten Arylsubstituenten infrage kommen,

M für Wasserstoff oder für ein Äquivalent eines entsprechenden Alkalimetall-, Erdalkalimetall-oder Ammoniumkations steht und

Z für eine geradkettige oder verzweigte Alkylkette mit 1 bis 8 Kohlenstoffatomen steht,

oder

$R^{2'}$ und $R^{3'}$ gemeinsam für einen der Reste

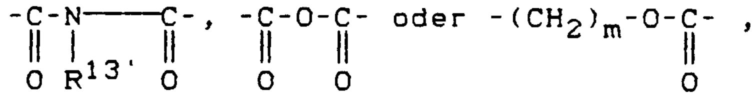

verbrückt über die Positionen 6 und 5, stehen,

worin

$R^{13'}$ für Wasserstoff, geradkettiges oder verzweigtes Alkyl mit 1 bis 6 Kohlenstoffatomen oder unsubstituiertes oder einfach bis fünffach, gleich oder verschieden substituiertes Aryl mit 6 bis 10 Kohlenstoffatomen steht, wobei als Arylsubstituenten die oben aufgeführten Arylsubstituenten in Frage kommen und

m für eine Zahl 1 oder 2 steht,

oder

$R^{4'}$ und $R^{5'}$ gemeinsam für eine Alkylkette mit 3- oder 4-Kohlenstoffatomen stehen, die über die Positionen 4 und 3 verbunden ist.

Bevorzugte erfindungsgemäße Verbindungen sind auch Additionsprodukte aus Säuren und denjenigen substituierten 2-Cyclohexen-1-yl-amin-Derivaten der Formel (Ia) in denen $R^{1'}$, $R^{2'}$, $R^{3'}$, $R^{4'}$, $R^{5'}$ und $R^{6'}$ die oben angegebenen Bedeutungen haben.

Zu den Säuren, die addiert werden können, gehören vorzugsweise Halogenwassersäuren, wie z.B. die Chlorwasserstoffsäure und die Bromwassersäure, insbesondere die Chlorwassersäure, ferner Phosphorsäure, Salpetersäure, mono- und bifunktionelle Carbonsäuren und Hydroxycarbonsäuren, wie z.B. Essigsäure, Trifluoressigsäure, Maleinsäure, Bernsteinsäure, Fumarsäure, Weinsäure, Zitronensäure, Salicylsäure, Sorbinsäure und Milchsäure, Ölsäure, Stearinsäure, gegebenenfalls einfach oder mehrfach durch Nitro oder Halogen substituierte Benzoesäure, Gluconsäure, Ascorbinsäure, Äpfelsäure, Sulfamidsäure, Sulfonsäuren, wie z.B. p-Toluolsulfonsäure, 1,5-Naphthalindisulfonsäure und Methansulfonsäure, sowie Imide wie z.B. Phthalimid, Saccharin und Thiosaccharin.

Außerdem bevorzugte erfindungsgemäße Verbindungen sind auch Additionsprodukte aus Salzen von Metallen der I., II. und III. Hauptgruppen sowie des Zinns, sowie ferner Salze von Metallen der I., II., VII. und VIII. Nebengruppe des Periodensystems der Elemente und denjenigen substituierten 2-Cyclohexen-1-yl-amin-Derivaten der Formel (Ia), in denen $R^{1'}$, $R^{2'}$, $R^{3'}$, $R^{4'}$, $R^{5'}$ und $R^{6'}$ die oben angegebenen Bedeutungen haben.

Hierbei sind Salze des Kupfers, Zinks, Mangans, Magnesiums, Calciums, Zinns, Eisens, Cobalts und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwassersäure, ferner Phosphorsäure, Salpetersäure und Schwefelsäure.

Besonders bevorzugt sind die Verbindungen der Formel (Ia), in welcher

$R^{1'}$ für Wasserstoff, geradkettiges oder verzweigtes Alkyl mit 1 bis 4 Kohlenstoffatomen oder Fluor, Chlor oder Brom steht,

$R^{2'}$ für Formyl, geradkettiges oder verzweigtes Hydroxyalkyl mit 1 bis 4 Kohlenstoffatomen im Alkylteil, Cyano, Nitro oder für einen der Reste

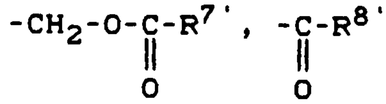

oder $-CH=CH-R^{9'}$ steht,

$R^{3'}$, $R^{4'}$, $R^{5'}$ und $R^{6'}$ gleich oder verschieden sind und jeweils für Wasserstoff, jeweils geradkettiges oder verzweigtes Alkyl oder Alkoxy mit 1 bis 6 Kohlenstoffatomen, jeweils geradkettiges oder verzweigtes Alkenyl, Alkinyl, Alkenyloxy oder Alkinyloxy mit jeweils 2 bis 6 Kohlenstoffatomen, Alkoxyalkyloxy mit jeweils 1 bis 6 Kohlenstoffatomen in den einzelnen Alkylteilen, für jeweils im Phenylteil unsubstituiertes oder einfach bis dreifach, gleich oder verschieden substituiertes Phenyl oder Phenylalkyl mit gegebenenfalls 1 oder 2 Kohlenstoffatomen im Alkylteil stehen, wobei als Phenylsubstituenten infrage kommen: Fluor, Chlor, Brom, Nitro, Cyano,

Amino, $C_1$-$C_2$-Alkyl, $C_1$-$C_3$-Alkoxy, $C_1$-$C_2$-Alkylthio, Halogen-($C_1$-$C_2$)-alkyl, Halogen-($C_1$-$C_2$)-alkoxy, Halogen-($C_1$-$C_2$)-alkylthio mit jeweils 1 bis 5 gleichen oder verschiedenen Fluor- und/ oder Chloratomen und Di-($C_1$-$C_2$)-alkylamino,
weiterhin für eine unsubstituierte oder einfach bis dreifach, gleich oder verschieden substituierte und gegebenenfalls über eine Methylengruppe gebundene heterocyclische fünf-bzw. sechsgliedrige Gruppierung aus der Reihe Furyl, Thienyl, Pyrrolyl, Pyrazolyl, Imidazolyl, 1,2,3- oder 1,2,4-Triazolyl, Oxazolyl, Isoxazolyl, 1,2,4- oder 1,3,4-Oxadiazolyl, Thiazolyl, Isothiazolyl, 1,2,3-, 1,2,4-, 1,2,5- oder 1,3,4-Thiadiazolyl, Pyridyl, Pyridazinyl, Pyrimidinyl und Pyrazinyl stehen, wobei als Substituenten für ,den Heterocyclus jeweils infrage kommen: Fluor, Chlor, Brom, Nitro, Cyano, Amino, $C_1$-$C_2$-Alkyl, $C_1$-$C_2$-Alkoxy oder $C_1$-$C_2$-Alkylthio, Halogen-($C_1$-$C_2$)-alkyl, Halogen-($C_1$-$C_2$)-alkoxy, Halogen-($C_1$-$C_2$)-alkylthio mit jeweils 1 bis 5 gleichen oder verschiedenen Fluor- und/oder Chloratomen und Di-($C_1$-$C_2$)-alkylamino,
weiterhin für Fluor, Chlor oder Brom stehen, wobei mindestens zwei der Reste $R^{3'}$, $R^{4'}$, $R^{5'}$ und $R^{6'}$ für Wasserstoff stehen,

$R^{7'}$ für jeweils geradkettiges oder verzweigtes Alkyl oder Alkoxy mit 1 bis 4 Kohlenstoffatomen steht,

$R^{8'}$ für Hydroxy, geradkettiges oder verzweigtes Hydroxyalkyloxy mit 1 bis 6 Kohlenstoffatomen, geradkettiges oder verzweigtes Halogenalkyloxy mit 1 bis 6 Kohlenstoffatomen und 1 bis 13 gleichen oder verschiedenen Halogenatomen, unsubstituiertes oder einfach bis dreifach, gleich oder verschieden durch Fluor, Chlor, Brom substituiertes Cycloalkyloxy mit 3 bis 6 Kohlenstoffatomen, jeweils geradkettiges oder verzweigtes Alkoxy oder Alkylthio mit 1 bis 4 Kohlenstoffatomen, geradkettiges oder verzweigtes Alkoxyalkylalkoxy mit jeweils 1 bis 4 Kohlenstoffatomen im Alkoxy- oder Alkylteil, jeweils im Phenylteil unsubstituiertes oder einfach bis dreifach, gleich oder verschieden substituiertes Phenyloxy, Phenylthio, Phenylalkyl oder Phenylalkyloxy mit jeweils gegebenenfalls 1 bis 6 Kohlenstoffatomen im Alkylteil steht, wobei als Phenylsubstituenten die oben aufgeführten Phenylsubstituenten in Frage kommen oder für eine Gruppe -O-Z-$NR^{11'}R^{12'}$, -$NHR^{10'}$, -$NR^{11'}R^{12'}$ oder -OM steht,

$R^{9'}$ für Formyl, Cyano oder für die Gruppe

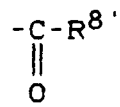

steht,

$R^{10'}$ für Wasserstoff, geradkettiges oder verzweigtes Alkyl mit 1 bis 4 Kohlenstoffatomen oder unsubstituiertes oder einfach bis dreifach, gleich oder verschieden substituiertes Phenyl steht, wobei als Phenylsubstituenten die oben aufgeführten Phenylsubstituenten infrage kommen,

$R^{11'}$ für geradkettiges oder verzweigtes Alkyl mit 1 bis 4 Kohlenstoffatomen oder unsubstituiertes oder einfach bis dreifach, gleich oder verschieden substituiertes Phenyl steht, wobei als Phenylsubstituenten die oben aufgeführten Phenylsubstituenten in Frage kommen,

$R^{12'}$ für geradkettiges oder verzweigtes Alkyl mit 1 bis 4 Kohlenstoffatomen oder unsubstituiertes oder einfach bis dreifach, gleich oder verschieden substituiertes Phenyl steht, wobei als Phenylsubstituenten die oben aufgeführten Phenylsubstituenten in Frage kommen,

M für Wasserstoff oder für ein Äquivalent eines entsprechenden Alkalimetall-, Erdalkalimetall- oder Ammoniumkations steht und

Z für eine geradkettige oder verzweigte Alkylkette mit 1 bis 6 Kohlenstoffatomen steht,

oder

$R^{2'}$ und $R^{3'}$ gemeinsam für einen der Reste :

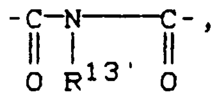, 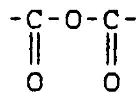 oder 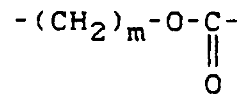,

verbrückt über die Positionen 6 und 5, stehen,

$R^{13'}$ für Wasserstoff, geradkettiges oder verzweigtes Alkyl mit 1 bis 6 Kohlenstoffatomen oder unsubstituiertes oder einfach bis dreifach, gleich oder verschieden substituiertes Phenyl steht, wobei als Phenylsubstituenten die oben aufgeführten Phenylsubstituenten infrage kommen und

m für eine Zahl 1 oder 2 steht,

oder

$R^{4'}$ und $R^{5'}$ gemeinsam für eine Alkylkette mit 3- oder 4-Kohlenstoffatomen stehen, die über die Positionen 4 und 3 verbunden ist.

In diesem Zusammenhang sind die gleichen Säureadditions-Salze und Metallsalz-Komplexe zu nennen, die bereits bei der Beschreibung der bevorzugten erfindungsgemäß substituierten 2-Cyclohexen-1-yl-amin-Derivate der Formel (Ia) genannt wurden.

Man erhält die substituierten 2-Cyclohexen-1-yl-amin-Derivate der Formel (Ia), wenn man

A) 2-Cyclohexen-1-yl-carbonsäurederivate der Formel (II)

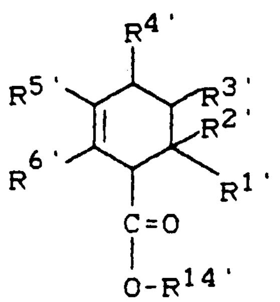

(II)

in welcher

$R^{1'}$, $R^{2'}$, $R^{3'}$, $R^{4'}$, $R^{5'}$, und $R^{6'}$ , die oben angegebene Bedeutung haben und

$R^{14'}$ für Wasserstoff, Methyl oder Ethyl steht,

in allgemein üblicher Weise, nach Curtius, gegebenenfalls in Gegenwart eines Verdünnungsmittels, wie beispielsweise Aceton und in Gegenwart einer Base, wie beispielsweise N,N-Diisopropylamin, bei Temperaturen zwischen -15 °C und +10 °C, mit Chlorameisensäureester versetzt und zu dieser Reaktionsmischung gegebenenfalls in Gegenwart eines Verdünnungsmittels, wie beispielsweise Wasser, bei Temperaturen zwischen -5 °C und +25 °C ein Azid, wie beispielsweise Natriumazid, zugibt,

und das intermediär auftretende Isocyanat der Formel (IIa)

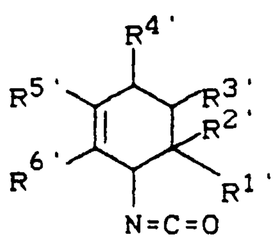

(IIa)

in welcher

$R^{1'}$, $R^{2'}$, $R^{3'}$, $R^{4'}$, $R^{5'}$, und $R^{6'}$ die oben angegebene Bedeutung haben,

mit Wasser, gegebenenfalls in Gegenwart einer Säure oder einer Base, hydrolysiert und die so erhaltenen Amine gegebenenfalls in Säureadditions-Salze und Metallsalz-Komplexe überführt [vgl. J. Org. Chem. 26, (1961), 3511].

Man erhält die substituierten 2-Cyclohexen-1-yl-amin-Derivate der Formel (Ia) außerdem
B) aus den 2-Cyclohexen-Derivaten der Formel (IIb)

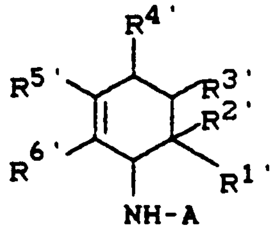

(IIb)

in welcher

| | |
|---|---|
| $R^{1'}$, $R^{2'}$, $R^{3'}$, $R^{4'}$, $R^{5'}$, und $R^{6'}$ | die oben angegebene Bedeutung haben und |
| A | für eine Aminoschutzgruppe steht, |

in an sich bekannter Weise nach üblichen Methoden z.B. durch Solvolyse, wie Hydrolyse, Acidolyse, durch Reduktion, wie z.B. durch Hydrogenolyse in Gegenwart eines Hydrierungskatalysators oder mittels eines Reduktionssystems aus Metall und protonenabspaltendem Mittel, wobei je nach Art der Schutzgruppe verschiedenartige (auch andersartige) sowie selektive Abspaltungsmethoden angewendet werden können, gegebenenfalls in Gegenwart eines geeigneten Lösungs- oder Verdünnungsmittels oder eines Gemisches aus ihnen, wobei man je nach Bedarf unter Kühlen, bei Raumtemperatur oder unter Erwärmen, z.B. in einem Temperaturbereich von etwa -10 °C bis zur Siedetemperatur des Reaktionsmediums, vorzugsweise von etwa -10 °C bis etwa 150 °C, und, falls erforderlich, in einem geschlossenen Gefäss, unter Druck, in einer Inertgasatmosphäre und/oder unter wasserfreien Bedingungen arbeitet und die so erhaltenen Produkte gegebenenfalls in Säureadditions-Salze oder Metallsalz-Komplexe überführt (vgl. Protective Groups in Organic Synthesis, Th. W. Greene, Wiley Interscience, 1981).

Die vorne u.a. als Aminoschutzgruppe erwähnte Formyl-, Acetyl- oder 2,2,2-Trichloracetyl-gruppe kann beispielsweise durch Hydrolyse abgespalten werden.

Die Hydrolyse erfolgt in an sich bekannter Weise mit Hilfe von Wasser, wobei vorteilhaft in Gegenwart einer die Hydrolyse unterstützenden Säure oder Base, gegebenenfalls in Gegenwart eines inerten Lösungsmittels oder Verdünnungsmittels und/oder unter Kühlen oder Erwärmen gearbeitet wird.

Als Säuren kommen beispielsweise anorganische Säuren, wie Mineralsäuren, z.B. Schwefelsäure, Phosphorsäure oder Halogenwassersäuren, organische Carbonsäuren, wie Niederalkancarbonsäuren, z.B. Eisessig, wie gegebenenfalls ungesättigte Dicarbonsäuren, z.B. Oxal-, Malon-, Malein- oder Fumarsäure, oder wie Hydroxycarbonsäuren, z.B. Weinsäure oder Citronensäure, oder Sulfonsäuren, wie $C_1$-$C_7$-Alkan- oder gegebenenfalls substituierte Benzolsulfonsaure, z.B. Methan- oder p-Toluolsulfonsäure, in Betracht.

Als Basen kommen beispielsweise Alkalimetallhydroxide, -hydride, -amide, -alkanolate, -carbonate, -triphenylmethylide, -di-$C_1$-$C_7$-alkylamide, -amino-$C_1$-$C_7$-alkylamide oder -$C_1$-$C_7$-alkylsilylamide, Naphthalin-amine, $C_1$-$C_7$-Alkylamine, basische Heterocyclen, Ammoniumhydroxide sowie carbocyclische Amine in Frage. Beispielhaft seien Lithium-hydroxid, Natrium-hydroxid, -hydrid, -amid, -ethylat, Kalium-tert-butylat, -carbonat, Lithiumtriphenylmethylid, -diisopropylamid, Kalium-3-(aminopropyl)amid, -bis-(trimethylsilyl)-amid, Dimethyl-aminonaphthalin, Di- oder Triethylamin, Pyridin, Benzyltrimethyl-ammoniumhydroxid, 1,5-Diazabicyclo[4.3.0]non-5-en (DBN) sowie 1,8-Diaza-bicyclo[5.4.0]undec-7-en (DBU) genannt.

Die Acidolyse gelingt z.B. mit starken Säuren, zweckmäßig mit Trifluoressigsäure oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich. Als inerte Lösungsmittel eignen sich vorzugsweise organische, beispielsweise Carbonsäuren wie Essigsäure, Ether wie Tetrahydrofuran oder Dioxan, Amide wie Dimethylformamid (DMF), halogenierte Kohlenwasserstoffe wie Dichlormethan, ferner auch Alkohole, wie Methanol, Ethanol oder Isopropanol sowie Wasser.

Ferner kommen Gemische der vorgenannten Lösungsmittel in Frage. Trifluoressigsäure wird vorzugsweise im Überschuß ohne Zusatz eines weiteren Lösungsmittels verwendet, Perchlorsäure vorzugsweise in Form eines Gemisches aus Essigsäure und 70 %iger Perchlorsäure im Verhältnis 9 : 1. Die Reaktionstemperaturen für diese Solvolysen liegen zweckmäßig zwischen etwa 0 und etwa 50 °C, vorzugsweise arbeitet man zwischen 15 und 30 °C (Raumtemperatur).

Die BOC-Gruppe kann z.B. bevorzugt mit 40 %iger Trifluoressigsäure in Methylenchlorid oder mit etwa 3

bis 5 n Salzsäure in Dioxan bei 15 - 30 °C abgespalten werden, die FMOC-Gruppe (9-Fluorenylmethyloxycarbonyl) mit einer etwa 5-bis 20%igen Lösung von Dimethylamin, Diethylamin oder Piperidin in Dimethylformamid bei 15 - 30 °C. Eine Abspaltung der DNP-Gruppe (2,4-Dinitrophenyl) gelingt z.B. auch mit einer etwa 3- bis 10%igen Lösung von 2-Mercaptoethanol in Dimethylformamid/Wasser bei 15-30 °C. Hydrogenolytisch entfernbare Schutzgruppen (z.B. BOM, CBZ oder Benzyl) können z.B. durch Behandeln mit Wasserstoff in Gegenwart eines Katalysators (z.B. eines Edelmetallkatalysators wie Palladium, zweckmäßig auf einem Träger wie Kohle) abgespalten werden. Als Lösungsmittel eignen sich dabei die oben angegebenen, insbesondere z.B. Alkohole, wie Methanol oder Ethanol oder Amide wie Dimethylformamid. Die Hydrogenolyse wird in der Regel bei Temperaturen von etwa 0 bis 100 °C und einem Druck von etwa 1 bis 200 bar, bevorzugt bei 20 bis 30 °C und 1 bis 10 bar durchgeführt. Eine Hydrogenolyse der CBZ-Gruppe gelingt z.B. gut an 5- bis 10%igem Pd-Kohle in Methanol bei 20 -30 °C.

Als Aminoschutzgruppen, die mittels eines Reduktionssystems aus Metall und protonenabspaltendem Mittel abgespalten werden, sind beispielsweise (4-Nitro)-benzyloxycarbonyl, 2-lod- oder 2,2,2-Trichlorethoxycarbonyl oder Phenacyloxycarbonyl, zu nennen.

Der Metallbestandteil des metallischen Reduktionssystems ist beispielsweise ein unedles Metall, wie Alkali- oder Erdalkalimetall, z.B. Lithium, Natrium, Kalium, Magnesium oder Calcium, oder Übergangsmetall, z.B. Zink, Zinn, Eisen oder Titan, während als Protonen-abspaltendes Mittel, z.B. Protonsäuren der vorstehend genannten Art, wie Salz- oder Essigsäure, $C_1$-$C_7$-Alkohole, wie Ethanol, und/oder Amine bzw. Ammoniak in Frage kommen. Solche Systeme sind beispielsweise Natrium/Ammoniak, Zink/Salz-oder Essigsäure oder Zink/Ethanol.

4-Nitrobenzyloxycarbonyl kann ferner z.B. mit einem Dithionit, wie Natriumdithionit, Phenacyloxycarbonyl und 2-Halogen-$C_2$-$C_7$-alkanoyl z.B. mit Hilfe eines nucleophilen Reagens, wie einem Thiolat, z.B. Natriumthiophenolat, oder Thioharnstoff und Base und sich anschließender Hydrolyse, und Allyl oder But-2-enyl, mit Hilfe eines Rhodium(III)halogenids, wie Rhodium(III)chlorid, gespalten werden.

Die bekannten Verbindungen der Formel (I) lassen sich in Analogie zu den neuen Verbindungen der Formel (Ia) herstellen.

Verwendet man als Ausgangsstoffe beispielsweise 2-Carboxy-5-methyl-cyclohex-3-en-carbonsäure-methylester, Chlorameisensäureethylester und N,N-Diisopropylethylamin als Base für die erste Stufe und Natriumazid und Wasser für die zweite Stufe, so läßt sich der Reaktionsablauf des Herstellungsverfahrens (A) durch das folgende Formelschema darstellen:

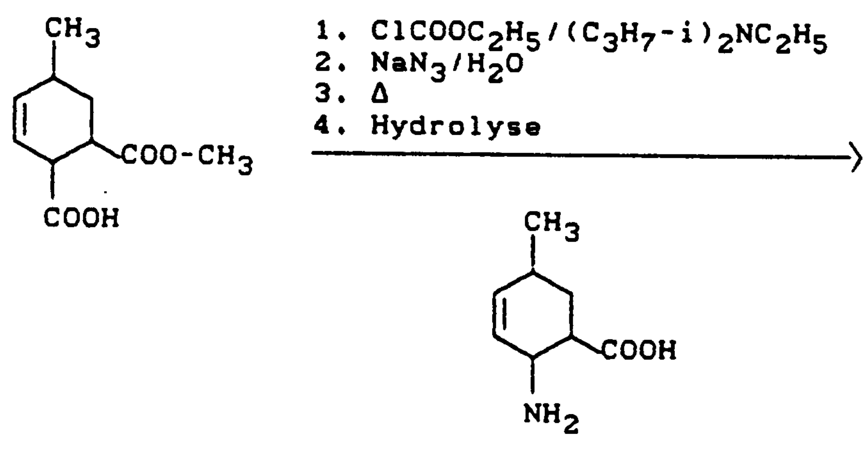


Verwendet man als Ausgangsstoffe beispielsweise tert-Butyl-(3-methyl-6-carboxy-2-cyclohexen-1-yl)-carbamat und 1 N Salzsäure, so läßt sich der Reaktionsablauf des Herstellungsverfahrens (B) durch das folgende Formelschema darstellen:

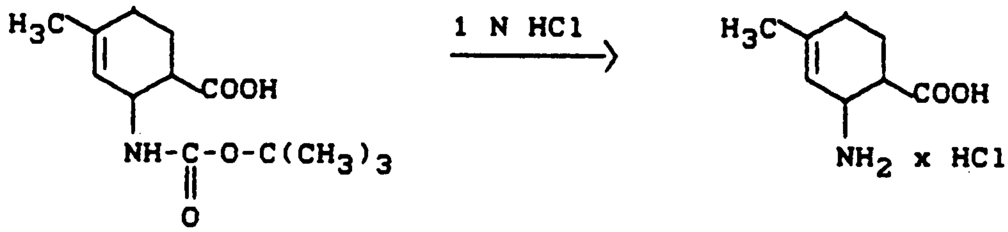


Die zur Durchführung des Herstellungsverfahrens (A) als Ausgangsstoffe benötigten 2-Cyclohexen-1-yl-carbonsäure-Derivate sind durch die Formel (II) allgemein definiert In dieser Formel (II) stehen $R^{1'}$, $R^{2'}$, $R^{3'}$, $R^{4'}$, $R^{5'}$, und $R^{6'}$ vorzugsweise bzw. insbesondere für diejenigen Substituenten, die oben bei der Beschreibung der neuen 2-Cyclohexen-1-yl-amin-Derivate der Formel (Ia) als bevorzugt bzw. besonders bevorzugt für diese Reste genannt wurden.

Die 2-Cyclohexen-1-yl-carbonsäure-Derivate der Formel (II) sind teilweise bekannt und/oder können nach bekannten Verfahren in einfacher analoger Weise hergestellt werden (vgl. Houben-Weyl, Methoden der Organischen Chemie, 4th Edition, E.Müller, Ed. Vol. 5/1c, Georg Thieme Verlag, Stuttgart, 1970, 977; J. Chem. Soc. Perkin Trans. 1, 1557-62, 1981), indem man beispielsweise die bekannten Dienophile der Formel (III)

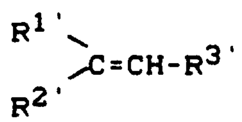 (III)

in welcher

$R^{1'}$, $R^{2'}$ und $R^{3'}$ die oben angegebene Bedeutung haben,

mit den entsprechenden Diencarbonsäurederivaten der Formel (IV)

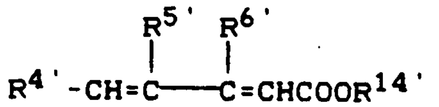 (IV)

in welcher

$R^{4'}$, $R^{5'}$ und $R^{6'}$ die oben angegebene Bedeutung haben und

$R^{14'}$ für Wasserstoff, Methyl oder Ethyl steht,

gegebenenfalls in Gegenwart eines Verdünnungsmittels, gegebenenfalls in Gegenwart eines Katalysators, gegebenenfalls in Gegenwart eines Inertgases und gegebenenfalls unter Druck bei Temperaturen zwischen -50 °C und 150 °C cyclisiert.

Die zur Herstellung der 2-Cyclohexen-1-yl-carbonsäure-Derivate der Formel (II) und der 2-Cyclohexen-Derivate der Formel (IIb) als Ausgangsstoffe benötigten Dienophile sind durch die Formel (III) allgemein definiert. In dieser Formel (III) stehen $R^{1'}$, $R^{2'}$, und $R^{3'}$ für diejenigen Reste, die bereits im Zusammenhang mit der Beschreibung der erfindungsgemäßen Verbindungen der Formel (Ia) für diese Substituenten genannt wurden.

Beispielhaft, aber nicht begrenzend, seien für Verbindungen der Formel (III) genannt: Acrylsäure, Acrylsäureester, wie beispielsweise Acrylsäuremethylester, Acrylsäureethylester, Acrylsäureamide, wie beispielsweise N,N-Dimethylacrylamid, Acrylnitril, Chloracrylnitril, Maleinsäureanhydrid, Maleinsäureimide, wie beispielsweise N-Phenylmaleinsäureimid, Vinylderivate, wie beispielsweise Vinylphosphonsäure, Vinylphosphonsäuredimethylester und ω-Nitrostyrol.

Die Verbindungen der Formel (III) sind allgemein bekannte Verbindungen der organischen Chemie.

Die zur Herstellung der 2-Cyclohexen-1-yl-carbonsäure-Derivate der Formel (II) außerdem als Ausgangsstoffe benötigten Diencarbonsäure-Derivate sind durch die Formel (IV) allgemein definiert. In dieser Formel (IV) stehen $R^{4'}$, $R^{5'}$ und $R^{6'}$ für diejenigen Reste, die bereits im Zusammenhang mit der Beschreibung der erfindungsgemäßen Verbindungen der Formel (Ia) für diese Substituenten genannt wurden.

Die Diencarbonsäure-Derivate der Formel (IV) sind bekannt und/oder können nach literaturbekannten Verfahren in einfacher, analoger Weise hergestellt werden (vgl. 'Some modern Methods of Organic Synthesis', W.Carruthers, Cambridge University Press, 1986, S.125; Acc. Chem. Res., 1979, 146).

Die zur Durchführung des Herstellungsverfahrens (B) als Ausgangsstoffe benötigten 2-Cyclohexen-Derivate sind durch die Formel (IIb) allgemein definiert. In dieser Formel (IIb) stehen $R^{1'}$, $R^{2'}$, $R^{3'}$, $R^{4'}$, $R^{5'}$, $R^{6'}$ und A vorzugsweise bzw. insbesondere für diejenigen Substituenten, die oben bei der Beschreibung der neuen 2-Cyclohexen-1-yl-amin-Derivate der Formel (Ia) als bevorzugt bzw. besonders bevorzugt für diese Reste genannt wurden.

Die 2-Cyclohexen-Derivate der Formel (IIb) sind teilweise neu und Teil der vorliegenden Erfindung.

Ausgenommen sind die bekannten nachfolgend aufgeführten Verbindungen und ihre Enantiomeren und Isomeren: 6-Formyl-5-{[(phenylmethoxy)-carbonyl]-amino}-3-cyclohexen-1-carbonsäuremethylester und 6-(3-Oxo-1-propenyl)-5-{[(phenylmethoxy)-carbonyl]-amino}-3-cyclohexen-1-carbonsäuremethylester (vgl. J. Med. Chem., 29, 1 - 8, 1986; J. Med. Chem., 24, 788-94, 1981), 3-Cyclohexen-2-[(trichloracetyl)-amino]-1-carbonsäuremethylester, 2,2,2-Trichlor-N-(6-formyl-2-cyclohexen-1-yl)-acetamid, (6-Formyl-5-methyl-2-cyclohexen-1-yl)-carbaminsäureethylester, 2-[(Ethoxy-carbonyl)-amino]-6-methyl-3-cyclohexen-1-carbonsäuremethylester, 2-[(Ethoxycarbonyl)-amino]-5-methyl-3-cyclohexen-1-carbonsäuremethylester, 2-[-(Ethoxycarbonyl)-amino]-3-cyclohexen-1-carbonsäuremethylester, 3-{2-[(Ethoxycarbonyl)-amino]-6-methyl-3-cyclohexen-1-yl}-2-propensäureethylester, (6-Formyl-5-propyl-2-cyclohexen-1-yl)-carbaminsäurephenylmethylester, (6-Formyl-5-methyl-2-cyclohexen-1-yl)-carbaminsäurephenylmethylester, 2-[-(Phenoxycarbonyl)-amino]-3-cyclohexan-1-carbonsäuremethylester und 3-⟨6-Methyl-2-{[(phenylmethoxy)-carbonyl]-amino}-3-cyclohexen-1-yl⟩-2-propensäureethylester, Phenyl-6-carbomethoxy-2-cyclohexen-1-yl-thiocarbamat, N-(6-carbomethoxy-2-cyclohexen-1-yl)-1-pyrrolidin-carboxamid (vgl. J. Am. Chem. Soc., 103, 2816-22, 1981; J. Am. Chem. Soc., 100, 3182-9, 1978; J. Am. Chem. Soc., 100, 5179-85, 1978 und Tetrahedron Lett. 25, 2183-6, 1984), (6-Formyl-5-pentyl-2-cyclohexen-1-yl)-carbaminsäurephenylmethylester (vgl. J. Org Chem., 46, 2833-5, 1981) und {6-Formyl-5-[2-(methoxymethoxy)-ethyl]-2-cyclohexen-1-yl}-carbaminsäurephenylmethylester (vgl. J. Am. Chem. Soc., 105, 5373-9, 1983), 2-[(p-Toluolsulfonyl)-amino]-3-cyclohexen-1-carbonsäuremethylester, - carbonsäure-t-butylester, - carbonsäure-benzylester und - carbonsäure-2,4,6-trimethylphenylester (vgl. J. Org. Chem., 43, 1448-1455, (1978).

Man erhält die neuen 2-Cyclohexen-Derivate der Formel (IIb) indem man Dienophile der Formel (III)

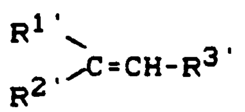 (III)

in welcher

$R^{1'}$, $R^{2'}$ und $R^{3'}$ die oben angegebene Bedeutung haben

(B/a)mit N-Acyl-1-amino-1,3-butadien-Derivaten der Formel (IVa)

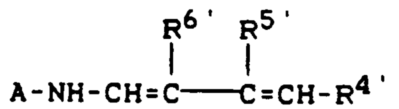 (IVa)

in welcher

$R^{4'}$, $R^{5'}$, $R^{6'}$ und A die oben angegebene Bedeutung haben,

gegebenenfalls in Gegenwart eines Verdünnungsmittels, gegebenenfalls in Gegenwart eines Katalysators, gegebenenfalls in Gegenwart eines Inertgases und gegebenenfalls unter Druck, cyclisiert oder

(B/b)mit substituierten Butadienen der Formel (IVb)

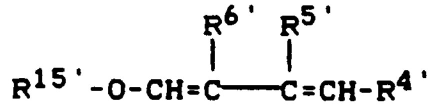 (IVb)

in welcher

$R^{4'}$, $R^{5'}$ und $R^{6'}$ die oben angegebene Bedeutung haben und

$R^{15'}$ für den Acetyl- oder Trimethylsilylrest steht,

zunächst in einer ersten Stufe gegebenenfalls in Gegenwart eines Verdünnungsmittels, gegebenenfalls in Gegenwart eines Katalysators, gegebenenfalls in Gegenwart eines Inertgases und gegebenenfalls unter Druck, cyclisiert

und die so erhältlichen 2-Cyclohexen-Derivate der Formel (IIc)

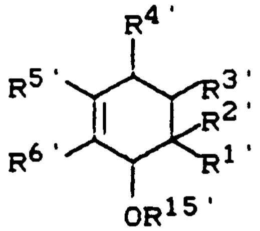

(IIc)

in welcher

$R^{1'}$, $R^{2'}$, $R^{3'}$, $R^{5'}$ und $R^{6'}$ die oben angegebene Bedeutung haben und

$R^{15'}$ für den Acetyl- oder Trimethylsilylrest steht

in einer zweiten Stufe, in allgemein üblicher Art und Weise, mit 4,4'-Dimethoxybenzhydrylamin (DMB) der Formel (V)

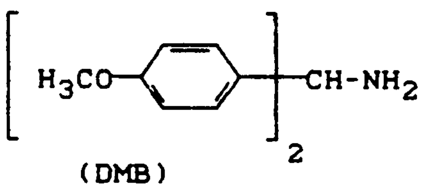

(V)

(DMB)

gegebenenfalls in Gegenwart eines Verdünnungsmittels, wie beispielsweise Tetrahydrofuran, bei Temperaturen von 0 °C bis zur Siedetemperatur des jeweils verwendeten Verdünnungsmittels und in Gegenwart eines Katalysators, wie beispielsweise Tetrakis(triphenylphosphin)-palladium(O) der Formel (VI)

$[(C_6H_5)_3P]_4Pd$ (VI)

umsetzt [vgl. J. Org. Chem. 1979, 3451 (1978)].

oder

(B/c)die nach Verfahren (A) intermediär auftretenden Isocyanate der Formel (IIa)

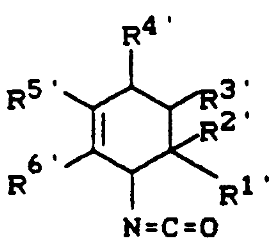

(IIa)

in welcher

$R^{1'}$, $R^{2'}$, $R^{3'}$, $R^{4'}$, $R^{5'}$ und $R^{6'}$ die oben angegebene Bedeutung haben,

mit Alkoholen der Formel (VII)

$R^{16'}$-OH (VII)

in welcher

$R^{16'}$ für jeweils geradkettiges oder verzweigtes, gegebenenfalls durch Halogen substituiertes Alkyl, Alkenyl, Alkinyl oder Alkoxyalkyl mit 1 bis 12 Kohlenstoffatomen (vorzugsweise 1 bis 6 Kohlenstoffatomen), unsubstituiertes oder substituiertes Phenyl oder Benzyl steht,

umsetzt,

oder

(B/d)man erhält die substituierten 2-Cyclohexen-Derivate der Formel (IIb), wenn man die nach Verfahren (B/a), (B/b) oder (B/c) erhältlichen 2-Cyclohexen-Derivate der Formel (IId)

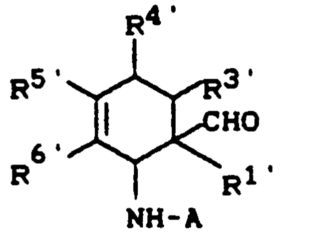

(IId)

in welcher

$R^{1'}$, $R^{3'}$, $R^{4'}$, $R^{5'}$, $R^{6'}$ und A die oben angegebene Bedeutung haben,

in allgemein üblicher Weise mit einem komplexen Metallhydrid, wie beispielsweise Natriumborhydrid, in einem geeigneten Lösungsmittel, wie beispielsweise Alkoholen, wie Methanol, Ethanol, Butanol oder Isopropanol und Ethern, wie beispielsweise Diethylether oder Tetrahydrofuran bei Temperaturen von 0 °C bis 2 °C reduziert und die so erhältlichen 2-Cyclohexen-1-yl-amin-alkohole der Formel (IIe)

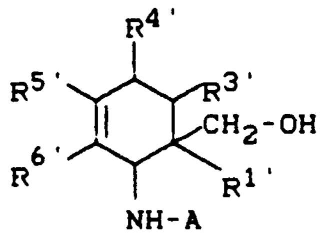

(IIe)

in welcher

$R^{1'}$, $R^{3'}$, $R^{4'}$, $R^{5'}$, $R^{6'}$ und A die oben angegebene Bedeutung haben,

durch weitere Umsetzungen an der Hydroxy-Gruppe zum Beispiel in Ester und Ether überführt. Weiterhin können durch Umsetzungen mit z.B. Acylhalogeniden oder Carbamoylchloriden Acyl- oder Carbamoyl-Derivate der Verbindungen der Formel (IIb) erhalten werden,

oder

(B/e)man erhält die substituierten 2-Cyclohexen-Derivate der Formel (IIb), wenn man die nach Verfahren (B/a), (B/b) oder (B/c) erhältlichen 2-Cyclohexen-Derivate der Formel (IId)

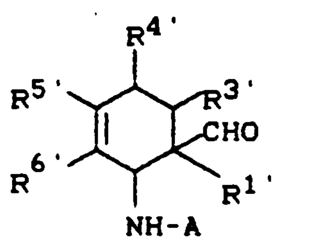

(IId)

in welcher

$R^{1'}$, $R^{3'}$, $R^{4'}$, $R^{5'}$, $R^{6'}$ und A die oben angegebene Bedeutung haben,

mit Alkanphosphonsäure-Derivaten der Formel (VIII)

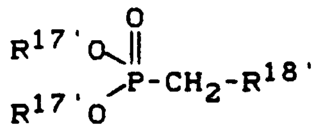

(VIII)

in welcher

$R^{17'}$ für Methyl oder Ethyl steht und

$R^{18'}$ für die Cyano- oder für die Alkoxycarbonylgruppe steht

gegebenenfalls in Gegenwart eines Verdünnungsmittels, gegebenenfalls in Gegenwart einer Base und gegebenenfalls in Gegenwart eines Inertgases, umsetzt.

Das erfindungsgemäßen Verfahren (B/a) zur Herstellung der neuen 2-Cyclohexen-Derivate der Formel (IIb) wird vorzugsweise unter Verwendung von Verdünnungsmitteln durchgeführt.

Als Verdünnungsmittel kommen dabie praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Acetone, Methyl-ethyl-, Methyl-isopropyl- und Methyl-isobutyl-keton, Ester wie Essigsäuremethylester und -ethylester, Nitrile wie Acetonitril und Propionitril, Amide wie z.B. Dimethylformamid, Dimethylacetamid und N-Methylpyrrolidon sowie Dimethylsulfoxid, Tetramethylensulfon und Hexamethylphosphorsäuretriamid.

Als Inertgase kommen dabei Stickstoff sowie praktisch alle Edelgase, insbesondere Argon infrage.

Die Reaktionstemperaturen können bei dem Verfahren (B/a) zur Herstellung der 2-Cyclohexen-Derivate der Formel (IIb) in einem größeren Bereich variiert werden. Im allgemeinen arbeitet man bei Temperaturen zwischen -70 °C und +250 °C vorzugsweise zwischen -50 °C und +150 °C.

Zur Durchführung des Verfahrens (B/a) zur Herstellung der 2-Cyclohexen-Derivate der Formel (IIb) setzt man auf 1 Mol der N-Acyl-1-amino-1,3-butadien-Derivate der Formel (IVa) im allgemeinen 1 bis 30 Mol, vorzugsweise 1 bis 3 Mol Dienophil der Formel (III), 0,01 bis 20,0 Mol, vorzugsweise 0,1 bis 5,0 Mol an Katalysator und gegebenenfalls 0,1 bis 5 % eines die radikalische Polymerisation hindernden Stabilisators, wie beispielsweise 4-tert-Butylcatechol, ein.

Das erfindungsgemäße Verfahren zur Herstellung der 2-Cyclohexen-Derivate der Formel (IIb) wird im allgemeinen unter erhöhtem Druck durchgeführt. Im allgemeinen arbeitet man bei einem Druck von 1 bis 200 bar, vorzugsweise bei 5 bis 20 bar.

Für die Herstellung der neuen 2-Cyclohexen-1-yl-carbonsäure-Derivate der Formel (IIb) gemäß Verfahrensvariante (B/a) kommen die für derartige Reaktionen üblichen Katalysatoren infrage; vorzugsweise verwendet man Lewis-Säuren, wie beispielsweise Titantetrachlorid, Zinntetrachlorid, Aluminiumtrichlorid und Bortrifluorid-etherat.

Das Verfahren zur Herstellung der neuen 2-Cyclohexen-Derivate der Formel (IIb) kann unter bestimmten Voraussetzungen jedoch auch ohne Verdünnungsmittel und einem Druck von 1 bis 200 bar durchgeführt werden.

Die Reaktionen werden im allgemeinen in einem geeigneten Verdünnungsmittel durchgeführt und das Reaktionsgemisch wird mehrere Stunden bei der jeweils erforderlichen Temperatur gerührt. Die Aufarbeitung erfolgt jeweils nach üblichen Methoden. Im allgemeinen verfährt man in der Weise, daß man das Reaktionsgemisch entweder unter vermindertem Druck einengt oder in Wasser gießt, das Produkt durch Extraktion oder Filtration isoliert und durch Chromatographie reinigt.

Die zur Herstellung der 2-Cyclohexen-Derivate der Formel (IIb) gemäß Verfahrensvariante (B/a) außerdem als Ausgangsstoffe benötigten N-Acyl-1-amino-1,3-butadien-Derivate sind durch die Formel (IVa) allgemein definiert. In dieser Formel (IVa) stehen $R^{4'}$, $R^{5'}$, $R^{6'}$ und A für diejenigen Reste, die bereits im Zusammenhang mit der Beschreibung der erfindungsgemäßen Verbindungen der Formel (Ia) für diese Substituenten genannt wurden.

Die N-Acyl-1-amino-1,3-butadien-Derivate der Formel (IVa) sind bekannt und/oder können nach literaturbekannten Verfahren in einfacher, analoger Weise hergestellt werden [vgl. J. Org. Chem., 43, 2164 (1978)].

Als Verdünnungsmittel zur Durchführung der ersten Stufe des erfindungsgemäßen Verfahrens (B/b) kommen dabei praktisch alle inerten organischen Lösungsmittel infrage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, TetrachlorkohDiethyl- und Dibutylether, Glykoldimethylether und Diglykoldimethylether, Tetrahydrofuran und

Dioxan, Ketone wie Aceton, Methyl-ethyl-, Methyl-isopropyl- und Methylisobutyl-keton, Ester wie Essigsäuremethylester und -ethylester, Nitrile wie z.B. Acetonitril und Propionitril, Amide wie z.B. Dimethylformamid, Dimethylacetamid und N-Methylpyrrolidon sowie Dimethylsulfoxid, Tetramethylensulfon und Hexamethylphosphorsäuretriamid.

Als Inertgase kommen dabei Stickstoff sowie praktisch alle Edelgase, insbesondere Argon infrage.

Die Reaktionstemperaturen können bei der Durchführung der ersten Stufe des erfindungsgemäßen Verfahrens (B/b) zur Herstellung der 2-Cyclohexen-Derivate der Formel (IIc) in einem größeren Bereich variiert werden. Im allgemeinen arbeitet man bei Temperaturen zwischen -70 °C und +250 °C, vorzugsweise zwischen -50 °C und +150 °C.

Zur Durchführung der ersten Stufe des erfindungsgemäßen Verfahrens (B/b) zur Herstellung der 2-Cyclohexen-Derivate der Formel (IIc) setzt man auf 1 Mol der Dienophile der Formel (III) im allgemeinen 1 bis 0,01 Mol, vorzugsweise 1 bis 0,3 Mol der substituierten Butadiene der Formel (IVb), 0,01 bis 20,0 Mol, vorzugsweise 0,1 bis 5,0 Mol an Katalysator und gegebenenfalls 0,1 bis 5 % eines die radikalische Polymerisation hindernden Stabilisators, wie beispielsweise 4-tert-Butylcatechol ein.

Die erste Stufe des erfindungsgemäßen Verfahrens zur Herstellung der 2-Cyclohexen-Derivate der Formel (IIc) wird im allgemeinen unter erhöhtem Druck durchgeführt. Im allgemeinen arbeitet man bei einem Druck von 1 bis 200 bar, vorzugsweise bei einem Druck von 1 bis 20 bar.

Für die erste Stufe des erfindungsgemäßen Verfahrens (B/b) zur Herstellung der neuen 2-Cyclohexen-Derivate der Formel (IIc) kommen die für derartige Reaktionen üblichen Katalysatoren infrage; vorzugsweise verwendet man Lewis-Säuren, wie beispielsweise Titantetrachlorid, Zinntetrachlorid, Aluminiumtrichlorid und Bortrifluoridetherat.

Die erste Stufe des erfindungsgemäßen Verfahrens (B/b) zur Herstellung der neuen 2-Cyclohexen-Derivate der Formel (IIc) kann unter bestimmten Voraussetzungen jedoch auch ohne Verdünnungsmittel und einem Druck von 1 bis 200 bar durchgeführt werden.

Die Reaktionen werden im allgemeinen in einem geeigneten Verdünnungsmittel durchgeführt und das Reaktionsgemisch wird mehrere Stunden bei der jeweils erforderlichen Temperatur gerührt. Die Aufarbeitung erfolgt jeweils nach üblichen Methoden, Im allgemeinen verfährt man in der Weise, daß man das Reaktionsgemisch entweder unter vermindertem Druck einengt oder in Wasser gießt, das Produkt durch Extraktion oder Filtration isoliert und durch Chromatographie reinigt.

Die zur Herstellung der 2-Cyclohexen-Derivate der Formel (IIc) außerdem als Ausgangsstoffe benötigten Butadiene sind durch die Formel (IVb) allgemein definiert. In dieser Formel (IVb) stehen $R^{4'}$, $R^{5'}$ und $R^{6'}$ für diejenigen Reste, die bereits im Zusammenhang mit der Beschreibung der erfindungsgemäßen Verbindungen der Formel (Ia) für diese Substituenten genannt wurden.

Die substituierten Butadiene der Formel (IVb) sind bekannt und/oder können nach literaturbekannten Verfahren in einfacher, analoger Weise hergestellt werden [vgl. J. Org. Chem., 30, 2414 (1965)].

Das für die zweite Stufe des erfindungsgemäßen Verfahrens (B/b) außerdem als Ausgangsstoff benötigte 4,4'-Dimethoxybenzhydrylamin (DMB) der Formel (V) und Tetrakis(triphenylphosphon)-palladium-(O) der Formel (VI) sind allgemein bekannte Verbindungen der organischen Chemie [vgl. J. Chem. Soc., 7285 (1965)].

Die zur Durchführung des erfindungsgemäßen Verfahrens (B/c) als Ausgangsstoffe benötigten Alkohole sind durch die Formel (VII) allgemein definiert und sind allgemein bekannte Verbindungen der organischen Chemie.

Das erfindungsgemäße Verfahren (B/e) zur Herstellung der neuen 2-Cyclohexen-Derivate der Formel (IIb) wird vorzugsweise unter Verwendung von Verdünnungsmitteln durchgeführt.

Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie Acetonitril und Propionitril, Amide wie z.B. Dimethylformamid, Dimethylacetamid und N-Methylpyrrolidon sowie Dimethylsulfoxid, Tetramethylensulfon und Hexamethylphosphorsäuretriamid.

Als Inertgase kommen dabei Stickstoff sowie praktisch alle Edelgase, insbesondere Argon infrage.

Die Reaktionstemperaturen können bei dem Verfahren (B/e) zur Herstellung der neuen 2-Cyclohexen-Derivate der Formel (IIb) in einem größeren Bereich variiert werden.

Im allgemeinen arbeitet man bei Temperaturen zwischen -70 °C und +150 °C, vorzugsweise zwischen -50 °C und +100 °C.

Das Verfahren (B/e) zur Herstellung der neuen 2-Cyclohexen-Derivate der Formel (IIb) wird im allgemeinen bei Normaldruck durchgeführt. Unter bestimmten Voraussetzungen kann jedoch auch unter erhöhtem oder vermindertem Druck gearbeitet werden.

Zur Durchführung des Verfahrens (B/e) zur Herstellung der neuen 2-Cyclohexen-Derivate der Formel (IIb) setzt man im allgemeinen auf 1 Mol der 2-Cyclohexen-1-yl-amin-Derivate der Formel (IId) im allgemeinen 1 bis 5 Mol, vorzugsweise 1 bis 2 Mol der Alkanphosphonsäure-Derivate der Formel (VIII) ein.

Als Basen können bei dem erfindungsgemäßen Verfahren (B/e) alle üblicherweise für derartige Umsetzungen verwendbaren Säurebindemittel eingesetzt werden. Vorzugsweise infrage kommen Alkalimetallhydroxide wie z.B. Natrium- und Kaliumhydroxid, Erdalkalihydroxide wie z.B Calciumhydroxid, Alkalicarbonate und -alkoholate wie Natrium- und Kaliumcarbonat, Natrium- und Kalium-, Thalliummethylat bzw. -ethylat, Hydride wie zum Beispiel Natriumhydrid, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4.3.0]-non-5-en (DBN), 1,8-Diazabicyclo-[5.4.0]-undec-7-en (DBU) und 1,4-Diazabicyclo-[2.2.2]-octan (DABCO).

Die Reaktion werden im allgemeinen in einem geeigneten Verdünnungsmittel in Gegenwart eines Säureakzeptors durchgeführt und das Reaktionsgemisch wird mehrere Stunden bei der jeweils erfordlichen Temperatur gerührt. Die Aufarbeitung erfolgt bei dem erfindungsgemäßen Verfahren jeweils nach üblichen Methoden.

Die zur Durchführung des Verfahrens (B/e) zur Herstellung der neuen 2-Cyclohexen-Derivate der Formel (IIb) als Ausgangsstoffe benötigten Alkanphosphonsäure-Derivate sind durch die Formel (VIII) allgemein definiert und sind bekannt und/oder können nach literaturbekannten Verfahren in einfacher, analoger Weise hergestellt werden.

Die Verbindungen der Formel (Ia), (II) und (IIa) bis (IIe) können als Enantiomeren- oder Diastereomerengemische anfallen.

Die Erfindung umfaßt sowohl die reinen Isomeren als auch die Gemische. Diese Gemische von Diastereomeren können nach gebräuchlichen Methoden, zum Beispiel durch selektive Kristallisation, aus geeigneten Lösungsmitteln oder Chromatographie an Kieselgel oder Aluminiumoxid in die Komponenten aufgetrennt werden. Racemate können nach üblichen Methoden in die einzelnen Enantiomeren aufgetrennt werden, so zum Beispiel durch Salzbildung mit optisch aktiven Säuren wie Camphersulfonsäure oder Dibenzoylweinsäure und selektive Kristallisation, oder durch Derivatisierung mit geeigneten optisch aktiven Reagenzien, Trennung der diastereomeren Derivate und Rückspaltung oder Trennung an optisch aktivem Säulenmaterial.

Zur Herstellung von Säureadditions-Salzen der Verbindungen der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der allgemeinen Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der allgemeinen Formel (I) kommen vorzugsweise diejenigen Salze von Metallen infrage, die bereits weiter oben beschrieben wurden.

Die Metallsalz-Komplexe von Verbindungen der allgemeinen Formel (I) können in einfacher Weise nach üblichen Verfahren erhalten werden, so z.B. durch Lösen des Metallsalzes in Alkohol, z.B. Ethanol, und Hinzufügen zu Verbindungen der allgemeinen Formel (I). Man kann Metallsalz-Komplexe in bekannter Weise, z.B. durch Abfiltrieren, isolieren und gegebenenfalls durch Umkristallisation reinigen.

Die erfindungsgemäßen Wirkstoffe weisen eine starke biologische Wirkung auf und können zur Bekämpfung von unerwünschten Schädlingen praktisch eingesetzt werden. Die Wirkstoffe sind z. B. für den Gebrauch als Pflanzenschutzmittel einsetzbar, vor allem als Fungizide.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt:

Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;

Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäß verwendbaren Wirkstoffe mit besonders gutem Erfolg protektiv zur Bekämpfung von Phytophthora-Arten an Tomaten und Venturia-Arten bei Äpfeln eingesetzt werden.

Außerdem besitzen einige der erfindungsgemäß verwendbaren Wirkstoffe gegen Pythium-Arten, Alternaria-Arten und Cercospora-Arten eine gute Wirkung.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

<u>Dikotyle Unkräuter der Gattungen:</u> Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

<u>Dikotyle Kulturen der Gattungen:</u> Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

<u>Monokotyle Unkräuter der Gattungen:</u> Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

<u>Monokotyle Kulturen der Gattungen:</u> Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe eignen sich sehr gut zur selektiven Bekämpfung mono- und dikotyler Unkräuter in monokotylen Kulturen, insbesondere im Nachauflaufverfahren.

Die erfindungsgemäß verwendbaren Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N′-dimethyl-harnstoff (METABENZTHIAZURON) zur Unkrautbekämpfung in Getreide; 4-Amino-3-methyl-6-phenyl-1,2,4-triazin-5(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, in Frage. Ferner auch 2,4-Dichlorphenoxyessigsäure (2,4-D); 4-(2,4-Dichlorphenoxy)-buttersäure (2,4-DB); 2,4-Dichlorphenoxypropionsäure (2,4-DP); 3-Isopropyl-2,1,3-benzothiadiazin-4-on-2,2-dioxid (BENTAZON); Methyl-5-(2,4-dichlorphenoxy)-2-nitrobenzoat (BIFENOX); N,N-Dimethyl-N′-(3-chlor-4-methylphenyl)-harnstoff (CHLORTOLURON); 2-[4-(2,4-Dichlorphenoxy)-phenoxy]-propionsäure, deren Methyl- oder deren Ethylester (DICLOFOP); 4-Amino-6-t-butyl-3-ethylthio-1,2,4-triazin-5(4H)-on (ETHIOZIN); 2-{4-[(6-Chlor-2-benzoxazolyl)-oxy]-phenoxy}-propansäure, deren Methyl-oder deren Ethylester (FENOXAPROP); N,N-Dimethyl-N′-(3-trifluormethylphenyl)-harnstoff (FLUOMETURON); Methyl-2-[4,5-dihydro-4-methyl-4-(1-methylethyl)-5-oxo-1H-imidazol-2-yl]-4(5)-methylbenzoat (IMAZAMETHABENZ); (4-Chlor-2-methylphenoxy)-propionsäure (MCPP); N-(1-Ethylpropyl)-3,4-dimethyl-2,6-dinitroanilin (PENDIMETHALIN); O-(6-Chlor-3-phenyl-pyridazin-4-yl)-S-octyl-thiocarbonat (PYRIDATE); 4-Ethylamino-2-t-butylamino-6-methylthio-s-triazin (TERBUTRYNE) und 3,5,6-Trichlor-2-pyridyloxyessigsäure (TRICLOPYR) sind möglich.

Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

Herstellungsbeispiele:

Beispiel 1:

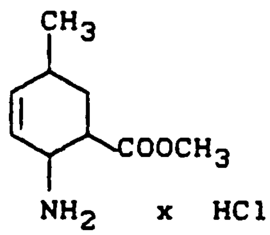

(I-1)

(Verfahren A)

Eine Lösung aus 10 g (0,05 Mol) 2-Carboxy-5-methyl-cyclohex-3-en-carbonsäuremethylester und 8 g (0,062 Mol) N,N-Diisopropyl-ethylamin in 30 ml Aceton wird bei -5 °C mit einer Lösung aus 5,4 g (0,05 Mol) Chlorameisensäureethylester in 15 ml Aceton über 30 Minuten versetzt. Nach weiteren 30 Minuten bei 0 °C tropft man eine eisgekühlte Lösung aus 6,5 g (0,1 Mol) Natriumazid in 15 ml Wasser zu. Man laßt 15 Minuten bei 0 °C rühren und arbeitet dann mit Wasser/Toluol auf.

Die nach dem Trocknen und Einengen auf ein Restvolumen von ca. 50 ml erhaltene organische Phase wird zu 50 ml siedendem Toluol getropft und der Reaktionsverlauf IR-spektroskopisch verfolgt. Nach vollständiger Umlagerung in das Isocyanat engt man ein, nimmt den Rückstand in 50 ml Tetrahydrofuran und 50 ml 1 N Salzsäure auf und rührt 10 Stunden bei 40 °C.

Nach vollständigem Einengen unter vermindertem Druck erhält man 2,8 g (30 % der Theorie) 4-Methyl-6-carbomethoxy-2-cyclohexen-1-yl-aminhydrochlorid.

$^1$H-NMR Daten*)(DMSO, 200 MHz): δ = 1,00(3H), 1,50-1,70(1H), 1,85-2,00 und 2,15-2,35(2H), 2,85-3,00(1H), 3,68(3H), 3,80-3,85(2H), 5,70-5,90(2H)

*) Die $^1$H-NMR-Spektren wurden in Dimethylsulfoxid (DMSO) mit Tetramethylsilan (TMS) als innerem Standard aufgenommen. Angegeben ist die chemische Verschiebung als δ-Wert in ppm.

Beispiel 2:

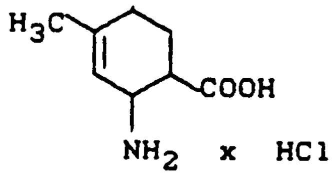

(I-2)

(Verfahren B)

2 g (7,8 mMol) tert-Butyl-(3-methyl-6-carboxy-2-cyclohexen-1-yl)-carbamat werden in 5 ml 1 n Salzsäure eingetragen. Nach 4 Stunden bei 50 ° C engt man zur Trockne ein und erhält 1,3 g (87 % der Theorie) 3-Methyl-6-carboxy-2-cyclohexen-1-yl-aminhydrochlorid als weißen Feststoff mit dem Schmelzpunkt 156-163 ° C.

In analoger Weise zu den in den Beispielen 1 und 2 beschriebenen Methoden und unter Berücksichtigung der Angaben in den Beschreibungen zu den erfindungsgemäßen Verfahren, werden die in der nachfolgenden Tabelle 1 aufgeführten Endprodukte der Formel (I)

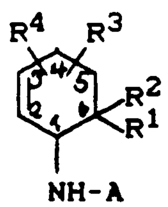

(I)

erhalten:

Tabelle 1

| Bsp-Nr. | $R^1$ | $R^2$ | $R^3$ | $R^4$ | A | physikalische Konstanten |
|---|---|---|---|---|---|---|
| I-3 | H | COOH | H | 4-$CH_3$ | H | Fp: 190-193° C<br>x HCl / einheitliches cis-Diastereomeres |
| I-4 | H | COOH | H | H | H | Fp: 169-172° C<br>x HCl / einheitliches cis-Diastereomeres |
| I-5 | H | $COOCH_3$ | H | H | H | $^1$H-NMR*) ($CDCl_3$, 200 MHz): δ= 1,65-2,20(6H), 2,57-2,68(1H), 3,60-3,70(2H), 3,71 (3H), 5,78(2H) |
| I-6 | H | COOH | 3,4-($CH_2$-$CH_2$-$CH_2$-$CH_2$)- | | H | Fp: 185-205° C<br>x HCl |
| I-7 | H | $COOCH_3$ | H | H | H | $^1$H-NMR*) ($CDCl_3$, 200MHz): δ= 1,90-2,4(4H), 3,03-3,17 (1H), 3,78(3H), 4,20(1H), 6,00(2H), 8,25-9,65(3H)<br>x HCl |
| I-8 | H | COOH | H | 4 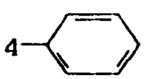 | H | Fp: 175-190° C<br>x HCl |
| I-9 | H | $COOCH_3$ | H | 4 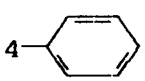 | H | $^1$H-NMR*) (DMSO, 200 MHz): δ= 2,75-2,85(1H), 3,30-3,43 (1H), 3,65 und 3,68(3H), 5,75-6,00(2H), 7,10-7,40(5H) |

<u>Tabelle 1</u> - Fortsetzung

| Bsp-Nr. | $R^1$ | $R^2$ | $R^3$ | $R^4$ | A | physikalische Konstanten |
|---|---|---|---|---|---|---|
| I-10 | H | $COOCH_2CH_2OCH_3$ | H | 4-$CH_3$ | H | $^1$H-NMR*) (DMSO, 200 MHz): δ= 1,05(3H), 3,30(3H), 5,75-5,95(2H), 8,10-8,50(3H) x HCl |
| I-11 | H | COOH | H | 4-$C_3H_7$ | H | Fp: 180-185° C x HCl |
| I-12 | Cl | COOH | H | 4-$CH_3$ | H | Fp: 208-214° C x HCl |
| I-13 | H | COOH | H | 4-$C_2H_5$ | H | Fp: 168-178° C x HCl |
| I-14 | H | COOH | H | 3-$C_2H_5$ | H | Fp: 140-150° C x HCl |
| I-15 | $CH_3$ | $COOCH_3$ | H | H | H | $^1$H-NMR*) (DMSO, 200 MHz): δ= 1,16 und 1,29(Isomere, 3H), 3,65(3H), 5,60-5,70 und 5,85-6,00(2H), 8,20-8,50 (3H) / x HCl |
| I-16 | H | COOH | H | 3-$C_4H_9$ | H | Fp: 180-188° C x HCl |
| I-17 | H | CN | H | 4-$CH_3$ | H | $^1$H-NMR (DMSO, 200 MHz): δ= 1,05(3H), 1,80-2,10 u. 2,20-2,40(3H), 3,48(1H), 3,95(1H), 5,65-5,95(2H), 8,60-9,00(3H) / x HCl |

Tabelle 1 - Fortsetzung

| Bsp-Nr. | $R^1$ | $R^2$ | $R^3$ | $R^4$ | A | physikalische Konstanten |
|---|---|---|---|---|---|---|
| I-18 | H | CN | H | $4-C_2H_5$ | H | Fp: 130-135° C<br>x HCl |
| I-19 | Cl | CN | H | $4-CH_3$ | H | 1H-NMR ($CDCl_3$, 200 MHz):<br>δ = 1,15(3H), 1,20-1,40 u.<br>1,85-2,55(5H), 3,60(1H),<br>5,60-5,85(2H) |
| I-20 | H | $NO_2$ | 5 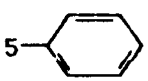 | $4-CH_3$ | H | Fp: 215-221° C<br>x HCl |
| I-21 | H | $-CH=CH-C=O$<br>\|<br>$OC_2H_5$ | H | $4-CH_3$ | H | $^1$H-NMR*) ($CDCl_3$, 200 MHz):<br>δ= 1,02(3H), 1,29(3H),<br>4,18(2H), 5,55-6,00(3H),<br>6,83-7,10(1H) |
| I-22 | H | -CH=CH-COOH | H | H | H | Fp: 186-218° C<br>x HCl |
| I-23 | H | $-CH=CH-C=O$<br>\|<br>$OC_2H_5$ | H | $4-CH_3$ | H | Fp: 191-214° C<br>x HCl |
| I-24 | H | $-CH_2-OH$ | H | $4-CH_3$ | H | Fp: 71-83° C<br>x HCl |

Tabelle 1 - Fortsetzung

| Bsp-Nr. | $R^1$ | $R^2$ | $R^3$ | $R^4$ | A | physikalische Konstanten |
|---|---|---|---|---|---|---|
| I-25 | H | 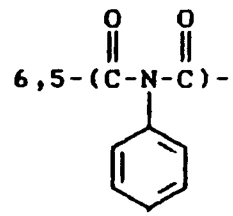 | | 4- 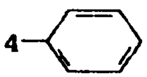 | H | Fp: 51-53° C<br>x $CF_3COOH$ |
| I-26 | H | 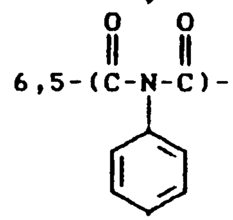 | | 4-$CH_3$ | H | Fp: 80-83° C<br>x $CF_3COOH$ |
| I-27 | H | 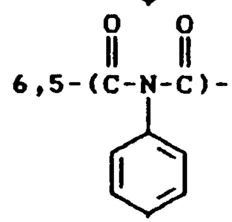 | | H | H | Fp: 75-81° C<br>x $CF_3COOH$ |
| I-28 | H | $-CH_2O-COCH_3$ | H | 4-$CH_3$ | H | $^1$H-NMR*) ($CDCl_3$, 200 MHz):<br>δ = 1,08(3H), 2,10(3H), 4,02-4,28(2H), 5,76-6,00 (2H), 8,10-8,55(3H)<br>x HCl |
| I-29 | H | $-CO-N(CH_3)_2$ | H | H | H | IR: 3500-3350, 1655 $cm^{-1}$ |
| I-30 | H | $-CO-N(CH_3)_2$ | H | H | H | IR: 1670 $cm^{-1}$<br>x $CF_3COOH$ |

<u>Tabelle 1</u> - Fortsetzung

| Bsp-Nr. | $R^1$ | $R^2$ | $R^3$ | $R^4$ | A | physikalische Konstanten |
|---|---|---|---|---|---|---|
| I-31 | H | $-CO-N(CH_3)_2$ | H | $4-CH_3$ | H | IR: 1670 $cm^{-1}$<br>x $CF_3COOH$ |
| I-32 | H | -CO-NH- 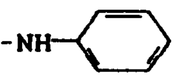 | H | $4-CH_3$ | H | Fp: 111-127° C |
| I-33 | H | $-CO-OCH(CH_3)_2$ | H | $4-CH_3$ | H | $^1$H-NMR*) ($CDCl_3$, 200 MHz):<br>δ= 1,05(3H), 1,26(6H),<br>5,08(1H), 5,53-5,80(2H) |
| I-34 | H | $-CO-CH_2-$ 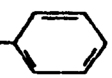 | H | $4-CH_3$ | H | $^1$H-NMR*) ($CDCl_3$, 200 MHz):<br>δ= 1,05(3H), 5,18(2H), 5,60-<br>5,85(2H), 7,25-7,40(5H) |
| I-35 | F | COOH | H | $4-CH_3$ | H | $^1$H-NMR*) ($CDCl_3$, 200 MHz):<br>δ= 1,16(3H), 1,80-2,60(3H),<br>3,83-4,00(1H), 5,65-5,78 u.<br>6,00-6,13(2H) |
| I-36 | H | $-COO-C_4H_9-n$ | H | $4-CH_3$ | H | $^1$H-NMR*) (DMSO, 200 MHz):<br>δ= 0,89(3H), 1,03(3H), 2,84-<br>2,98(1H), 4,00-4,20(2H),<br>5,70-5,90(2H), 8,10-8,40(3H) |
| I-37 | H | $-COOC_4H_9-n$ | H | $4-CH_3$ | H | IR: 3500-3410, 1735 $cm^{-1}$ |
| I-38 | H | $-COOC_2H_5$ | H | $4-CH_3$ | H | IR: 3500-3400, 1735 $cm^{-1}$ |

<u>Tabelle 1</u> - Fortsetzung

| Bsp-Nr. | $R^1$ | $R^2$ | $R^3$ | $R^4$ | A | physikalische Konstanten |
|---|---|---|---|---|---|---|
| I-39 | H | CN | H | $4-C_2H_5$ | H | $^1H$-NMR*) ($CDCl_3$, 200 MHz): δ= 0,92(3H), 1,27-1,70(5H), 1,90-2,10(2H), 2,78-2,88 (1H), 3,53-3,60(1H), 5,65-5,80(2H) |
| I-40 | H | $-CH_2OH$ | H | $4-CH_3$ | H | MS: m/z (rel.Int.): 141 (7), 99 (32), 83 (100) |
| I-41 | H | $-CH_2OH$ | H | H | H | $^1H$-NMR*) (DMSO, 200 MHz): δ= 1,30-2,10(5H), 5,63-5,82 u. 5,90-6,08(2H), 8,00-8,40 (3H) |
| I-42 | H | -COOH | H | $5-CH(CH_3)_2$ | H | Fp: 189-205°C / x HCl |
| I-43 | H | -COOH | H | $4-CH_3$ | H | Fp: 130-138°C Diastereomerenverhältnis 70:30/xHCl |
| I-44 | H | $6,5\text{-}(\underset{\underset{O}{\|}}{C}\text{-}O\text{-}CH_2\text{-}CH_2)\text{-}$ | | $4-CH_3$ | H | $^1H$-NMR*) ($CDCl_3$, 200 MHz): δ= 0,80(3H), 1,80-2,20 u. 2,30-2,50 (4H), 2,90-3,05 (1H), 4,20-4,60(2H), 6,05-6,20 u. 6,30-6,40(2H), 7,30 (1H)/ x HCl |
| I-45 | H | $-COOC_2H_5$ | $5-CH_3$ | H | H | $^1H$-NMR*) (DMSO, 200 MHz): δ= 0,93(3H), 1,21(3H), 2,55-2,65(1H), 4,10-4,25(2H), 5,70-6,63(2H), 8,10-8,30 (3H)/ x HCl |

Tabelle 1 - Fortsetzung

| Bsp-Nr. | $R^1$ | $R^2$ | $R^3$ | $R^4$ | A | physikalische Konstanten |
|---|---|---|---|---|---|---|
| I-46 | H | -COOH | H | $4-CH_3$ | H | Drehwert: $[\alpha]_D^{20} = +76.6$ ($C=0,3$, $H_2O$) x HCl Enantiomere zu I-3 |
| I-47 | H | -COOH | H | $4-CH_3$ | H | Drehwert: $[\alpha]_D^{20} = -84,1$ ($C=0,3$, $H_2O$) x HCl Enantiomere zu I-3 |
| I-48 | H | -CONH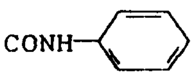 | H | $4-CH_3$ | H | Fp: 128-133° C x HCl |
| I-49 | H | $-COOCH(CH_3)_2$ | H | $4-CH_3$ | H | $^1$H-NMR*) ($CDCl_3$, 200 MHz): $\delta$= 1,15(3H), 1,25(6H), 2,71-2,85(1H), 5,00-5,18(1H), 5,87-6,08(2H), 8,30-8,70 (3H) / x HCl |
| I-50 | H | 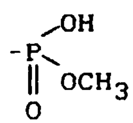 | H | $4-CH_3$ | H | MS (FAB): 205 [$M^+$] x HCl |
| I-51 | H | -COOH | H | $4-CH_3$ | H x $HO_2C-CH_2-CH-CO_2H$ (OH) | $^1$H-NMR (DMSO): $\delta$ = 1,00 (3H), 1,40 (m, 1H); 2,20-2,60 (6H); 3,75 (1H); 4,05 (1H); 5,60-5,90 (2H). |

Tabelle 1 - Fortsetzung

| Bsp-Nr. | $R^1$ | $R^2$ | $R^3$ | $R^4$ | A | physikalische Konstanten |
|---|---|---|---|---|---|---|
| I-52 | H | -COOH | H | 4-$CH_3$ | H x 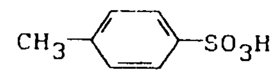 | $^1$H-NMR (DMSO): δ = 1,00 (3H); 1,50 (m, 1H); 2,25 (3H); 2,80 (1H); 5,60-5,90 (2H); 7,10 (2H); 7,50 (2H). |
| I-53 | H | -COOH | H | 4-$CH_3$ | H x $CuSO_4$ | Fp.: 210-219°C (Zers.) |
| I-54 | H | -COOH | H | 4-$CH_3$ | H x HBr | $^1$H-NMR (DMSO): δ = 1,00 (3H), 1,50 (1H); 2,80 (1H); 5,60-5,90 (2H); 7,90 (br, 3H). |
| I-55 | H | -COOH | H | 4-$CH_3$ | H x $CH_3CO_2H$ | $^1$H-NMR (DMSO): δ = 0,95 (3H); 1,85 (3H); 5,60-5,80 (2H). |
| I-56 | H | -COOH | H | 4-$CH_3$ | H x HCl | Isomer zu I-3 $^1$H-NMR ($CD_3OD$): δ = 1,06 (3H); 1,83 (1H); 2,00 (1H); 2,38 (1H); 2,81 (1H); 4,08 (1H); 5,60 (1H); 6,00 (1H). |
| I-57 | H | -$CH_2$-OH | H | 4-$CH_3$ | H x HCl | $^1$H-NMR (DMSO): δ = 1,00 (3H); 3,45 (2H); 5,70-5,90 (2H); 8,06 (3H). |
| I-58 | H | -COOH | H | 4-$CH_3$ | H | (Zwitterion) Fp.: 208-211°C |

Tabelle 1 - Fortsetzung

| Bsp-Nr. | $R^1$ | $R^2$ | $R^3$ | $R^4$ | A | physikalische Konstanten |
|---|---|---|---|---|---|---|
| I-59 | H | $-CO_2CH_3$ | H | 4-$CH_3$ | H x 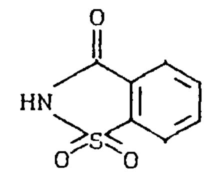 | stark hygroskopisch |
| I-60 | H | $-CO_2CH_3$ | H | 4-$CH_3$ | H x $HO_2CCH_2C(OH)-(CO_2H)CH_2CO_2H$ | hygroskopisch |
| I-61 | H | $-CO_2CH_3$ | H | 4-$CH_3$ | H x $HO_2CCO_2H$ | Fp.: 152-158° C |
| I-62 | H | $-CO_2CH_3$ | H | 4-$CH_3$ | H x $HO_3S$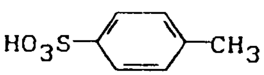$CH_3$ | Fp.: 125-128° C |
| I-63 | H | $-CO_2CH_3$ | H | 4-$CH_3$ | H x $CH_3CO_2H$ | Fp.: 89,5-90° C |
| I-64 | H | $-CO_2(CH_2)_2OCH_3$ | H | 4-$CH_3$ | H | $n_D^{20}$ 1,4763 |
| I-65 | H | $-CO_2CH_3$ | H | 4-$CH_3$ | H | $n_D^{20}$ 1,4791 |
| I-66 | H | $-CO_2H$ | H | 4-$CH_3$ | H x 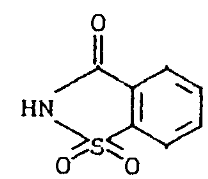 | $^1$H-NMR (DMSO): $\delta$ = 1,00 (3H); 1,50 (1H); 2,00 (1H); 2,30 (1H); 2,85 (1H); 3,90 (1H); 5,60-5,95 (2H); 7,61 (5H). |

Tabelle 1 - Fortsetzung

| Bsp-Nr. | $R^1$ | $R^2$ | $R^3$ | $R^4$ | A | physikalische Konstanten |
|---|---|---|---|---|---|---|
| I-67 | H | $-CO_2H$ | H | $4-CH_3$ | H x $HO_2CCO_2H$ | $^1H$-NMR (DMSO): $\delta$ = 1,05 (3H), 1,45 (1H); 2,05 (1H), 2,31 (1H); 2,85 (1H); 3,80 (1H); 5,60-5,80 (2H). |
| I-68 | H | $-CO_2(CH_2)_3CH_3$ | H | $4-CH_3$ | H x 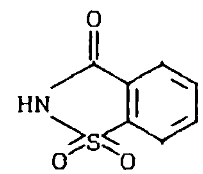 | wachsartiger Feststoff |
| I-69 | H | $-CO_2CH_2CH(CH_3)_2$ | H | $4-CH_3$ | H | $^1H$-NMR ($CDCl_3$): $\delta$ = 0,90-1,08 (9H); 2,60-2,73 (1H); 3,90 (2H); 5,60-5,80 (2H). |
| I-70 | H | $-CO_2CH_2CH(CH_3)_2$ | H | $4-CH_3$ | H x HCl | $^1$-NMR (DMSO): $\delta$ = 0,85-1,05 (9H); 2,90-3,00 (1H); 3,90 (2H); 5,70-5,90 (2H); 5,20 (3H). |
| I-71 | H | $-CO_2H$ | H | $4-CH_3$ | H x $Cu(OAc)_2$ | MS (FAB): 155 [$M^+$ $-Cu(Ac)_2$] |
| I-72 | H | $-CO_2H$ | H | $4-CH_3$ | H x $HO_2CCH_2CO_2H$ | MS (FAB): 259 [$M^+$] |
| I-73 | H | $-CO_2H$ | H | $4-CH_3$ | H x $HO_2C-CH=CH-CH=CH-CH_3$ | MS (FAB): 267 [$M^+$] |
| I-74 | H | $-CO_2H$ | H | $CH(CH_3)_2$ | H x HCl | Fp.: 203-206°C (Zers.) |

Tabelle 1 - Fortsetzung

| Bsp-Nr. | $R^1$ | $R^2$ | $R^3$ | $R^4$ | A | physikalische Konstanten |
|---|---|---|---|---|---|---|
| I-75 | H | $-CO_2H$ | H | H | Zwitterion | Fp.: 98° C |
| I-76 | H | $-CO_2H$ | $3-CH_3$ | $4-CH_3$ | H x HCl | Fp.: 133-135° C |
| I-77 | H | $-CO_2H$ | $3-C_6H_5$ | $4-CH_2CH(CH_3)_2$ | H x HCl | Fp.: 140-145° C |
| I-78 | H | $-CO_2^-Na^+$ | H | $4-CH_3$ | H | Fp.: >250° C (Zersetzung) |
| I-79 | H | $-CO_2^-NH_4^+$ | H | $4-CH_3$ | H | Fp.: >250° C (Zersetzung) |
| I-80 | H | $-CO_2CH_3$ | $3-C_6H_5$ | $4-CH_2CH(CH_3)_2$ | H | $^1H$-NMR ($CDCl_3$): $\delta$ = 0,75-0,95 (9H); 2,65-2,85 (1H); 3,75 (3H); 5,90 (1H); 7,15-7,40 (5H). |
| I-81 | H | $-CO_2H$ | H | H | H x $HO_2CCO_2H$ | Fp.: 147-150° C (Zersetzung) |
| I-82 | H | $-CO_2H$ | H | H | H x 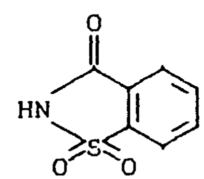 | Fp.: 106-112° C (Zersetzung) |
| I-83 | H | $-CO_2H$ | $3-C_6H_5$ | $4-CH(CH_3)_2$ | H x HCl | Fp.: 245-248° C |
| I-84 | H | $-CO_2H$ | $3-CH(CH_3)_2$ | $4-CH_2CH(CH_3)_2$ | H x HCl | Fp.: 210-218° C |
| I-85 | H | -CN | $3-CH(CH_3)_2$ | $4-CH_2CH(CH_3)_2$ | H x HCl | Fp.: 164° C |
| I-86 | H | -CN | $3-C_6H_5$ | $4-CH(CH_3)_2$ | H x HCL | Fp.: 233-261° C |

Tabelle 1 - Fortsetzung

| Bsp-Nr. | $R^1$ | $R^2$ | $R^3$ | $R^4$ | A | physikalische Konstanten |
|---|---|---|---|---|---|---|
| I-87 | H | $-CO_2C_2H_5$ | H | H | H x HCl | Fp.: 139-148° C |
| I-88 | H | $-CO_2CH(CH_3)_2$ | H | H | H x HCl | Fp.: 188-188,5° C |
| I-89 | H | $-CO_2C_6H_5$ | H | $4-CH_3$ | H | Fp.: 117° C |
| I-90 | H | $-CO_2C_6H_5$ | H | $4-CH_3$ | H x HCl | $^1$H-NMR (DMSO) δ = 1,00 (3H); 1,40 (1H); 2,80 (1H); 5,65-5,95 (2H); 7,20 (5H). |
| I-91 | H | $-CO_2CH_3$ | H | $4-CH(CH_3)_2$ | H x HCl | MS: 197 [$M^+$ - HCl] |
| I-92 | H | $-CO_2$ 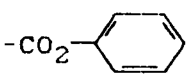 | H | $4-CH_3$ | H x 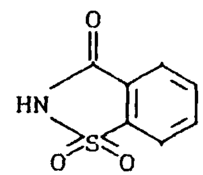 | MS: 231 [$M^+$ - 183] |
| I-93 | H | $-CO_2H$ | H | H | H x HCl | (Enantiomer) Fp.: 210-213,5° C |
| I-94 | H | $-CO_2H$ | H | H | H x HCl | (Enantiomer) Fp.: 208-210° C |
| I-95 | H | $-CO_2H$ | H | 4- 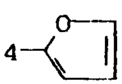 | H x HCl | MS: $M^+$ von 207 durch FAB-Spektroskopie |
| I-96 | H | $-CO_2CH_3$ | H | H | H x HCl | (Enantiomer) MS (FAB): 191 [$M^+$] |
| I-97 | H | $-CO_2CH_3$ | H | H | H x HCl | (Enantiomer) MS (FAB): 191 [$M^+$] |

<u>Tabelle 1</u> - Fortsetzung

| Bsp-Nr. | $R^1$ | $R^2$ | $R^3$ | $R^4$ | A | physikalische Konstanten |
|---|---|---|---|---|---|---|
| I-98 | H | $CO_2CH_2-C_6H_5$ | H | H | H | $^1$H-NMR ($CDCl_3$): δ = 1,50 (2H); 2,60-2,70 (1H); 3,67 (1H); 5,15 (2H); 5,75 (2H); 7,32 (5H). |
| I-99 | H | $CO_2CH_2$-2,4-$Cl_2C_6H_3$ | H | 4-$CH_3$ | H | $^1$H-NMR ($CDCl_3$): δ = 1,05 (d, 3H); 2,73-2,85 (1H); 5,22 (2H); 5,78 (2H); 7,22-7,40 (3H). |
| I-100 | H | $CO_2CH_2-C_6H_5$ | H | H | H x HCl | MS: 231 [$M^+$ - 36] |
| I-101 | H | $CO_2CH_2$-2,4-$Cl_2C_6H_3$ | H | 4-$CH_3$ | H x HCl | MS: 313 [$M^+$ - 36] |
| I-102 | H | $CO_2CH_2$-2-$ClC_6H_4$ | H | 4-$CH_3$ | H | $^1$H-NMR ($CDCl_3$): δ = 1,05 (3H); 2,60 (2H); 2,65-2,77 (1H); 5,26 (2H); 5,56-5,80 (2H); 7,20-7,50 (4H). |
| I-103 | H | $CO_2CH_2$-3-$ClC_6H_4$ | H | 4-$CH_3$ | H | $^1$H-NMR ($CDCl_3$): δ = 1,06 (3H); 2,68-2,80 (1H); 3,77 (1H); 5,15 (2H); 5,60-5,80 (2H); 7,3 (4H). |
| I-104 | H | $CO_2CH_2$-4-$ClC_6H_4$ | H | 4-$CH_3$ | H | $^1$H-NMR ($CDCl_3$): δ = 1,05 (3H); 2,65-2,78 (1H); 5,15 (2H); 5,60-5,80 (2H); 7,32 (4H). |
| I-105 | H | $CO_2CH_2$-4-$NO_2C_6H_4$ | H | 4-$CH_3$ | H | Fp.: 118-124°C |
| I-106 | H | $CO_2CH_2$-2-$ClC_6H_4$ | H | 4-$CH_3$ | H x HCl | MS: 279 [$M^+$ - 36] |

Tabelle 1 - Fortsetzung

| Bsp-Nr. | $R^1$ | $R^2$ | $R^3$ | $R^4$ | A | physikalische Konstanten |
|---|---|---|---|---|---|---|
| I-107 | H | $CO_2CH_2$-3-$ClC_6H_4$ | H | 4-$CH_3$ | H x HCl | MS: 279 [$M^+$ -36] |
| I-108 | H | $CO_2CH_2$-4-$ClC_6H_4$ | H | 4-$CH_3$ | H x HCl | Fp.: 167° C |
| I-109 | H | $CO_2CH_2$-4-$NO_2C_6H_4$ | H | 4-$CH_3$ | H x HCl | Fp.: 196-202° C |
| I-110 | H | $CO_2CH_2$-2-$ClC_6H_4$ | H | H | H | $^1$H-NMR ($CDCl_3$): δ = 1,80-2,15 (4H); 2,65-2,85 (3H); 5,29 (2H); 5,78 (2H); 7,20-7,50 (4H). |
| I-111 | H | $CO_2CH_2$-3-$ClC_6H_4$ | H | H | H | $^1$H-NMR ($CDCl_3$): δ = 1,75-2,15 (4H), 2,56 (2H); 2,65-2,75 (1H); 5,15 (2H); 5,79 (2H); 7,20-7,40 (4H). |
| I-112 | H | $CO_2CH_2$-4-$ClC_6H_4$ | H | H | H | Fp.: 142° C |
| I-113 | H | $CO_2CH_2$-4-$NO_2C_6H_4$ | H | H | H | $^1$H-NMR ($CDCl_3$): δ = 1,75-2,15 (6H); 5,28 (2H); 5,78 (2H); 7,55 (2H); 8,20 (2H). |
| I-114 | H | $CO_2CH_2$-2-$ClC_6H_4$ | H | H | H x HCl | $^1$H-NMR (DMSO): δ = 1,95-2,12 (4H); 5,15-5,35 (2H); 5,65-6,05 (2H); 7,30-7,60 (4H); 8,25 (3H). |
| I-115 | H | $CO_2CH_2$-3-$ClC_6H_4$ | H | H | H x HCl | $^1$H-NMR (DMSO): δ = 1,95-2,12 (4H); 3,10 (1H); 5,19 (2H); 7,39 (4H); 8,25 (3H). |
| I-116 | H | $CO_2CH_2$-4-$ClC_6H_4$ | H | H | H x HCl | MS: 265 [$M^+$ - 36] |

Tabelle 1 - Fortsetzung

| Bsp-Nr. | $R^1$ | $R^2$ | $R^3$ | $R^4$ | A | physikalische Konstanten |
|---|---|---|---|---|---|---|
| I-117 | H | $CO_2CH_2$-4-$NO_2C_6H_4$ | H | H | H | $^1$H-NMR (DMSO): δ = 1,90-2,15 (4H); 5,33 (2H); 5,70-6,05 (2H); 7,70 (2H); 8,20 (2H); 8,45 (3H). |
| I-118 | H | $CO_2CH_2$-3,5-$(OCH_3)_2C_6H_3$ | H | 4-$CH_3$ | H | $^1$H-NMR ($CDCl_3$): δ = 1,03 (3H); 3,79 (6H); 5,10 (2H); 5,58-5,80 (2H); 6,40-6,55 (3H). |
| I-119 | H | $CO_2CH_2$-2-$NO_2C_6H_4$ | H | 4-$CH_3$ | H | $^1$H-NMR ($CDCl_3$): δ = 1,05 (3H); 5,56 (2H); 5,60-5,85 (2H); 7,45-7,70 (3H); 8,10 (1H). |
| I-120 | H | $CO_2CH_2$-2,4-$Cl_2C_6H_3$ | H | H | H | $^1$H-NMR ($CDCl_3$): δ = 2,65-2,78 (1H); 2,70 (1H); 5,21 (2H); 5,78 (2H); 7,20-7,35 (3H). |
| I-121 | H | $CO_2CH_2$-3-$NO_2C_6H_4$ | H | H | H | MS: 276 [$M^+$] |
| I-122 | H | $CO_2CH_2$-3,5-$(OCH_3)_2C_6H_3$ | H | H | H | $^1$H-NMR ($CDCl_3$): δ = 3,79 (6H); 5,10 (2H); 5,80 (2H); 6,40-6,60 (3H). |
| I-123 | H | $CO_2CH_2$-3-$NO_2C_6H_4$ | H | 4-$CH_3$ | H | $^1$H-NMR ($CDCl_3$): δ = 1,05 (3H); 2,68-2,80 (1H); 5,28 (2H); 5,55-5,80 (2H); 7,50-7,75 (2H); 8,15-8,30 (2H). |

Tabelle 1 - Fortsetzung

| Bsp-Nr. | $R^1$ | $R^2$ | $R^3$ | $R^4$ | A | physikalische Konstanten |
|---|---|---|---|---|---|---|
| I-124 | H | $CO_2CH_2$-$C_6H_5$ | H | 4-$CH(CH_3)_2$ | H | $^1H$-NMR ($CDCl_3$): $\delta$ = 0,90 (6H); 2,60-2,70 (1H); 5,17 (2H); 5,60-5,85 (2H); 7,35 (5H). |
| I-125 | H | $CO_2CH_2$-3,5-$(OCH_3)_2C_6H_3$ | H | 4-$CH_3$ | H x HCl | $^1H$-NMR (DMSO): $\delta$ = 8,29 ($-NH_3^{\oplus}$, 3H). |
| I-126 | H | $CO_2CH_2$-2-$NO_2C_6H_4$ | H | 4-$CH_3$ | H x HCl | $^1H$-NMR (DMSO): $\delta$ = 8,35 ($-NH_3^{\oplus}$, 3H). |
| I-127 | H | $CO_2CH_2$-2,4-$Cl_2C_6H_3$ | H | H | H x HCl | $^1H$-NMR (DMSO): $\delta$ = 8,25 ($-NH_3^{\oplus}$, 3H). |
| I-128 | H | $CO_2CH_2$-3-$NO_2C_6H_4$ | H | H | H x HCl | $^1H$-NMR (DMSO): $\delta$ = 8,40 ($-NH_3^{\oplus}$, 3H). |
| I-129 | H | $CO_2CH_2$-3,5-$(OCH_3)_2C_6H_3$ | H | H | H x HCl | MS: 291 [$M^+$ - 36] |
| I-131 | H | $CO_2CH_2C_6H_5$ | H | 4-$CH(CH_3)_2$ | H x HCl | $^1H$-NMR (DMSO): $\delta$ = 0,85 (6H); 1,55-2,10 (4H); 5,20 (2H); 5,80-6,00 (2H); 7,38 (5H); 8,35 (3H). |
| I-132 | H | $CO_2CH_2$-3,5-$Cl_2C_6H_3$ | H | H | H | $^1H$-NMR ($CDCl_3$): $\delta$ = 1,75-2,15 (6H); 2,65-2,80 (1H); 5,22 (2H); 5,78 (2H); 7,20-7,50 (3H). |
| I-133 | H | $CO_2CH_2$-2,6-$Cl_2C_6H_3$ | H | H | H | $^1H$-NMR ($CDCl_3$): $\delta$ = 1,75-2,15 (6H); 5,40 (2H); 5,75 (2H); 7,20-7,40 (3H). |

Tabelle 1 - Fortsetzung

| Bsp-Nr. | $R^1$ | $R^2$ | $R^3$ | $R^4$ | A | physikalische Konstanten |
|---|---|---|---|---|---|---|
| I-134 | H | $CO_2CH_2$-3,5-$Cl_2C_6H_3$ | H | H | H x HCl | $^1$H-NMR (DMSO): $\delta$ = 8,43 ($-NH_3^{\oplus}$, 3H). |
| I-135 | H | $CO_2CH_2$-2,6-$Cl_2C_6H_3$ | H | H | H x HCl | $^1$H-NMR (DMSO): $\delta$ = 8,25 ($-NH_3^{\oplus}$, 3H). |

*) Die $^1$H-NMR-Spektren wurden in Deuterochloroform ($CDCl_3$) bzw. Dimethylsulfoxid (DMSO) mit Tetramethylsilan (TMS) als innerem Standard aufgenommen. Angegeben ist die chemische Verschiebung als $\delta$-Wert in ppm.

Herstellung der Ausgangsstoffe:

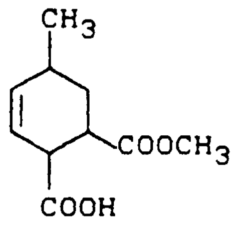

(II)

Eine Lösung aus 10 g (0,089 Mol) Sorbinsäure, 24,1 ml (0,27 Mol) Acrylsäuremethylester, 0,1 g (0,6 mMol) 4-tert-Butylcatechol und 100 ml Dioxan wird 30 Stunden bei einer Temperatur von 110 °C bei 5 bar umgesetzt. Man destilliert das Dioxan ab und trennt mit dem Laufmittelgemisch Petrolether-Essigsäureethylester (2 : 1) von den polaren Nebenprodukten ab.
Man erhält 13,2 g (75 % der Theorie) 2-Carboxy-5-methylcyclohex-3-en-carbonsäuremethylester als Isomerengemisch.
$^1$H-NMR*) ($CDCl_3$, 200 MHz): $\delta$ = 1,05 (d, 3H); 3,65 (s, 3H), 5,40-5,90 (m, 2H).

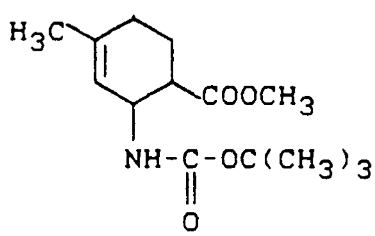

(IIb-1)

(Verfahren B/a)

Eine Lösung von 20,5 g (0,11 Mol) tert-Butyl-(trans-3-methyl-1,3-butadien-1)-carbamat, 34 g (0,39 Mol) Acrylsäuremethylester, 1 g (6 mMol) 4-tert-Butylcatechol und 90 ml Dioxan wird bei 110 °C und 6 bar 20 Stunden umgesetzt. Nach Abdestillieren des überschüssigen Acrylsäuremethylesters trennt man mit dem Laufmittelgemisch Petrolether-Essigsäureethylester (5 : 1) an Kieselgel.

Man erhält 24 g (80 % der Theorie) tert-Butyl-(3-methyl-6-carbomethoxy-2-cyclohexen-1-yl)-carbamat als wachsartigen Feststoff.
$^1$H-NMR*) ($CDCl_3$, 200 MHz): $\delta$ = 1,42(9H), 1,68(3H), 1,70-2,00(4H), 2,70(1H), 3,68(3H), 4,50(1H), 4,80(1H), 5,30-5,45(1H)

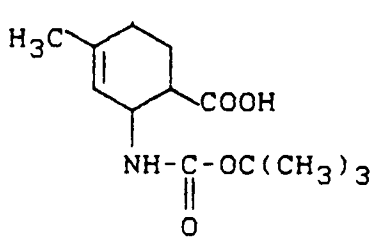

(IIb-2)

5 g (18,6 mMol) tert-Butyl-(3-methyl-6-carbomethoxy-2-cyclohexen-1-yl)-carbamat werden in 40 ml 1 n Natronlauge eingetragen und bei 50 °C so lange gerührt, bis die Lösung klar geworden ist. Man extrahiert einmal mit Diethylether und stellt dann bei 0 °C mit konzentrierter Salzsäure den pH-Wert von 1 ein. Nach Extraktion mit Diethylether und Einengen erhält man 3,5 g (74 % der Theorie) tert-Butyl-3-methyl-6-carboxy-2-cyclohexen-1-yl-carbamatmit dem Schmelzpunkt 132-136 °C.

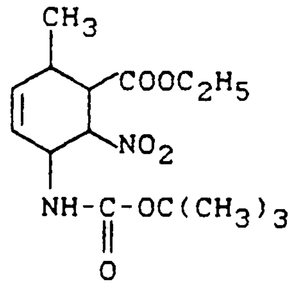

(IIb-3)

(Verfahren B/a)

Zu einer Lösung von 20,1 g (0,11 Mol) tert-Butyl-trans-1,3-pentadien-1-carbamat und 0,25 g (1,5 mMol) 4-tert-Butylcatechol in 50 ml Benzol tropft man bei Raumtemperatur eine Lösung aus 18,8 g (0,13 Mol) trans-3-Nitroacrylsäureethylester in 100 ml Benzol zu und läßt 20 Stunden bei Raumtemperatur rühren. Man engt auf die Hälfte des Volumens ein ,und läßt bei +4°C auskristallisieren.
Man erhält 18,3 g (53 % der Theorie) an 3-(N-tert-Butyloxycarbonylamino)-2-nitro-6-methyl-4-cyclohexencarbonsäureethylester vom Schmelzpunkt 139-45°C.

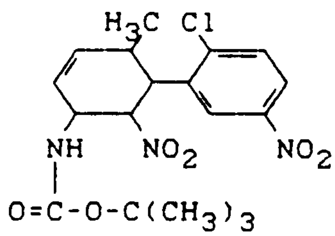

(IIb-4)

(Verfahren B/a)

10 g (55 mMol) tert-Butyl-(trans-1,3-pentadien-1)-carbamat, 12,4 g (55 mMol) trans-2-(2-Chlor-5-nitrophenyl)-nitroethen und 0,6 g (3,6 mMol) 4-tert-Butylcatechol werden in 100 ml Dioxan 30 Stunden bei 110°C und 4 bar umgesetzt. Das Reaktionsgemisch wird eingeengt und zweimal aus Ethanol umkristallisiert.

Man erhält 7 g (31 % der Theorie) tert-Butyl-4-methyl-5-(2-chlor-5-nitro-phenyl)-6-nitro-2-cyclohexen-1-yl-carbamat vom Schmelzpunkt 195-203°C.

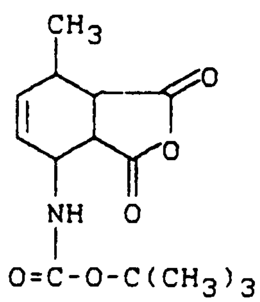

(IIb-5)

(Verfahren B/a)

Eine Lösung aus 1 g (5,5 mMol) tert-Butyl-trans-1,3-pentadien-1-carbamat, 1,1 g (11 mMol) Maleinsäureanhydrid, 40 mg (0,2 mMol) 4-tert-Butylcatechol und 3 ml Dioxan wird 2 Stunden auf 100°C erhitzt.

Man engt zur Trockne ein und erhält nach dem Umkristallisieren aus Benzol 0,8 g (52 % der Theorie) tert-Butyl-(4-methyl-cyclohex-2-en-5,6-dicarbonsäureanhydrid-1-yl)-carbamat mit einem Schmelzpunkt von 180-182 °C.

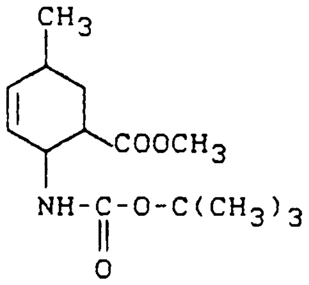

(IIb-6)

(Verfahren B/c)

Eine Lösung aus 10 g (0,05 Mol) 2-Carboxy-5-methylcyclohex-3-en-carbonsäuremethylester und 8 g (0,062 Mol) N,N-Diisopropylethylamin in 30 ml Aceton wird bei -5 °C mit einer Lösung aus 5,4 g (0,05 Mol) Chlorameisensäureethylester in 15 ml Aceton über 30 Minuten versetzt. Nach weiteren 30 Minuten bei 0 °C tropft man eine eisgekühlte Lösung aus 6,5 g (0,1 Mol) Natriumazid in 15 ml Wasser zu. Man läßt 15 Minuten bei 0 °C rühren und arbeitet dann mit Wasser-Toluol auf.
Die auf ca. 50 ml eingeengte Toluol-Phase wird dann zu einer unter Rückfluß siedenden Lösung aus 3 g (0,04 Mol) tert-Butanol, 25 mg (0,15 mMol) tert-Butylcatechol in 20 ml Toluol getropft. Der Reaktionsverlauf wird IR-spektroskopisch verfolgt.
Man läßt auf Raumtemperatur abkühlen und engt ein. Nach säulenchromatographischer Trennung an Kieselgel mit dem Laufmittel Petrolether-Essigsäureethylester (6 : 1) erhält man 4 g (30 % der Theorie) tert-Butyl-(4-methyl-6-carbomethoxy-2-cyclohexen-1-yl)-carbamat vom Schmelzpunkt 89-91 °C.

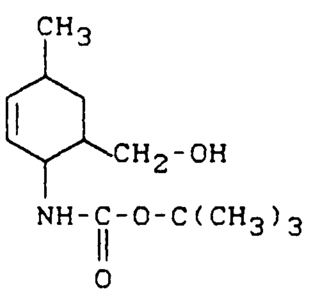

(IIb-7)

(Verfahren B/d)

5 g (21 mMol) tert-Butyl-(4-methyl-6-formyl-2-cyclohexen-1-yl)-carbamat werden in 70 ml Tetrahydrofuran gelöst, nach Zugabe von 1,6 g ( 42 mMol) Natriumborhydrid läßt man 15 Minuten bei 55 °C rühren. Dann werden langsam 17 ml Methanol bei 55 °C zugetropft und nach beendeter Zugabe läßt man 3 Stunden bei Raumtemperatur nachrühren. Man arbeitet wässrig auf, extrahiert mit Diethylether und erhält nach Trocknen über Magnesiumsulfat und Einengen 4,8 g (95 % der Theorie) 2-[(tert-Butyloxycarbonylamino)-5-methyl-3-cyclohexen-1-yl]-methanol als weißen, wachsartigen Feststoff.
$^1$H-NMR*) ($CDCl_3$, 200 MHz): $\delta$ = 1,00(d, 3H), 1,45(s, 9H), 1,75-2,40(m, 2H), 3,28-3,52 und 3,67-3,80(m, 2H), 4,00-4,30 und 4,45-4,75 (m, 2H), 5,43-5,51 und 5,63-5,78 (m, 2H).

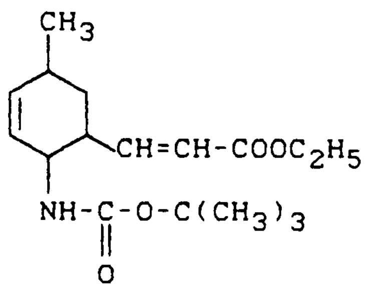

(IIb-8)

(Verfahren B/e)

Man tropft bei 0 ° C 24 g (0,105 Mol) Phosphonoessigsäuretriethylester zu einer Suspension von 3,4 g ( 80 % in Öl = 0,1 Mol) Natriumhydrid. Nach Beendigung der Wasserstoffentwicklung tropft man eine Lösung von 25 g (0,105 Mol) tert-Butyl-(4-methyl-6-formyl-2-cyclohexen-1-yl)-carbamat in 30 ml Tetrahydrofuran zu. Nach 4-stündigem Rühren bei Raumtemperatur gibt man die Reaktionsmischung zu 500 ml Wasser und extrahiert mehrmals mit Essigsäureethylester. Die vereinigten organischen Phasen werden mit Wasser und gesättigter Natriumchloridlösung gewaschen, getrocknet und eingeengt. Nach säulenchromatographischer Reinigung an Kieselgel mit dem Laufmittel Petrolether-Essigsäureethylester (5 : 1) werden 26 g (80 % der Theorie) an trans-(2-N-tert-Butoxycarbonylamino-4 methyl-cyclohex-2-en)-1-yl)-acrylsäureethylester erhalten.

$^1$H-NMR*⁾(CDCl$_3$, 200 MHz): $\delta$ = 1,05(d, 3H), 1,20-1,35(m, 3H), 1,43(s,9H), 1,60-2,60(m, 4H), 4,15(q, 2H), 4,25-4,60(br.m, 2H), 5,50-5,95(m, 3H), 6,86-7,10 (m, 1H).

In analoger Weise zu den in den Beispielen (IIb-1) bis (IIb-8) beschriebenen Methoden und unter Berücksichtigung der Angaben in den Beschreibungen zu den erfindungsgemäßen Verfahren, werden die in der nachfolgenden Tabelle 2 aufgeführten Vorprodukte der Formel (IIb)

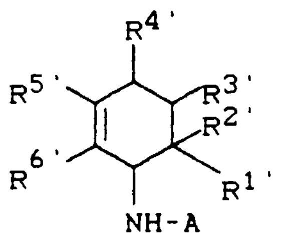

(IIb)

*⁾ Die $^1$H-NMR-Spektren wurden in Deuterochloroform (CDCl$_3$) mit Tetramethylsilan (TMS) als innerem Standard aufgenommen. Angegeben ist die chemische Verschiebung als $\delta$-Wert in ppm.

erhalten:

**Tabelle 2**

| Bsp-Nr. | A | $R^{1'}$ | $R^{2'}$ | $R^{3'}$ | $R^{4'}$ | $R^{5'}$ | $R^{6'}$ | physikalische Konstanten |
|---|---|---|---|---|---|---|---|---|
| IIb-9 | $-\underset{\overset{\|}{O}}{C}-OC(CH_3)_3$ | H | -COOH | H | H | H | H | Fp: 110-118° C |
| IIb-10 | $-\underset{\overset{\|}{O}}{C}-OC(CH_3)_3$ | H | -COOH | H | $CH_3$ | H | H | Fp: 194-196° C |
| IIb-11 | $-\underset{\overset{\|}{O}}{C}-OC(CH_3)_3$ | H | $-COOCH_3$ | H | H | H | H | $^1$H-NMR*) ($CDCl_3$, 200 MHz) δ= 1,42(9H), 1,70-2,10(4H), 2,73-2,85(1H), 3,68(3H), 4,48-4,59(1H), 4,85-4,95 (1H), 5,60-5,86(2H) |
| IIb-12 | $-\underset{\overset{\|}{O}}{C}-OC(CH_3)_3$ | H | $-COOCH_3$ | H | 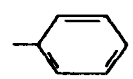 | H | H | $^1$H-NMR*) ($CDCl_3$, 200 MHz) δ= 1,44(9H), 1,58-1,78(1H), 2,08-2,20(1H), 3,30-3,40(1H), 3,62(3H), 4,60-4,80(2H), 6,90(2H), 7,12-7,35(5H) |
| IIb-13 | $-\underset{\overset{\|}{O}}{C}-OC(CH_3)_3$ | H | $-COOCH_3$ | H | $-CH_2-(CH_2)_2-CH_2-$ | | H | $^1$H-NMR*) ($CDCl_3$, 200 MHz) δ= 0,80-2,30(20H), 2,60-2,71(1H), 3,65(3H), 4,50(2H), 5,43(1H) |
| IIb-14 | $-\underset{\overset{\|}{O}}{C}-OC(CH_3)_3$ | H | -COOH | H | $-CH_2-(CH_2)_2-CH_2-$ | | H | Fp: 160-168° C |

Tabelle 2 - Fortsetzung

| Bsp-Nr. | A | $R^{1'}$ | $R^{2'}$ | $R^{3'}$ | $R^{4'}$ | $R^{5'}$ | $R^{6'}$ | physikalische Konstanten |
|---|---|---|---|---|---|---|---|---|
| IIb-15 | $-\underset{\underset{O}{\Vert}}{C}-OC(CH_3)_3$ | $CH_3$ | $-COOCH_3$ | H | H | H | H | $^1H$-NMR*) ($CDCl_3$, 200 MHz) δ= 1,18(3H), 1,44(9H), 3,68(3H), 5,50-5,80(2H) |
| IIb-16 | $-\underset{\underset{O}{\Vert}}{C}-OC(CH_3)_3$ | H | $-CONH_2$ | H | $CH_3$ | H | H | Fp: 148-150° C |
| IIb-17 | $-\underset{\underset{O}{\Vert}}{C}-OC(CH_3)_3$ | H | $-COOCH_3$ | H | $C_3H_7$ | H | H | $^1H$-NMR*) ($CDCl_3$, 200 MHz) δ= 0,90(3H), 1,20-1,48(9H), 1,85-2,12(2H), 3,67(3H), 4,52(2H), 5,71(2H) |
| IIb-18 | $-\underset{\underset{O}{\Vert}}{C}-OC(CH_3)_3$ | H | $-COOCH_3$ | H | $C_2H_5$ | H | H | Fp: 79-85° C |
| IIb-19 | $-\underset{\underset{O}{\Vert}}{C}-OC(CH_3)_3$ | H | -COOH | H | $C_2H_5$ | H | H | Fp: 149-167° C |
| IIb-20 | $-\underset{\underset{O}{\Vert}}{C}-OC(CH_3)_3$ | H | -COOH | H | H | $n-C_4H_9$ | H | $^1H$-NMR*) ($CDCl_3$, 200 MHz) δ= 0,89(3H), 1,10-1,50 u. 1,80-2,00(20H), 2,70-2,85(1H), 4,40-4,60 u. 5,00-5,20(1H), 5,35(2H) |
| IIb-21 | $-\underset{\underset{O}{\Vert}}{C}-OC(CH_3)_3$ | H | -COOH | H | H | $C_2H_5$ | H | Fp: 120-128° C (hygroskopisch) |

Tabelle 2 - Fortsetzung

| Bsp-Nr. | A | $R^{1'}$ | $R^{2'}$ | $R^{3'}$ | $R^{4'}$ | $R^{5'}$ | $R^{6'}$ | physikalische Konstanten |
|---|---|---|---|---|---|---|---|---|
| IIb-22 | $-\underset{\overset{\|}{O}}{C}-OC(CH_3)_3$ | H | $-COOCH_3$ | H | H | $C_2H_5$ | H | Fp: 65-71° C |
| IIb-23 | $-\underset{\overset{\|}{O}}{C}-OC(CH_3)_3$ | H | -COOH | H | $C_3H_7$ | H | H | Fp: 116-142° C |
| IIb-24 | $-\underset{\overset{\|}{O}}{C}-OC(CH_3)_3$ | H | -CN | H | $C_2H_5$ | H | H | Fp: 121-122° C |
| IIb-25 | $-\underset{\overset{\|}{O}}{C}-OC(CH_3)_3$ | H | -CN | H | $CH_3$ | H | H | MS: 183 $M^+$(12), 153, 127, 83, 57(100) |
| IIb-26 | $-\underset{\overset{\|}{O}}{C}-OC(CH_3)_3$ | Cl | -CN | H | $CH_3$ | H | H | Fp: 116-121° C |
| IIb-27 | $-\underset{\overset{\|}{O}}{C}-OC(CH_3)_3$ | H | $-NO_2$ | $-COOC_2H_5$ | H | H | H | Fp: 95-96° C |
| IIb-28 | $-\underset{\overset{\|}{O}}{C}-OC(CH_3)_3$ | H | $-NO_2$ | 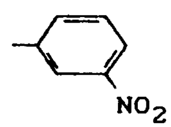 | $CH_3$ | H | H | Fp: 180-185° C |
| IIb-29 | $-\underset{\overset{\|}{O}}{C}-OCH_2$ 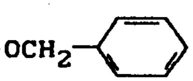 | H | $-NO_2$ | $-COOC_2H_5$ | $CH_3$ | H | H | Fp: 88-91° C |

Tabelle 2 - Fortsetzung

| Bsp-Nr. | A | $R^{1'}$ | $R^{2'}$ | $R^{3'}$ | $R^{4'}$ | $R^{5'}$ | $R^{6'}$ | physikalische Konstanten |
|---|---|---|---|---|---|---|---|---|
| IIb-30 | 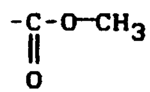 | H | $-NO_2$ | $-COO-C_4H_9-t$ | $CH_3$ | H | H | Fp: 178-181° C |
| IIb-31 | 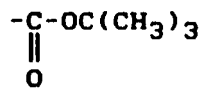 | H | $-NO_2$ | 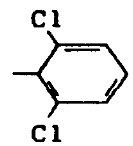 | $CH_3$ | H | H | Fp: 180-183° C |
| IIb-32 | 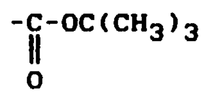 | H | $-NO_2$ | 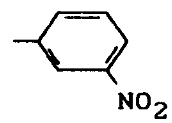 | $C_2H_5$ | H | H | Fp: 168-174° C |
| IIb-33 | 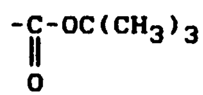 | H | $-NO_2$ | 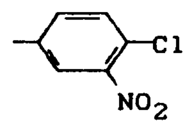 | $CH_3$ | H | H | Fp: 171-176° C |
| IIb-34 | 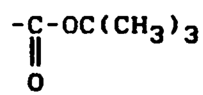 | H | $-NO_2$ | 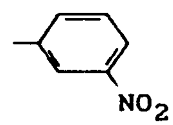 | H | $C_4H_9-n$ | H | Fp: 98-107° C |
| IIb-35 | 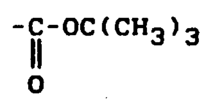 | H | $-NO_2$ | 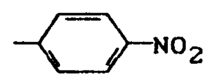 | $CH_3$ | H | H | Fp: 205-206° C |

Tabelle 2 - Fortsetzung

| Bsp-Nr. | A | $R^{1'}$ | $R^{2'}$ | $R^{3'}$ | $R^{4'}$ | $R^{5'}$ | $R^{6'}$ | physikalische Konstanten |
|---|---|---|---|---|---|---|---|---|
| IIb-36 | 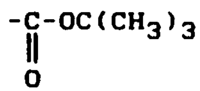 | H | $-NO_2$ | 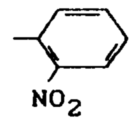 | $CH_3$ | H | H | Fp: 145-146° C |
| IIb-37 | 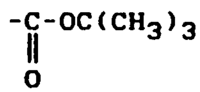 | H | $-NO_2$ | $-COOC_2H_5$ | $-CH_2-(CH_2)_2-CH_2-$ | | H | MS: 368 $M^{+\bullet}$ (0,1), 57 (100) |
| IIb-38 | 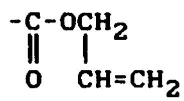 | H | $-NO_2$ | $-COOC_4H_9$-t | H | H | H | Fp: 123-124° C |
| IIb-39 | 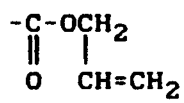 | H | $-NO_2$ | $-COOC_4H_9$-t | $CH_3$ | H | H | Fp: 120-126° C |
| IIb-40 | 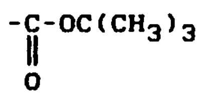 | H | 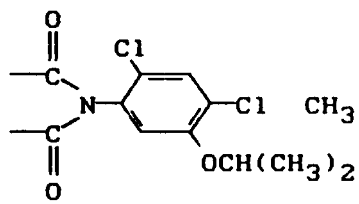 | | $CH_3$ | H | H | Fp: 186-191° C |
| IIb-41 | 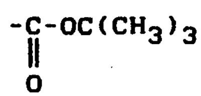 | H | 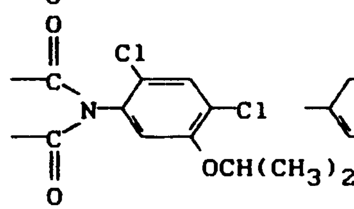 | | 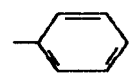 | H | H | Fp: 204-205° C |

Tabelle 2 - Fortsetzung

| Bsp-Nr. | A | $R^{1'}$ | $R^{2'}$ | $R^{3'}$ | $R^{4'}$ | $R^{5'}$ | $R^{6'}$ | physikalische Konstanten |
|---|---|---|---|---|---|---|---|---|
| IIb-42 | 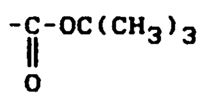 | H | 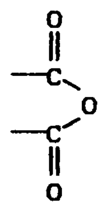 | | 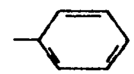 | H | H | Fp: 182-184° C |
| IIb-43 | 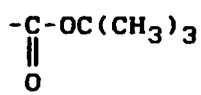 | H | 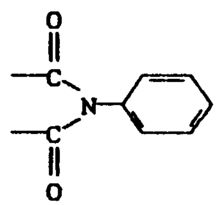 | | H | H | H | Fp: 88-93° C |
| IIb-44 | 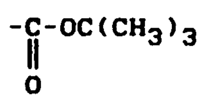 | H | 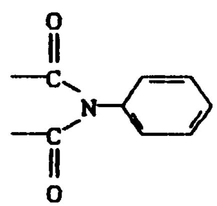 | | 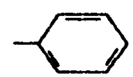 | H | H | Fp: 45-51° C |
| IIb-45 | 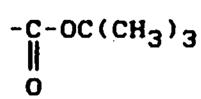 | H | 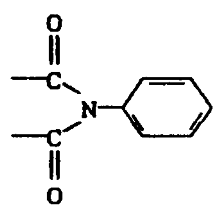 | | $CH_3$ | H | H | Fp: 132-135° C |

<u>Tabelle 2</u> - Fortsetzung

| Bsp-Nr. | A | $R^{1'}$ | $R^{2'}$ | $R^{3'}$ | $R^{4'}$ | $R^{5'}$ | $R^{6'}$ | physikalische Konstanten |
|---|---|---|---|---|---|---|---|---|
| IIb-46 | $-\underset{\underset{O}{\|}}{C}-OC(CH_3)_3$ | H | $NO_2$ | $-COO-C_4H_9-t$ | 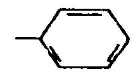 | H | H | Fp: 197-199° C |
| IIb-47 | $-\underset{\underset{O}{\|}}{C}-OC(CH_3)_3$ | H | $NO_2$ | 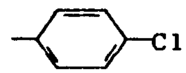 | $CH_3$ | H | H | Fp: 165-168° C |
| IIb-48 | $-\underset{\underset{O}{\|}}{C}-OC(CH_3)_3$ | H | $NO_2$ | 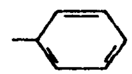 | $CH_3$ | H | H | Fp: 168-172° C |
| IIb-49 | $-\underset{\underset{O}{\|}}{C}-OC(CH_3)_3$ | H | $NO_2$ | $-COOC_2H_5$ | 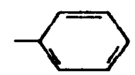 | H | H | Fp: 130-135° C |
| IIb-50 | $-\underset{\underset{O}{\|}}{C}-OC(CH_3)_3$ | H | $NO_2$ | $-COO-C_4H_9-t$ | $CH_3$ | H | H | Fp: 180-185° C |
| IIb-51 | $-\underset{\underset{O}{\|}}{C}-OC(CH_3)_3$ | H | $NO_2$ | $-COO-C_4H_9-t$ | H | H | H | Fp: 165-168° C |

<u>Tabelle 2</u>

| Bsp-Nr. | A | $R^{1'}$ | $R^{2'}$ | $R^{3'}$ | $R^{4'}$ | $R^{5'}$ | $R^{6'}$ | physikalische Konstanten |
|---|---|---|---|---|---|---|---|---|
| IIb-52 | $-\underset{\overset{\|}{O}}{C}-OC(CH_3)_3$ | H | CHO | $CH_3$ | H | H | H | $^1H$-NMR*) ($CDCl_3$, 200 MHz) δ= 0,95-1,25(4 Isomere,3H), 1,42(s, 9H), 5,55-5,90 (m,2H), 9,60-9,85(4 Isomere, 1H) |
| IIb-53 | $-\underset{\overset{\|}{O}}{C}-OC(CH_3)_3$ | H | CHO | H | H | H | H | IR ($CH_2Cl_2$): 3300, 2950, 2710, 1700 $cm^{-1}$ |
| IIb-54 | $-\underset{\overset{\|}{O}}{C}-OC(CH_3)_3$ | H | CHO | H | $CH_3$ | H | H | IR ($CH_2Cl_2$): 3400, 2950, 2860, 1720 $cm^{-1}$ |
| IIb-55 | $-\underset{\overset{\|}{O}}{C}-OC(CH_3)_3$ | F | CHO | H | $CH_3$ | H | H | MS: 201 [$M^+$ -Isobuten] (5), 183, 153, 127, 83, 57 |
| IIb-56 | $-\underset{\overset{\|}{O}}{C}-OC(CH_3)_3$ | H | $-CH=\underset{\overset{\|}{COOH}}{CH}$ | H | $CH_3$ | H | H | Fp: 106-136° C |
| IIb-57 | $-\underset{\overset{\|}{O}}{C}-OC(CH_3)_3$ | H | $-CH=\underset{\overset{\|}{COOH}}{CH}$ | H | H | H | H | Fp: 107-130° C |
| IIb-58 | $-\underset{\overset{\|}{O}}{C}-OC(CH_3)_3$ | H | $-CH=\underset{\overset{\|}{COOC_2H_5}}{CH}$ | H | H | H | H | Fp: 59-65° C |

**Tabelle 2** - Fortsetzung

| Bsp-Nr. | A | $R^{1'}$ | $R^{2'}$ | $R^{3'}$ | $R^{4'}$ | $R^{5'}$ | $R^{6'}$ | physikalische Konstanten |
|---|---|---|---|---|---|---|---|---|
| IIb-59 | $-\underset{\underset{O}{\|}}{C}-OC(CH_3)_3$ | H | $-CH_2OH$ | H | H | H | H | $^1H$-NMR*) ($CDCl_3$, 200 MHz) δ= 1,45 (9H), 1,65-2,13 (4H), 3,30-3,75 (2H), 4,08-4,30 (1H), 4,78-4,98 (1H), 5,45-5,93 (2H) |
| IIb-60 | $-\underset{\underset{O}{\|}}{C}-OC(CH_3)_3$ | H | 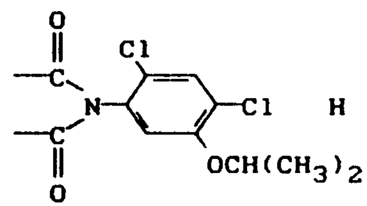 | | H | H | H | Fp: 83° C |
| IIb-61 | $-\underset{\underset{O}{\|}}{C}-OC(CH_3)_3$ | H | $NO_2$ | 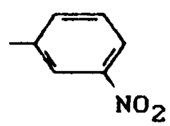 | H | $-CH(CH_3)_2$ | H | Fp: 166-170° C |
| IIb-62 | $-\underset{\underset{O}{\|}}{C}-OC(CH_3)_3$ | H | COOH | H | H | $-CH(CH_3)_2$ | H | Fp: 100-124° C |
| IIb-63 | $-\underset{\underset{O}{\|}}{C}-OC(CH_3)_3$ | H | $COOCH_3$ | H | H | $-CH(CH_3)_2$ | H | Fp: 85-93° C |
| IIb-64 | $-\underset{\underset{O}{\|}}{C}-OC(CH_3)_3$ | H | $NO_2$ | 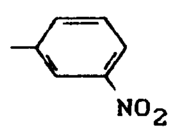 | $C_3H_7$ | H | H | Fp: 167-181° C |

Tabelle 2 - Fortsetzung

| Bsp-Nr. | A | $R^{1'}$ | $R^{2'}$ | $R^{3'}$ | $R^{4'}$ | $R^{5'}$ | $R^{6'}$ | physikalische Konstanten |
|---|---|---|---|---|---|---|---|---|
| IIb-65 | $-\underset{\underset{O}{\|}}{C}-OCH_2CCl_3$ | H | $NO_2$ | Cl 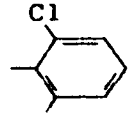 Cl | $CH_3$ | H | H | Fp: 99-100$^0$ C |
| IIb-66 | $-\underset{\underset{O}{\|}}{C}-OC(CH_3)_3$ | H | $-COOC_2H_5$ | H | $CH_3$ | H | H | Fp: 70-75$^0$ C |
| IIb-67 | $-\underset{\underset{O}{\|}}{C}-OC(CH_3)_3$ | H | $-COOC_4H_9$ | H | $CH_3$ | H | H | Fp: 65-71$^0$ C |
| IIb-68 | $-\underset{\underset{O}{\|}}{C}-OC(CH_3)_3$ | H | $-\underset{\underset{O}{\|}}{C}-N(CH_3)_2$ | H | H | H | H | Fp: 115-118$^0$ C |
| IIb-69 | $-\underset{\underset{O}{\|}}{C}-OC(CH_3)_3$ | H | $-\underset{\underset{O}{\|}}{C}-N(CH_3)_2$ | H | $CH_3$ | H | H | Fp: 121-124$^0$ C |
| IIb-70 | $-\underset{\underset{O}{\|}}{C}-OC(CH_3)_3$ | H | $-\underset{\underset{O}{\|}}{P}{<}^{OH}_{OCH_3}$ | H | $CH_3$ | H | H | MS [m/Z, % rel.Int.]: 305 [$M^+$](0,9), 249 (21), 205 (10), 188(15), 83 (100), 57 (68) |

Tabelle 2 - Fortsetzung

| Bsp-Nr. | A | R1' | R2' | R3' | R4' | R5' | R6' | physikalische Konstanten |
|---|---|---|---|---|---|---|---|---|
| IIb-71 | 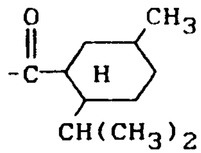 | H | $-C(=O)-OC(CH_3)_3$ | H | $CH_3$ | H | H | Fp: 72-78° C |
| IIb-72 | $-C(=O)-OCH_2CH_2OCH_3$ | H | $-CO_2CH_3$ | H | $CH_3$ | H | H | $n_D^{20}$ 1.4802 |
| IIb-73 | $-C(=O)-OCH_2CH_2OCH_3$ | H | $-NO_2$ | 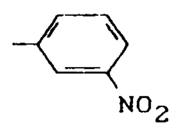 | $CH_3$ | H | H | Fp.: 166-170° C |
| IIb-74 | $-C(=O)-CH_3$ | H | $-CO_2H$ | H | $CH_3$ | H | H | Fp.: 202-220° C |
| IIb-75 | $-C(=O)-(CH_2)_5CH_3$ | H | $-CO_2H$ | H | $CH_3$ | H | H | Fp.: 144-146° C |
| IIb-76 | $-C(=O)-(CH_2)_{16}CH_3$ | H | $-CO_2H$ | H | $CH_3$ | H | H | Fp.: 107° C |
| IIb-77 | $-C(=O)-OC(CH_3)_3$ | H | $-CO_2CH_2CH(CH_3)_2$ | H | $CH_3$ | H | H | Fp.: 63-64° C |

Tabelle 2 - Fortsetzung

| Bsp-Nr. | A | $R^{1'}$ | $R^{2'}$ | $R^{3'}$ | $R^{4'}$ | $R^{5'}$ | $R^{6'}$ | physikalische Konstanten |
|---|---|---|---|---|---|---|---|---|
| IIb-78 | 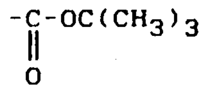 | H | $-CO_2CH_3$ | H | 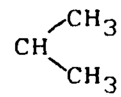 | H | H | $^1$H-NMR ($CDCl_3$): $\delta$ = 0,90 (6H); 1,43 (9H); 3,68 (3H); 5,75 (2H). |
| IIb-79 | 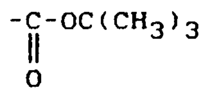 | H | $-CO_2CH_3$ | H | $CH_3$ | $CH_3$ | H | $^1$H-NMR ($CDCl_3$): $\delta$ = 1,05 (3H); 1,40 (9H); 1,68 (3H); 3,67 (3H); 5,45 (1H). |
| IIb-80 | 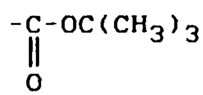 | H | $-CO_2CH_3$ | H | 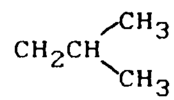 | 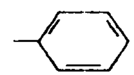 | H | $^1$H-NMR ($CDCl_3$): $\delta$ = 0,73-0,86 (8H); 1,43 (9H); 3,68 (3H); 5,82 (1H); 7,15-7,30 (5H). |
| IIb-81 | 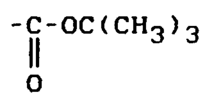 | H | $-CO_2H$ | H | 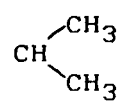 | H | H | Fp.: 154-158° C |
| IIb-82 | 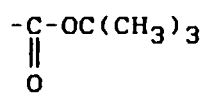 | H | $-CO_2H$ | H | $CH_3$ | $CH_3$ | H | Fp.: 147-152° C |
| IIb-83 | 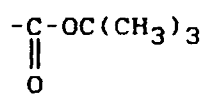 | H | $-CO_2H$ | H | 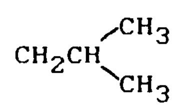 | 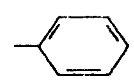 | H | Fp.: 122-125° C |

Tabelle 2 - Fortsetzung

| Bsp-Nr. | A | $R^{1'}$ | $R^{2'}$ | $R^{3'}$ | $R^{4'}$ | $R^{5'}$ | $R^{6'}$ | physikalische Konstanten |
|---|---|---|---|---|---|---|---|---|
| IIb-84 | $-\underset{\underset{O}{\|}}{C}-OC(CH_3)_3$ | H | $-NO_2$ | 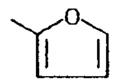 | $CH_3$ | H | H | Fp.: 169-170° C |
| IIb-85 | $-\underset{\underset{O}{\|}}{C}-OC(CH_3)_3$ | H | -CN | H | $CH_2CH(CH_3)_2$ | $CH(CH_3)_2$ | H | Fp.: 117-121° C |
| IIb-86 | $-\underset{\underset{O}{\|}}{C}-OC(CH_3)_3$ | H | -CN | H | $CH(CH_3)_2$ | $C_6H_5$ | H | Fp.: 84-105° C |
| IIb-87 | $-\underset{\underset{O}{\|}}{C}-OC(CH_3)_3$ | H | $-CO_2H$ | H | $CH(CH_3)_2$ | $C_6H_5$ | H | Fp.: 151-171° C (Z) |
| IIb-88 | $-\underset{\underset{O}{\|}}{C}-OC(CH_3)_3$ | H | $-CO_2H$ | H | $CH_2CH(CH_3)_2$ | $CH(CH_3)_2$ | H | Fp.: 150-156° C |
| IIb-89 | $-\underset{\underset{O}{\|}}{C}-OC(CH_3)_3$ | H | $-CO_2H$ | H | 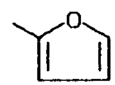 | H | H | MS: 307 [$M^+$] |

*) Die $^1H$-NMR-Spektren wurden in Deuterochloroform ($CDCl_3$) mit Tetramethylsilan (TMS) als innerem Standard aufgenommen. Angegeben ist die chemische Verschiebung als δ-Wert in ppm.

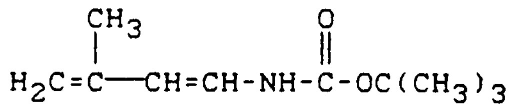 (IVa-1)

Eine Lösung aus 56 g (0,5 Mol) trans-4-Methyl-2,4-pentadiencarbonsäure und 80 g (0,62 Mol) N,N-Diisopropylethylamin in 300 ml Aceton wird bei -5 °C mit einer Lösung aus 54 g (0,5 Mol) Chlorameisensäureethylester in 150 ml Aceton über 30 Minuten versetzt. Nach weiteren 30 Minuten bei 0 °C tropft man eine eisgekühlte Lösung aus 65 g (1 mol) Natriumazid in 150 ml Wasser zu. Man läßt 15 Minuten bei 0 °C rühren und arbeitet dann mit Wasser-Toluol auf. Die auf ca. 300 ml eingeengte Toluol-Phase wird dann zu einer unter Rückfluß siedenden Lösung aus 29,6 g (0,4 Mol) tert-Butanol, 250 mg (1,5 mMol) tert-Butylcatechol in 200 ml Toluol getropft. Der Reaktionsverlauf wird IR-spektroskopisch verfolgt. Man läßt auf Raumtemperatur abkühlen und engt ein. Nach säulenchromatographischer Trennung mit dem Laufmittel Petrolether-Essigsäureethylester (6 : 1), erhält man 59 g (65 % der Theorie) tert-Butyl-trans-3-methyl-1,3-butadien-1-carbamat.

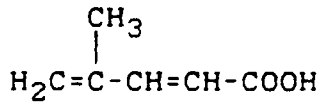 (IV-2)

10 g (0,07 Mol) trans-4-Methyl-2,4-pentadiencarbonsäureethylester, gelöst in einem Lösungsmittelgemisch aus 75 ml Methanol, 17 ml Tetrahydrofuran und 2,5 ml Wasser werden bei 0 °C mit 2,5 g (0,1 Mol) Lithiumhydroxid versetzt und 20 Stunden bei Raumtemperatur gerührt. Nach Verdünnen mit 200 ml Wasser wird einmal mit Diethylether extrahiert und die wässrige Phase bei 0 °C mit konzentrierter Salzsäure auf pH 1 eingestellt. Man extrahiert mit Diethylether, wäscht die vereinigten organischen Phasen mehrmals mit Wasser und gesättigter Natriumchlorid-Lösung und erhält nach Trocknen und Einengen 6,1 g (76 % der Theorie) trans-4-Methyl-2,4-pentadiencarbonsäure.

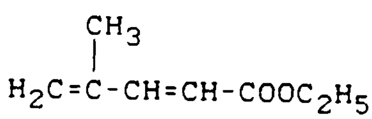 (IV-1)

Unter Stickstoffatmosphäre gibt man bei 0 °C portionsweise 77 g (2,57 Mol) Natriumhydrid (80 %ig in Öl) zu einer Lösung aus 630 g (2,8 Mol) Phosphonoessigsäuretriethylester in 500 ml Tetrahydrofuran. Man entfernt das Kältebad und läßt bis zur Beendigung der Wasserstoffentwicklung rühren (ca. 30 Minuten). Bei 0 °C tropft man dann eine Lösung aus 200 g (2,8 Mol) Methacrolein in 2000 ml Tetrahydrofuran zu und rührt 1 Stunde bei 0 °C und 2 Stunden bei Raumtemperatur nach. Zur Aufarbeitung wird der Ansatz geteilt, mit Wasser versetzt und mehrmals mit Essigester extrahiert. Nach Trocknen, Einengen und Destillieren werden 146 g (37 % der Theorie) an trans-4-Methyl-2,4-pentadiencarbonsäureethylester vom Siedepunkt 76-90 °C/ 20 bar erhalten.

Anwendungsbeispiele:

In den folgenden Anwendungsbeispielen werden die nachstehend aufgeführten Verbindungen als Vergleichssubstanzen eingesetzt:

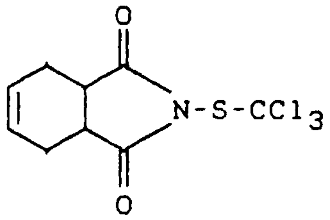

(A)

cis-N-[(Trichlormethyl)thio]-4-cyclohexen-1,2-dicarboimid
(bekannt aus Science, (Washington) 115, 84 (1952); US-PS 2 553 770).

Beispiel A

Venturia-Test (Apfel) /protektiv

Lösungsmittel: 4,7 Gewichtsteile Aceton
Emulgator: 0,3 Gewichtsteile Alkylarylpolyglykolether

Zur Herstellung einer zweckmäßig Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20 °C und 100 % relativer Lufteuchtigkeit in einer Inkubationskabine.

Die Pflanzen werden dann im Gewächshaus bei 20 °C und einer relativen Luftfeuchigkeit von ca. 70 % aufgestellt.

12 Tage nach der Inokulation erfolgt die Auswertung.

In diesem Test zeigt z.B. die erfindungsgemäße Verbindung (I-3) eine bessere Wirksamkeit als die Vergleichssubstanz (A).

Tabelle A

Venturia-Test (Apfel)/ protektiv

| Wirkstoff | Wirkungsgrad in % der unbehandelten Kontrolle bei einer Wirkstoffkonzentration von 5 ppm |
|---|---|
| 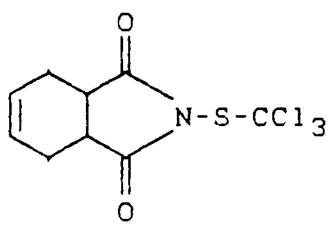 (bekannt) (A) | 57 |
| 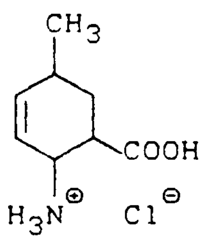 (I-3) | 74 |

Beispiel B

Phytophthora-Test (Tomate)/protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzübereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzübereitung bis zur Tropfnässe. Nach Antrocknen des Spritzbelages werden die Pflanzen mit einer wäßrigen Sporensuspension von Phytophthora infestans inokuliert.

Die Pflanzen werden in einer Inkubationskabine mit 100% relativer Luftfeuchtigkeit und ca. 20 °C aufgestellt.

3 Tage nach der Inokulation erfolgt die Auswertung.

In diesem Test zeigen z.B. die erfindungsgemäßen Stoffe (I-3) und (I-4) bei einer Wirkstoffkonzentration von 10 ppm einen sehr hohen Wirkungsgrad.

Tabelle B

Phytophthora-Test (Tomate)/protektiv/kurativ/systemisch

| Wirkstoff | Wirkungsgrad in % der unbehandelten Kontrolle bei einer Wirkstoffkonzentration von 10 ppm |
|---|---|
| 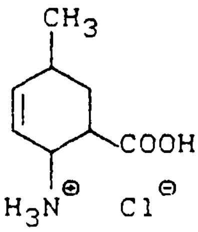 (I-3) | 78 |
| 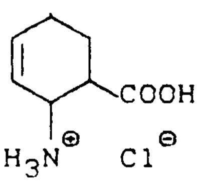 (I-4) | 89 |

Beispiel C

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

O % = keine Wirkung (wie unbehandelte Kontrolle)
100 % = totale Vernichtung

In diesem Test zeigt z.B. die in der folgenden Tabelle C aufgeführte Verbindung (I-4) insbesondere in Weizen eine sehr gute herbizide Wirkung.

Tabelle C

| Wirkstoff | Aufwandmenge g/ha | Post emergence / Gewächshaus Weizen | Echinochloa | Panicum | Abutilon | Amaranthus | Chenopodium | Sida | Xanthium |
|---|---|---|---|---|---|---|---|---|---|
| 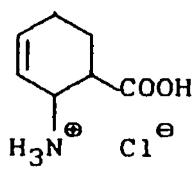 (I-4) | 500 | 0 | 100 | 100 | 100 | 90 | 95 | 100 | 95 |

## Patentansprüche

## Patentansprüche für folgende Vertragsstaaten : BE, CH, DE, FR, GB, IT, LI, NL

**1.** Verwendung von substituierten 2-Cyclohexen-1-yl-amin-Derivaten der Formel (I)

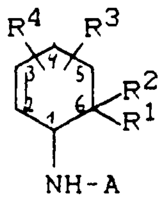

(I)

in welcher

$R^1$ für Wasserstoff, Alkyl oder Halogen steht,

$R^2$ für Formyl, Hydroxyalkyl, Cyano, Nitro oder für einen der Reste $-NHR^5$, $-NR^6R^7$,

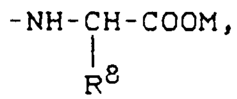

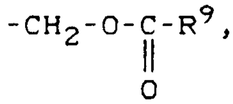, 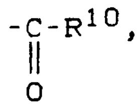, 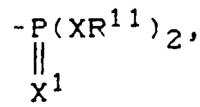, 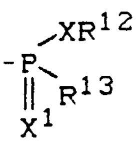,

$-S(O)_nR^{14}$ oder $-CH=CH-R^{15}$ steht,

$R^3$ und $R^4$ gleich oder verschieden sind und jeweils für Wasserstoff, Alkyl, Alkenyl, Alkinyl, Alkoxy, Alkenyloxy, Alkinyloxy, für unsubstituiertes oder substituiertes Aryl, für unsubstituiertes oder substituiertes Aralkyl, für unsubstituiertes oder substituiertes Heteroaryl, für unsubstituiertes oder substituiertes Heterocyclyalkyl, Alkoxyalkyloxy, Halogen oder für einer der Reste $-NH-R^5$, $-NR^6R^7$ oder $-S(O)_n-R^{14}$ stehen

oder

$R^2$ und $R^3$ gemeinsam für einen der Reste

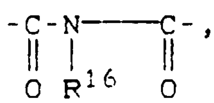, 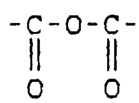 oder 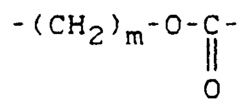,

verbrückt über die Positionen 6 und 5, stehen,

oder

$R^3$ und $R^4$ gemeinsam für eine Alkylkette mit 3- oder 4-Kohlenstoffatomen stehen, die über die Positionen 4 und 3 verbunden ist,

$R^5$ für Wasserstoff, Alkyl oder unsubstituiertes oder substituiertes Aryl steht,

$R^6$ für Alkyl oder unsubstituiertes oder substituiertes Aryl steht,

$R^7$ für Alkyl oder unsubstituiertes oder substituiertes Aryl steht,

$R^8$ für Wasserstoff, Alkyl, unsubstituiertes oder substituiertes Aryl oder unsubstituiertes oder substituiertes Aralkyl steht,

$R^9$ für Alkyl Alkoxy steht,

$R^{10}$ für Hydroxy, Hydroxyalkyloxy, Halogenalkyloxy, Alkoxy, Alkoxyalkyloxy, unsubstituiertes oder substituiertes Cycloalkyloxy, unsubstituiertes oder substituiertes Aralkyloxy,

unsubstituiertes oder substituiertes Aryloxy, unsubstituiertes oder substituiertes Aralkyl, Alkylthio, unsubstituiertes oder substituiertes Arylthio oder für eine Gruppe -OM, $-NHR^5$, $-NR^6R^7$ oder $-O-Z-NR^5R^6$ steht,

$R^{11}$ für Wasserstoff oder Alkyl steht,

$R^{12}$ für Wasserstoff oder Alkyl steht,

$R^{13}$ für Alkyl steht,

$R^{14}$ für Alkyl, Alkoxy, unsubstituiertes oder substituiertes Aryl oder für die Gruppe -OM steht,

$R^{15}$ für Formyl, Cyano oder für die Gruppierung

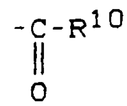

steht,

$R^{16}$ für Wasserstoff, Alkyl oder unsubstituiertes oder substituiertes Aryl steht,

M für Wasserstoff oder für ein Äquivalent eines entsprechenden Alkalimetall-, Erdalkalimetall-oder Ammoniumkations steht,

n für eine Zahl 0, 1 oder 2 steht,

X und $X^1$ gleich oder verschieden sind und für Sauerstoff oder Schwefel stehen,

m für eine Zahl 1 oder 2 steht,

A für Wasserstoff oder eine Aminoschutzgruppe steht und

Z für eine geradkettige oder verzweigte Alkylkette steht,

sowie deren Säureadditions-Salze und Metallsalz-Komplexe zur Bekämpfung von Schädlingen.

**2.** Substituierte 2-Cyclohexen-1-yl-amin-Derivate der Formel (Ia)

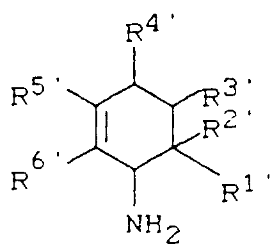

(Ia)

in welcher

$R^{1'}$ für Wasserstoff, Alkyl oder Halogen steht,

$R^{2'}$ für Formyl, Hydroxyalkyl, Cyano, Nitro oder für einen der Reste

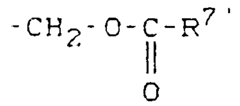 , 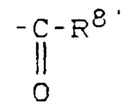

oder $-CH=CH-R^{9'}$ steht,

$R^{3'}$, $R^{4'}$, $R^{5'}$ und $R^{6'}$ gleich oder verschieden sind und jeweils für Wasserstoff, Alkyl, Alkenyl, Alkinyl, Alkoxy, Alkenyloxy, Alkinyloxy, für unsubstituiertes oder substituiertes Aryl, für unsubstituiertes oder substituiertes Aralkyl, für unsubstituiertes oder substituiertes Heteroaryl, für unsubstitrniertes oder substituiertes Heterocyclylalkyl, Alkoxyalkyloxy oder für Halogen steht, wobei mindestens zwei der Reste $R^{3'}$, $R^{4'}$, $R^{5'}$ oder $R^{6'}$ für Wasserstoff stehen,

$R^{7'}$ für Alkyl oder Alkoxy steht,

$R^{8'}$ für Hydroxy, Hydroxyalkyloxy, Halogenalkyloxy, Alkoxy, Alkoxyalkyloxy, unsubstituiertes oder substituiertes Cycloalkyloxy, unsubstituiertes oder substituiertes Aralkyloxy, unsubstituiertes oder substituiertes Aryloxy, unsubstituiertes oder substituiertes Aralkyl, Alkylthio, unsubstituiertes oder substituiertes Arylthio oder für eine Gruppe $-O-Z-NR^{11'}R^{12'}$, $-NHR^{10'}$, $-NR^{11'}R^{12'}$ oder -OM steht,

$R^{9'}$ für Formyl, Cyano oder für die Gruppe

$$-\underset{\underset{O}{\|}}{C}-R^{8'}$$

steht,

$R^{10'}$ für Wasserstoff, Alkyl oder unsubstituiertes oder substituiertes Aryl steht,

$R^{11'}$ und $R^{12'}$ gleich oder verschieden sind und jeweils für Alkyl oder unsubstituiertes oder substituiertes Aryl stehen,

Z für eine geradkettige oder verzweigte Alkylkette steht und

M für Wasserstoff oder für ein Äquivalent eines entsprechenden Alkalimetall-, Erdalkalimetall- oder Ammoniumkations steht

oder

$R^{2'}$ und $R^{3'}$ gemeinsan für einen der Reste

$$-\underset{\underset{O}{\|}}{C}-\underset{\underset{R^{13'}}{|}}{N}-\underset{\underset{O}{\|}}{C}-,\quad -\underset{\underset{O}{\|}}{C}-O-\underset{\underset{O}{\|}}{C}-\quad \text{oder}\quad -(CH_2)_m-O-\underset{\underset{O}{\|}}{C}-\ ,$$

verbrückt über die Positionen 6 und 5, stehen,

worin

$R^{13'}$ für Wasserstoff, Alkyl oder unsubstituiertes oder substituiertes Aryl steht und

m für eine Zahl 1 oder 2 steht,

oder

$R^{4'}$ und $R^{5'}$ gemeinsam für eine Alkylkette mit 3- oder 4-Kohlenstoffatomen stehen, die über die Positionen 4 und 3 verbunden ist

sowie deren Säureadditions-Salze und Metallsalz-Komplexe ausgenommen die Verbindungen 2-Amino-cyclohex-3-en carbonsäure und 2-Amino-cyclohex-3-en carbonsäureethylester sowie deren Hydrochlorid-Salze.

**3.** Substituierte 2-Cyclohexen-1-yl-amin-Derivate der Formel (Ia) gemäß Anspruch 2, in welcher

$R^{1'}$ für Wasserstoff, geradkettiges oder verzweigtes Alkyl mit 1 bis 6 Kohlenstoffatomen oder Fluor, Chlor oder Brom steht,

$R^{2'}$ für Formyl, geradkettiges oder verzweigtes Hydroxyalkyl mit 1 bis 8 Kohlenstoffatomen im Alkylteil, Cyano, Nitro oder für einen der Reste

$$-CH_2-O-\underset{\underset{O}{\|}}{C}-R^{7'},\quad -\underset{\underset{O}{\|}}{C}-R^{8'}$$

oder $-CH=CH-R^{9'}$ steht,

$R^{3'}$, $R^{4'}$, $R^{5'}$ und $R^{6'}$ gleich oder verschieden sind und jeweils für Wasserstoff, jeweils geradkettiges oder verzweigtes Alkyl oder Alkoxy mit 1 bis 8 Kohlenstoffatomen, jeweils geradkettiges oder verzweigtes Alkenyl, Alkinyl, Alkenyloxy oder Alkinyloxy mit jeweils 2 bis 8 Kohlenstoffatomen, Alkoxyalkyloxy mit jeweils 1

bis 8 Kohlenstoffatomen in den einzelnen Alkylteilen, für jeweils im Arylteil unsubtituiertes oder einfach bis fünffach, gleich oder verschieden substituiertes Aryl oder Aralkyl mit jeweils 6 bis 10 Kohlenstoffatomen im Arylteil und gegebenenfalls 1 bis 4 Kohlenstoffatomen im Alkylteil stehen, wobei als Arylsubstituenten infrage kommen: Halogen, Nitro, Cyano, Amino, $C_1$-$C_4$-Alkyl, $C_1$-$C_4$-Alkoxy, $C_1$-$C_4$-Alkylthio, Halogen-($C_1$-$C_4$)-alkyl, Halogen-($C_1$-$C_4$)-alkoxy, Halogen-($C_1$-$C_4$)-alkylthio mit jeweils 1 bis 9 gleichen oder verschiedenen Halogenatomen und Di-($C_1$-$C_4$)-alkylamino,

weiterhin für eine unsubstituierte oder einfach bis dreifach, gleich oder verschieden substituierte und gegebenenfalls über eine Methylengruppe gebundene heterocyclische 5- oder 6-gliedrige Gruppierung aus der Reihe Furyl, Thienyl, Pyrrolyl, Pyrazolyl, Imidazolyl, 1,2,3- oder 1,2,4-Triazolyl, Oxazolyl, Isoxazolyl, 1,2,4- oder 1,3,4-Oxadiazolyl, Thiazolyl, Isothiazolyl, 1,2,3-, 1,2,4-, 1,2,5- oder 1,3,4-Thiadiazolyl, Pyridyl, Pyridazinyl, Pyrimidinyl und Pyrazinyl stehen, wobei als Substituenten für den Heterocyclus jeweils infrage kommen: Halogen, Nitro, Cyano, Amino, $C_1$-$C_4$-Alkyl, $C_1$-$C_4$-Alkoxy oder $C_1$-$C_4$-Alkylthio, Halogen-($C_1$-$C_4$)-alkyl, Halogen-($C_1$-$C_4$)-alkoxy, Halogen-($C_1$-$C_4$)-alkylthio mit jeweils 1 bis 9 gleichen oder verschiedenen Halogenatomen und Di-($C_1$-$C_4$)-alkylamino, weiterhin für Fluor, Chlor oder Brom stehen,

wobei mindestens zwei der Reste $R^{3'}$, $R^{4'}$, $R^{5'}$ oder $R^{6'}$ für Wasserstoff stehen,

$R^{7'}$ für jeweils geradkettiges oder verzweigtes Alkyl oder Alkoxy mit 1 bis 6 Kohlenstoffatomen steht,

$R^{8'}$ für Hydroxy, geradkettiges oder verzweigtes Hydroxyalkyloxy mit 1 bis 8 Kohlenstoffatomen, geradkettiges oder verzweigtes Halogenalkyloxy mit 1 bis 8 Kohlenstoffatomen und 1 bis 17 gleichen oder verschiedenen Halogenatomen, unsubstituiertes oder einfach bis mehrfach, gleich oder verschieden durch Halogen substituiertes Cycloalkyloxy mit 3 bis 6 Kohlenstoffatomen, jeweils geradkettiges oder verzweigtes Alkoxy oder Alkylthio mit 1 bis 6 Kohlenstoffatomen, geradkettiges oder verzweigtes Alkoxyalkyloxy mit jeweils 1 bis 6 Kohlenstoffatomen im Alkoxy- oder Alkylteil, jeweils im Arylteil unsubstituiertes oder einfach bis fünffach, gleich oder verschieden substituiertes Aryloxy, Arylthio, Aralkyl oder Aralkyloxy mit jeweils 6 bis 10 Kohlenstoffatomen im Arylteil und gegebenenfalls 1 bis 8 Kohlenstoffatomen im Alkylteil steht, wobei als Arylsubstituenten die oben aufgeführten Arylsubstituenten in Frage kommen oder für eine Gruppe -O-Z-$NR^{11'}R^{12'}$, -$NHR^{10'}$, -$NR^{11'}R^{12'}$ oder -OM steht,

$R^{9'}$ für Formyl, Cyano oder für die Gruppe

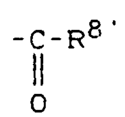

steht,

$R^{10'}$ für Wasserstoff, geradkettiges oder verzweigtes Alkyl mit 1 bis 6 Kohlenstoffatomen oder unsubstituiertes oder einfach bis fünffach, gleich oder verschieden substituiertes Aryl mit 6 bis 10 Kohlenstoffatomen steht, wobei als Arylsubstituenten die oben aufgeführten Arylsubstituenten in Frage kommen,

$R^{11'}$ für geradkettiges oder verzweigtes Alkyl mit 1 bis 6 Kohlenstoffatomen oder unsubstituiertes oder einfach bis fünffach, gleich oder verschieden substituiertes Aryl mit 6 bis 10 Kohlenstoffatomen steht, wobei als Arylsubstituenten die oben aufgeführten Arylsubstituenten in Frage kommen,

$R^{12'}$ für geradkettiges oder verzweigtes Alkyl mit 1 bis 6 Kohlenstoffatomen oder unsubstituiertes oder einfach bis fünffach, gleich oder verschieden substituiertes Aryl mit 6 bis 10 Kohlenstoffatomen steht, wobei als Arylsubstituenten

| | | |
|---|---|---|
| | | die oben aufgeführten Arylsubstituenten infrage kommen, |
| M | | für Wasserstoff oder für ein Äquivalent eines entsprechenden Alkalimetall-, Erdalkalimetall- oder Ammoniumkations steht und |
| Z | | für eine geradkettige oder verzweigte Alkylkette mit 1 bis 8 Kohlenstoffatomen steht |

oder

$R^{2'}$ und $R^{3'}$ gemeinsam für einen der Reste

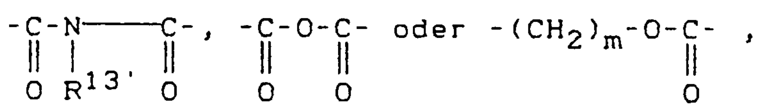

verbrückt über die Positionen 6 und 5, stehen,

worin

$R^{13'}$ für Wasserstoff, geradkettiges oder verzweigtes Alkyl mit 1 bis 6 Kohlenstoffatomen oder unsubstituiertes oder einfach bis fünffach, gleich oder verschieden substituiertes Aryl mit 6 bis 10 Kohlenstoffatomen steht, wobei als Arylsubstituenten die oben aufgeführten Arylsubstituenten in Frage kommen und

m für eine Zahl 1 oder 2 steht,

oder

$R^{4'}$ und $R^{5'}$ gemeinsam für eine Alkylkette mit 3- oder 4-Kohlenstoffatomen steht, die über die Positionen 4 und 3 verbunden ist

sowie deren Säureadditions-Salze und Metallsalz-Komplexe.

**4.** Substituierte 2-Cyclohexen-1-yl-amin-Derivate der Formel (Ia) gemäß Anspruch 2, in welcher

| | |
|---|---|
| $R^{1'}$ | für Wasserstoff, geradkettiges oder verzweigtes Alkyl mit 1 bis 4 Kohlenstoffatomen oder Fluor, Chlor oder Brom steht, |
| $R^{2'}$ | für Formyl, geradkettiges oder verzweigtes Hydroxyalkyl mit 1 bis 4 Kohlenstoffatomen im Alkylteil, Cyano, Nitro oder für einen der Reste |

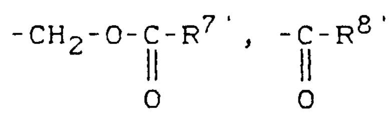

oder $-CH=CH-R^{9'}$ steht,

$R^{3'}$, $R^{4'}$, $R^{5'}$ und $R^{6'}$ gleich oder verschieden sind und jeweils für Wasserstoff, jeweils geradkettiges oder verzweigtes Alkyl oder Alkoxy mit 1 bis 6 Kohlenstoffatomen, jeweils geradkettiges oder verzweigtes Alkenyl, Alkinyl, Alkenyloxy oder Alkinyloxy mit jeweils 2 bis 6 Kohlenstoffatomen, Alkoxyalkyloxy mit jeweils 1 bis 6 Kohlenstoffatomen in den einzelnen Alkylteilen, für jeweils im Phenylteil unsubstituiertes oder einfach bis dreifach, gleich oder verschieden substituiertes Phenyl oder Phenylalkyl mit gegebenenfalls 1 oder 2 Kohlenstoffatomen im Alkylteil stehen, wobei als Phenylsubstituenten infrage kommen: Fluor, Chlor, Brom, Nitro, Cyano, Amino, $C_1$-$C_2$-Alkyl, $C_1$-$C_3$-Alkoxy, $C_1$-$C_2$-Alkylthio, Halogen-($C_1$-$C_2$)-alkyl, Halogen-($C_1$-$C_2$)-alkoxy, Halogen-($C_1$-$C_2$)-alkylthio mit jeweils 1 bis 5 gleichen oder verschiedenen Fluor- und/ oder Chloratomen und Di-($C_1$-$C_2$)-alkylamino,
weiterhin für eine unsubstituierte oder einfach bis dreifach, gleich oder verschieden substituierte und gegebenenfalls über eine Methylengruppe gebundene heterocyclische fünf- bzw. sechsgliedrige Gruppierung aus der Reihe Furyl, Thienyl, Pyrrolyl, Pyrazolyl, Imidazolyl, 1,2,3- oder 1,2,4-Triazolyl, Oxazolyl, Isoxazolyl, 1,2,4- oder 1,3,4-Oxadiazolyl, Thiazolyl, Isothiazolyl, 1,2,3-, 1,2,4-, 1,2,5- oder 1,3,4-Thiadiazolyl, Pyridyl, Pyridazinyl, Pyrimidinyl und Pyrazinyl stehen, wobei als Substituenten für den Heterocyclus jeweils in

Frage kommen: Fluor, Chlor, Brom, Nitro, Cyano, Amino, $C_1$-$C_2$-Alkyl, $C_1$-$C_2$-Alkoxy oder $C_1$-$C_2$-Alkylthio, Halogen-($C_1$-$C_2$)-alkyl, Halogen-($C_1$-$C_2$)-alkoxy, Halogen-($C_1$-$C_2$)-alkylthio mit jeweils 1 bis 5 gleichen oder verschiedenen Fluor- und/oder Chloratomen und Di-($C_1$-$C_2$)-alkylamino,
weiterhin für Fluor, Chlor oder Brom stehen,
wobei mindestens zwei der Reste $R^{3'}$, $R^{4'}$, $R^{5'}$ und $R^{6'}$ für Wasserstoff stehen,

$R^{7'}$ für jeweils geradkettiges oder verzweigtes Alkyl oder Alkoxy mit 1 bis 4 Kohlenstoffatomen steht,

$R^{8'}$ für Hydroxy, geradkettiges oder verzweigtes Hydroxyalkyloxy mit 1 bis 6 Kohlenstoffatomen, geradkettiges oder verzweigtes Halogenalkyloxy mit 1 bis 6 Kohlenstoffatomen und 1 bis 13 gleichen oder verschiedenen Halogenatomen, unsubstituiertes oder einfach bis dreifach, gleich oder verschieden durch Fluor, Chlor, Brom substituiertes Cycloalkyloxy mit 3 bis 6 Kohlenstoffatomen, jeweils geradkettiges oder verzweigtes Alkoxy oder Alkylthio mit 1 bis 4 Kohlenstoffatomen, geradkettiges oder verzweigtes Alkoxyalkyloxy mit jeweils 1 bis 4 Kohlenstoffatomen im Alkoxy- oder Alkylteil, jeweils im Phenylteil unsubstituiertes oder einfach bis dreifach, gleich oder verschieden substituiertes Phenyloxy, Phenylthio, Phenylalkyl oder Phenylalkyloxy mit jeweils gegebenenfalls 1 bis 6 Kohlenstoffatomen im Alkylteil steht, wobei als Phenylsubstituenten die oben aufgeführten Phenylsubstituenten in Frage kommen
oder für eine Gruppe -O-Z-$NR^{11'}R^{12'}$, -$NHR^{10'}$, -$NR^{11'}R^{12'}$ oder -OM steht,

$R^{9'}$ für Formyl, Cyano oder für die Gruppe

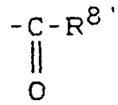

steht,

$R^{10'}$ für Wasserstoff, geradkettiges oder verzweigtes Alkyl mit 1 bis 4 Kohlenstoffatomen oder unsubstituiertes oder einfach bis dreifach, gleich oder verschieden substituiertes Phenyl steht, wobei als Phenylsubstituenten die oben aufgeführten Phenylsubstituenten in Frage kommen,

$R^{11'}$ für geradkettiges oder verzweigtes Alkyl mit 1 bis 4 Kohlenstoffatomen oder unsubstituiertes oder einfach Bis dreifach, gleich oder verschieden substituiertes Phenyl steht, wobei als Phenylsubstituenten die oben aufgeführten Phenylsubstituenten in Frage kommen,

$R^{12'}$ für geradkettiges oder verzweigtes Alkyl mit 1 bis 4 Kohlenstoffatomen oder unsubstituiertes oder einfach bis dreifach, gleich oder verschieden substituiertes Phenyl steht, wobei als Phenylsubstituenten die oben aufgeführten Phenylsubstituenten in Frage kommen,

M für Wasserstoff oder für ein Äquivalent eines entsprechenden Alkalimetall-, Erdalkalimetall- oder Ammoniumkations steht und

Z für eine geradkettige oder verzweigte Alkylkette mit 1 bis 6 Kohlenstoffatomen steht

oder

$R^{2'}$ und $R^{3'}$ gemeinsam für einen der Reste

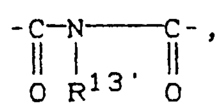, 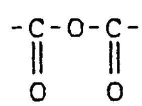 oder 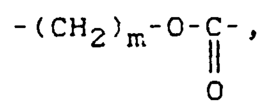,

verbrückt über die Positionen 6 und 5, stehen,

$R^{13'}$ für Wasserstoff, geradkettiges oder verzweigtes Alkyl mit 1 bis 6 Kohlenstoffatomen

oder unsubstituiertes oder einfach bis dreifach, gleich oder verschieden substituiertes Phenyl steht, wobei als Phenylsubstituenten die oben aufgeführten Phenylsubstituenten in Frage kommen und

m für eine Zahl 1 oder 2 steht,

oder

$R^{4'}$ und $R^{5'}$ gemeinsam für eine Alkylkette mit 3- oder 4-Kohlenstoffatomen steht, die über die Positionen 4 und 3 verbunden ist,

sowie deren Säureadditions-Salze und Metallsalz-Komplexe.

**5.** Das Hydrochlorid des cis-Diastereomeren der 2-Amino-cyclohex-3-en-carbonsäure. - -

**6.** Die Hydrochloride der Enantiomeren der 2-Amino-cyclohex-3-en-carbonsäure vom Schmelzpunkt 210 - 213,5° C und 208 - 210° C.

**7.** Die Isomere 2-Amino-cyclohex-3-en-carbonsäure vom Schmelzpunkt 98° C.

**8.** Das Hydrochlorid des Isomeren des 2-Amino-cyclohex-3-en-carbonsäureethylester vom Schmelzpunkt 139 - 148° C.

**9.** Verfahren zur Herstellung von substituierten 2-Cyclo-hexen-1-yl-amin-Derivaten der Formel (Ia)

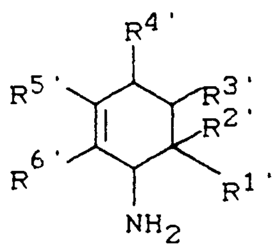

(Ia)

in welcher

$R^{1'}$ für Wasserstoff, Alkyl oder Halogen steht,

$R^{2'}$ für Formyl, Hydroxyalkyl, Cyano, Nitro oder für einen der Reste

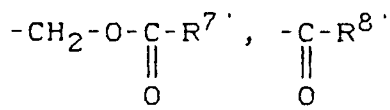

oder $-CH=CH-R^{9'}$ steht,

$R^{3'}$, $R^{4'}$, $R^{5'}$ und $R^{6'}$ gleich oder verschieden sind und jeweils für Wasserstoff, Alkyl, Alkenyl, Alkinyl, Alkoxy, Alkenyloxy, Alkinyloxy, für unsubstituiertes oder substituiertes Aryl, für unsubstituiertes oder substituiertes Aralkyl, für unsubstituiertes oder substituiertes Heteroaryl, für unsubstituiertes oder substituiertes Heterocyclylalkyl, Alkoxyalkyloxy oder für Halogen steht,

wobei mindestens zwei der Reste $R^{3'}$, $R^{4'}$, $R^{5'}$ oder $R^{6'}$ für Wasserstoff stehen,

$R^{7'}$ für Alkyl oder Alkoxy steht,

$R^{8'}$ für Hydroxy, Hydroxyalkyloxy, Halogenalkyloxy, Alkoxy, Alkoxyalkyloxy, unsubstituiertes oder substituiertes Cycloalkyloxy, unsubstituiertes oder substituiertes Aralkyloxy, unsubstituiertes oder substituiertes Aryloxy, unsubstituiertes oder substituiertes Aralkyl, Alkylthio, unsubstituiertes oder substituiertes Arylthio oder für eine Gruppe $-O-Z-NR^{11'}R^{12'}$, $-NHR^{10'}$, $-NR^{11'}R^{12'}$ oder -OM steht,

$R^{9'}$ für Formyl, Cyano oder für die Gruppe

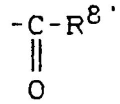

steht,

$R^{10'}$ für Wasserstoff, Alkyl oder unsubstituiertes oder substituiertes Aryl steht,

$R^{11'}$ und $R^{12'}$ gleich oder verschieden sind und jeweils für Alkyl oder unsubstituiertes oder substituiertes Aryl stehen,

Z für eine geradkettige oder verzweigte Alkylkette steht und

M für Wasserstoff oder für ein Äquivalent eines entsprechenden Alkalimetall-, Erd-alkalimetall-oder Ammoniumkations steht

oder

$R^{2'}$ und $R^{3'}$ gemeinsam für einen der Reste

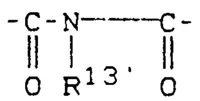 , 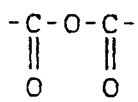 oder 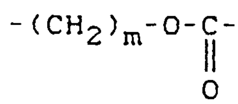 ,

verbrückt über die Positionen 6 und 5, stehen,

worin

$R^{13'}$ für Wasserstoff, Alkyl oder unsubstituiertes oder substituiertes Aryl steht und

m für eine Zahl 1 oder 2 steht,

oder

$R^{4'}$ und $R^{5'}$ gemeinsam für eine Alkylkette mit 3- oder 4-Kohlenstoffatomen stehen, die über die Positionen 4 und 3 verbunden ist,

sowie deren Säureadditions-Salze und Metallsalz-Komplexe ausgenommen die Verbindungen 2-Amino-cyclohex-3-en-carbonsäure und 2-Amino-cyclohex-3-en-carbonsäureethylester, dadurch gekennzeichnet, daß man

A) 2-Cyclohexen-1-yl-carbonsäurederivate der Formel (II)

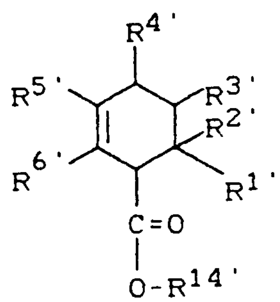

(II)

in welcher

$R^{1'}$, $R^{2'}$, $R^{3'}$, $R^{4'}$, $R^{5'}$ und $R^{6'}$ die oben angegebene Bedeutung haben und

$R^{14'}$ für Wasserstoff, Methyl oder Ethyl steht,

in allgemein üblicher Weise, nach Curtius, gegebenenfalls in Gegenwart eines Verdünnungsmittels und in Gegenwart einer Base Bei Temperaturen zwischen -15 °C und +10 °C, mit Chlorameisensäureester versetzt und zu dieser Reaktionsmischung gegebenenfalls in Gegenwart eines Verdünnungsmittels bei Temperaturen zwischen -5 °C und +25 °C ein Azid zugibt,

und das intermediär auftretende Isocyanat der Formel (IIa)

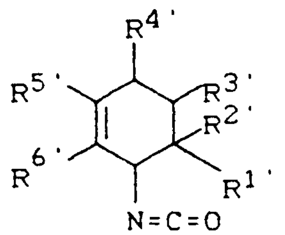

(IIa)

in welcher

$R^{1'}$, $R^{2'}$, $R^{3'}$, $R^{4'}$, $R^{5'}$ und $R^{6'}$ die oben angegebene Bedeutung haben,

mit Wasser, gegebenenfalls in Gegenwart einer Säure oder einer Base, hydrolysiert und die so erhaltenen Amine gegebenenfalls in Säureadditions-Salze und Metallsalz-Komplexe überführt, oder

B) aus den 2-Cyclohexen-Derivaten der Formel (IIb)

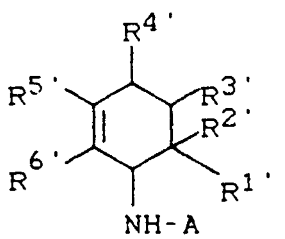

(IIb)

in welcher

$R^{1'}$, $R^{2'}$, $R^{3'}$, $R^{4'}$, $R^{5'}$ und $R^{6'}$ die oben angegebene Bedeutung haben und

A für eine Aminoschutzgruppe steht,

die Aminoschutzgruppe,

in an sich bekannter Weise nach üblichen Methoden, gegebenenfalls in Gegenwart eines geeigneten Lösungs- oder Verdünnungsmittels oder eines Gemisches aus ihnen, unter Kühlen, bei Raumtemperatur oder unter Erwärmen und, falls erforderlich, in einem geschlossenen Gefäss, unter Druck, in einer Inertgasatmosphäre und/oder unter wasserfreien Bedingungen, abspaltet und die so erhaltenen Produkte gegebenenfalls in Säureadditions-Salze oder Metallsalz-Komplexe überführt.

**10.** 2-Cyclohexen-Derivate der Formel (IIb)

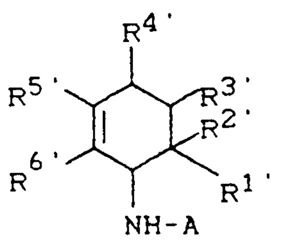

(IIb)

in welcher

$R^{1'}$, $R^{2'}$, $R^{3'}$, $R^{4'}$, $R^{5'}$ und $R^{6'}$ die in Anspruch 2 angegebene Bedeutung haben und

A für eine Aminoschutzgruppe steht,

ausgenommen die Verbindungen und ihre Enantiomeren und Isomeren: 6-Formyl-5-{[(phenylmethoxy)-carbonyl]-amino}-3-cyclohexen-1-carbonsäuremethylester, 6-[3-Oxo-1-propenyl)-5-{[(phenylmethoxy)-carbonyl]-amino}-3-cyclohexen-1-carbonsäuremethylester , 3-Cyclohexen-2-[(trichloracetyl)-amino)-1-carbonsäuremethylester, 2,2,2-Trichlor-N-(6-formyl-2-cyclohexen-1-yl)-acetamid, (6-Formyl-5-methyl-2-cyclohexen-1-yl)-carbaminsäureethylester, 2-[(Ethoxy-carbonyl)-amino]-6-methyl-3-cyclohexen-1-carbonsäuremethylester, 2-[(Ethoxycarbonyl)-amino]-5-methyl-3-cyclohexen-1-carbonsäuremethylester, 2-[(Ethoxycarbonyl)-amino]-3-cyclohexen-1-carbon-säuremethylester, 3-{2-[(Ethoxycarbonyl)-amino] -6-methyl-3-cyclohexen-1-yl}-2-propensäureethylester, (6-Formyl-5-propyl-2-cyclohexen-1-yl)-carbaminsäurephenylmethylester, (6-Formyl-5-methyl-2-cyclohexen-1-yl)-carbaminsäurephenylmethylester, 2-[-(Phenoxycarbonyl)-amino)-3-cyclohexen-1-carbonsäuremethylester, 3-(6-Methyl-2-{[(phenylmethoxy)-carbonyl]-amino}-3-cyclohexen-1-yl)-2-propensäureethylester, (6-Formyl-5-pentyl-2-cyclohexen-1-yl)-carbaminsäurephenylmethylesterPhenyl-6-carbomethoxy-2-cyclohexen-1-yl-thiocarbamat, N-(6-carbomethoxy-2-cyclohexan-1-yl)-1-pyrrolidin-carboxamid, 2-[(p-Toluolsulfonyl)-amino]-3-cyclohexen-1 -1-carbonsäuremethylester, -carbonsäure-t-butylester, -carbonsäure-benzylester und -carbonsäure-2,4,6-trimethylphenylester und {6-Formyl-5-[2-(methoxymethoxy)-ethyl]-2-cyclohexen-1-yl}-carbaminsäurephenylmethylester.

**11.** Schädlingsbekämpfungsmittel, gekennzeichnet durch einen Gehalt an mindestens einem 2-Cyclohexen-1-yl-amin-Derivat der Formel (I) nach Anspruch 1 und unter Verwendung von oberflächenaktiven Mitteln.

**12.** Verfahren zur Bekämpfung von Schädlingen, dadurch gekennzeichnet, daß man 2-Cyclohexen-1-yl-amin-Derivate der Formel (I) nach Anspruch 1 auf Schädlinge und/oder ihren Lebensraum einwirken läßt.

**13.** Verfahren zur Herstellung von Schädlingsbekämpfungsmitteln, dadurch gekennzeichnet, daß man 2-Cyclohexen-1-yl-amin-Derivate der Formel (I) nach Anspruch 1 mit Streckmitteln und/oder oberflächenaktiven Mitteln vermischt.

**Patentansprüche für folgenden Vertragsstaat : ES**

**1.** Verwendung von substituierten 2-Cyclohexen-1-yl-amin-Derivaten der Formel (I)

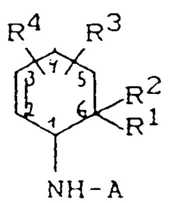

(I)

in welcher

$R^1$ für Wasserstoff, Alkyl oder Halogen steht,

$R^2$ für Formyl, Hydroxyalkyl, Cyano, Nitro oder für einen der Reste $-NHR^5$, $-NR^6R^7$,

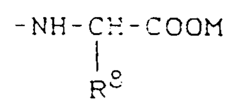,

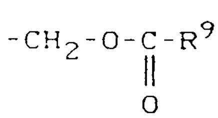, 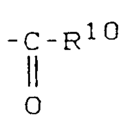, 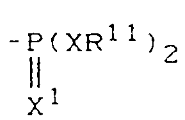, 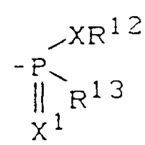,

$-S(O)_nR^{14}$ oder $-CH=CH-R^{15}$ steht,

$R^3$ und $R^4$ gleich oder verschieden sind und jeweils für Wasserstoff, Alkyl, Alkenyl, Alyinyl, Alkoxy, Alkenyloxy, Alkinyloxy, für unsubstituiertes oder substituiertes Aryl, für unsubstituiertes oder substituiertes Aralkyl, für unsubstituiertes oder substituiertes Heteroaryl, für unsubstituiertes oder substituiertes Heterocyclylalkyl, Alkoxyalkyloxy, Halogen oder für einen der Reste $-NH-R^5$, $-NR^6R^7$ oder $-S(O)_n-R^{14}$ stehen

oder

$R^2$ und $R^3$ gemeinsam für einen der Reste

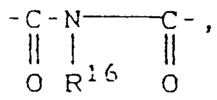, 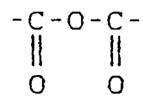 oder 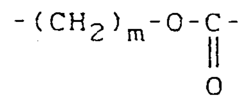 ,

verbrückt über die Positionen 6 und 5, stehen,

oder

$R^3$ und $R^4$ gemeinsam für eine Alkylkette mit 3-oder 4-Kohlenstoffatomen stehen, die über die Positionen 4 und 3 verbunden ist,

$R^5$ für Wasserstoff, Alkyl oder unsubstituiertes oder substituiertes Aryl steht,

$R^6$ für Alkyl oder unsubstituiertes oder substituiertes Aryl steht,

$R^7$ für Alkyl oder unsubstituiertes oder substituiertes Aryl steht,

$R^8$ für Wasserstoff, Alkyl, unsubstituiertes oder substituiertes Aryl oder unsubstituiertes oder substituiertes Aralkyl steht,

$R^9$ für Alkyl oder Alkoxy stellt,

$R^{10}$ für Hydroxy, Hydroxyalkyloxy, Halogenalkyloxy, Alkoxy, Alkoxyalkyloxy, unsubstituiertes oder substituiertes Cycloalkyloxy, unsubstituiertes oder substituiertes Aralkyloxy, unsubstituiertes oder substituiertes Aryloxy, unsubstituiertes oder substituiertes Aralkyl, Alkylthio, unsubstituiertes oder substituiertes Arylthio oder für eine Gruppe -OM, $-NHR^5$, $-NR^6R^7$ oder $-O-Z-NR^5R^6$ steht,

$R^{11}$ für Wasserstoff oder Alkyl steht,

$R^{12}$ für Wasserstoff oder Alkyl steht,

$R^{13}$ für Alkyl steht,

$R^{14}$ für Alkyl, Alkoxy, unsubstituiertes oder substituiertes Aryl oder für die Gruppe -OM steht,

$R^{15}$ für Formyl, Cyano oder für die Gruppierung

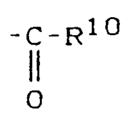

steht,

$R^{16}$ für Wasserstoff, Alkyl oder unsubstituiertes oder substituiertes Aryl steht,

M für Wasserstoff oder für ein Äquivalent eines entsprechenden Alkalimetall-, Erdalkalimetall- oder Ammoniumkations steht,

n für eine Zahl 0, 1 oder 2 steht,

X und $X^1$ gleich oder verschieden sind und für Sauerstoff oder Schwefel stehen,

m für eine Zahl 1 oder 2 steht,

A für Wasserstoff oder eine Aminoschutsgruppe steht und

Z für eine geradkettige oder verzweigte Alkylkette steht,

sowie deren Säureadditions-Salze und Metallsalz-Komplexe zur Bekämpfung von Schädlingen.

**2.** Verfahren zur Herstellung von substituierten 2-Cyclo-hexen-1-yl-amin-Derivaten der Formel (Ia)

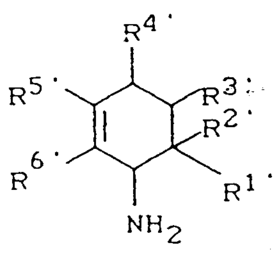

(Ia)

in welcher

$R^{1'}$ für Wasserstoff, Alkyl oder Halogen steht,

$R^{2'}$ für Formyl, Hydroxyalkyl, Cyano, Nitro oder für einen der Reste

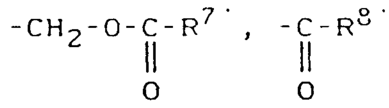

oder $-CH=CH-R^{9'}$ steht,

$R^{3'}$, $R^{4'}$, $R^{5'}$ und $R^{6'}$ gleich oder verschieden sind und jeweils für Wasserstoff, Alkyl, Alkenyl, Alkinyl, Alkoxy, Alkenyloxy, Alkinyloxy, für unsubstituiertes oder substituiertes Aryl, für unsubstituiertes oder substituiertes Aralkyl, für unsubstituiertes oder substituiertes Heteroaryl, für unsubstituiertes oder substituiertes Heterocyclylalkyl, Alkoxyalkyloxy oder für Halogen steht, wobei mindestens zwei der Reste $R^{3'}$, $R^{4'}$, $R^{5'}$ oder $R^{6'}$ für Wasserstoff stehen,

$R^{7'}$ für Alkyl oder Alkoxy steht,

$R^{8'}$ für Hydroxy, Hydroxyalkyloxy, Halogenalkyloxy, Alkoxy, Alkoxyalkyloxy, unsubstituiertes oder substituiertes Cycloalkyloxy, unsubstituiertes oder substituiertes Aralkyloxy, unsubstituiertes oder substituiertes Aryloxy, unsubstituiertes oder substituiertes Aralkyl, Alkylthio, unsubstituiertes oder substituiertes Arylthio oder für eine Gruppe $-O-Z-NR^{11'}R^{12'}$, $-NHR^{10'}$, $-NR^{11'}R^{12'}$ oder -OM steht,

$R^{9'}$ für Formyl, Cyano oder für die Gruppe

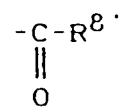

steht,

$R^{10'}$ für Wasserstoff, Alkyl oder unsubstituiertes oder substituiertes Aryl steht,

$R^{11'}$ und $R^{12'}$ gleich oder verschieden sind und jeweils für Alkyl oder unsubstituiertes oder substituiertes Aryl stehen,

Z für eine geradkettige oder verzweigte Alkyl-kette steht und

M für Wasserstoff oder für ein Äquivalent eines entsprechenden Alkalimetall-, Erdalkalimetall-oder Ammoniumkations steht

oder

$R^{2'}$ und $R^{3'}$ gemeinsam für einen der Reste

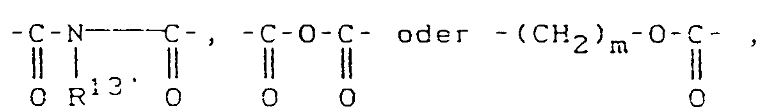

verbrückt über die Positionen 6 und 5, stehen,

worin

$R^{13'}$ für Wasserstoff, Alkyl oder unsubstituiertes oder substituiertes Aryl steht und

m für eine Zahl 1 oder 2 steht,

oder

$R^{4'}$ und $R^{5'}$ gemeinsam für eine Alkylkette mit 3- oder 4-Kohlenstoffatomen stehen, die über die Positionen 4 und 3 verbunden ist,

sowie deren Säureadditions-Salze und Metallsalz-Komplexe ausgenommen die Verbindungen 2-Amino-cyclohex-3-en-carbonsäure und 2-Amino-cyclohex-3-en-carbonsäureethylester sowie deren Hydrochlorid-Salze, dadurch gekennzeichnet, daß man

A) 2-Cyclohexen-1-yl-carbonsäurederivate der Formel (II)

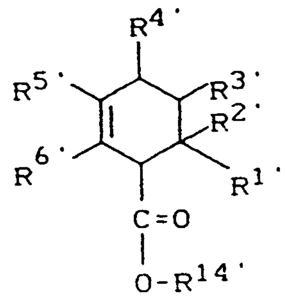

(II)

in welcher
$R^{1'}$, $R^{2'}$, $R^{3'}$, $R^{4'}$, $R^{5'}$ und $R^{6'}$ die oben angegebene Bedeutung haben und
$R^{14'}$ für Wasserstoff, Methyl oder Ethyl steht,
in allgemein üblicher Weise, nach Curtius, gegebenenfalls in Gegenwart eines Verdünnungsmittels und in Gegenwart einer Base bei Temperaturen zwischen -15 °C und +10 °C, mit Chlorameisensäureester versetzt und zu dieser Reaktionsmischung gegebenenfalls in Gegenwart eines Verdünnungsmittels bei Temperaturen zwischen -5 °C und +25 °C ein Azid zugibt,
und das intermediär auftretende Isocyanat der Formel (IIa)

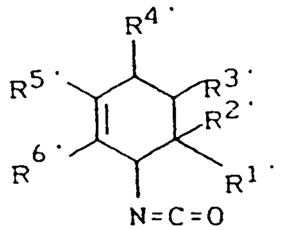

(IIa)

in welcher
$R^{1'}$, $R^{2'}$, $R^{3'}$, $R^{4'}$, $R^{5'}$ und $R^{6'}$ die oben angegebene Bedeutung haben,
mit Wasser, gegebenenfalls in Gegenwart einer Säure oder einer Base, hydrolysiert und die so erhaltenen Amine gegebenenfalls in Säureadditions-Salze und Metallsalz-Komplexe überführt, oder
B) aus den 2-Cyclohexen-Derivaten der Formel (IIb)

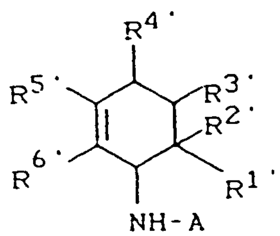

(IIb)

in welcher
$R^{1'}$, $R^{2'}$, $R^{3'}$, $R^{4'}$, $R^{5'}$ und $R^{6'}$ die oben angegebene Bedeutung haben und
A für eine Aminoschutzgruppe steht,
die Aminoschutzgruppe,
in an sich bekannter Weise nach üblichen Methoden, gegebenenfalls in Gegenwart eines geeigneten Lösungs- oder Verdünnungsmittels oder eines Gemisches aus ihnen, unter Kühlen, bei Raumtemperatur oder unter Erwärmen und, falls erforderlich, in einem geschlossenen Gefäss, unter Druck, in einer Inertgasatmosphäre und/oder unter wasserfreien Bedingungen, abspaltet und die so erhaltenen Produkte gegebenenfalls in Säureadditions-Salze oder Metallsalz-Komplexe überführt.

**3.** Verfahren gemäß Anspruch 2, in welchem

$R^{1'}$ für Wasserstoff, geradkettiges oder verzweigtes Alkyl mit 1 bis 6 Kohlenstoffatomen oder Fluor, Chlor oder Brom steht,

$R^{2'}$ für Formyl, geradkettiges oder verzweigtes Hydroxyalkyl mit 1 bis 8 Kohlenstoffatomen im Alkylteil, Cyano, Nitro oder für einen der Reste

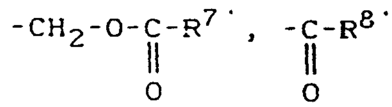

oder $-CH=CH-R^{9'}$ steht,

$R^{3'}$, $R^{4'}$, $R^{5'}$ und $R^{6'}$ gleich oder verschieden sind und jeweils für Wasserstoff, jeweils geradkettiges oder verzweigtes Alkyl oder Alkoxy mit 1 bis 8 Kohlenstoffatomen, jeweils geradkettiges oder verzweigtes Alkenyl, Alkinyl, Alkenyloxy oder Alkinyloxy mit jeweils 2 bis 8 Kohlenstoffatomen, Alkoxyalkyloxy mit jeweils 1 bis 8 Kohlenstoffatomen in den einzelnen Alkylteilen, für jeweils im Arylteil unsubtituiertes oder einfach bis fünffach, gleich oder verschieden substituiertes Aryl oder Aralkyl mit jeweils 6 bis 10 Kohlenstoffatomen im Arylteil und gegebenenfalls 1 bis 4 Kohlenstoffatomen im Alkylteil stehen, wobei als Arylsubstituenten infrage kommen: Halogen, Nitro, Cyano, Amino, $C_1$-$C_4$-Alkyl, $C_1$-$C_4$-Alkoxy, $C_1$-$C_4$-Alkylthio, Halogen-($C_1$-$C_4$)-alkyl, Halogen-($C_1$-$C_4$)-alkoxy, Halogen-($C_1$-$C_4$)-alkylthio mit jeweils 1 bis 9 gleichen oder verschiedenen Halogenatomen und Di-($C_1$-$C_4$)-alkylamino,
weiterhin für eine unsubstituierte oder einfach bis dreifach, gleich oder verschieden substituierte und gegebenenfalls über eine Methylengruppe gebundene heterocyclische 5- oder 6-gliedrige Gruppierung aus der Reihe Furyl, Thienyl, Pyrrolyl, Pyrazolyl, Imidazolyl, 1,2,3- oder 1,2,4-Triazolyl, Oxazolyl, Isoxazolyl, 1,2,4- oder 1,3,4-Oxadiazolyl, Thiazolyl, Isothiazolyl, 1,2,3-, 1,2,4-, 1,2,5- oder 1,3,4-Thiadiazolyl, Pyridyl, Pyridazinyl, Pyrimidinyl und Pyrazinyl stehen, wobei als Substituenten für den Heterocyclus jeweils infrage kommen: Halogen, Nitro, Cyano, Amino, $C_1$-$C_4$-Alkyl, $C_1$-$C_4$-Alkoxy oder $C_1$-$C_4$-Alkylthio, Halogen-($C_1$-$C_4$)-alkyl, Halogen-($C_1$-$C_4$)-alkoxy, Halogen-($C_1$-$C_4$)-alkylthio mit jeweils 1 bis 9 gleichen oder verschiedenen Halogenatomen und Di-($C_1$-$C_4$)-alkylamino,
weiterhin für Fluor, Chlor oder Brom stehen, wobei mindestens zwei der Reste $R^{3'}$, $R^{4'}$, $R^{5'}$ oder $R^{6'}$ für Wasserstoff stehen,

$R^{7'}$ für jeweils geradkettiges oder verzweigtes Alkyl oder Alkoxy mit 1 bis 6 Kohlenstoffatomen steht,

$R^{8'}$ für Hydroxy, geradkettiges oder verzweigtes Hydroxyalkyloxy mit 1 bis 8 Kohlenstoffatomen, geradkettiges oder verzweigtes Halogenalkyloxy mit 1 bis 8 Kohlenstoffatomen und 1 bis 17 gleichen oder verschiedenen Halogenatomen, unsubstituiertes oder einfach bis mehrfach, gleich oder verschieden durch Halogen substituiertes Cycloalkyloxy mit 3 bis 6 Kohlenstoffatomen, jeweils geradkettiges oder verzweigtes Alkoxy oder Alkylthio mit 1 bis 6 Kohlenstoffatomen, geradkettiges oder verzweigtes Alkoxyalkyloxy mit jeweils 1 bis 6 Kohlenstoffatomen im Alkoxy- oder Alkylteil, jeweils im Arylteil unsubstituiertes oder einfach bis fünffach, gleich oder verschieden substituiertes Aryloxy, Arylthio, Aralkyl oder Aralkyloxy mit jeweils 6 bis 10 Kohlenstoffatomen im Arylteil und gegebenenfalls 1 bis 8 Kohlenstoffatomen im Alkylteil steht, wobei als Arylsubstituenten die oben aufgeführten Arylsubstituenten in Frage kommen oder für eine Gruppe $-O-Z-NR^{11'}R^{12'}$, $-NHR^{10'}$, $-NR^{11'}R^{12'}$ oder -OM steht,

$R^{9'}$ für Formyl, Cyano oder für die Gruppe

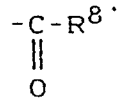

steht,

$R^{10'}$ für Wasserstoff, geradkettiges oder verzweigtes Alkyl mit 1 bis 6 Kohlenstoffatomen oder unsubstituiertes oder einfach bis fünffach, gleich oder verschieden substituiertes Aryl mit 6 bis 10 Kohlenstoffatomen steht, wobei als Arylsubstituenten die oben aufgeführten Arylsubstituenten in Frage kommen,

$R^{11'}$ für geradkettiges oder verzweigtes Alkyl mit 1 bis 6 Kohlenstoffatomen oder unsubstituiertes oder einfach bis fünffach, gleich oder verschieden substituiertes Aryl mit 6 bis 10 Kohlenstoffatomen steht, wobei als Arylsubstituenten die oben aufgeführten Arylsubstituenten in Frage kommen,

$R^{12'}$ für geradkettiges oder verzweigtes Alkyl mit 1 bis 6 Kohlenstoffatomen oder unsubstituiertes oder einfach bis fünffach, gleich oder verschieden substituiertes Aryl mit 6 bis 10 Kohlenstoffatomen steht, wobei als Arylsubstituenten die oben aufgeführten Arylsubstituenten infrage kommen,

M für Wasserstoff oder für ein Äquivalent eines entsprechenden Alkalimetall-, Erdalkalimetall- oder Ammoniumkations steht und

Z für eine geradkettige oder verzweigte Alkylkette mit 1 bis 8 Kohlenstoffatomen steht

oder

$R^{2'}$ und $R^{3'}$ gemeinsam für einen der Reste

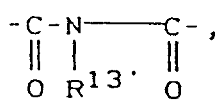 , 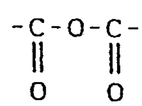 oder 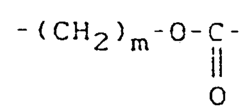 ,

verbrückt über die Positionen 6 und 5, stehen,

worin

$R^{13'}$ für Wasserstoff, geradkettiges oder verzweigtes Alkyl mit 1 bis 6 Kohlenstoffatomen oder unsubstituiertes oder einfach bis fünffach, gleich oder verschieden substituiertes Aryl mit 6 bis 10 Kohlenstoffatomen steht, wobei als Arylsubstituenten die oben aufgeführten Arylsubstituenten in Frage kommen und

m für eine Zahl 1 oder 2 steht,

oder

$R^{4'}$ und $R^{5'}$ gemeinsam für eine Alkylkette mit 3- oder 4-Kohlenstoffatomen steht, die über die Positionen 4 und 3 verbunden ist

sowie deren Säureadditions-Salze und Metallsalz-Komplexe.

**4.** Verfahren gemäß Anspruch 2, in welchen

$R^{1'}$ für Wasserstoff, geradkettiges oder verzweigtes Alkyl mit 1 bis 4 Kohlenstoffatomen oder Fluor, Chlor oder Brom steht,

$R^{2'}$ für Formyl, geradkettiges oder verzweigtes Hydroxyalkyl mit 1 bis 4 Kohlenstoffatomen im Alkylteil, Cyano, Nitro oder für einen der Reste

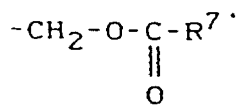 , 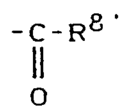

oder $-CH=CH-R^{9'}$ steht,

$R^{3'}$, $R^{4'}$, $R^{5'}$ und $R^{6'}$ gleich oder verschieden sind und jeweils für Wasserstoff, jeweils geradkettiges oder verzweigtes Alkyl oder Alkoxy mit 1 bis 6 Kohlenstoffatomen, jeweils geradkettiges oder verzweigtes Alkenyl, Alkinyl, Alkenyloxy oder Alkinyloxy mit jeweils 2 bis 6 Kohlenstoffatomen, Alkoxyalkyloxy mit jeweils 1 bis 6 Kohlenstoffatomen in den einzelnen Alkylteilen, für jeweils im Phenylteil unsubstituiertes oder einfach bis dreifach, gleich oder verschieden substituiertes Phenyl oder Phenylalkyl mit gegebenenfalls 1 oder 2 Kohlenstoffatomen im Alkylteil stehen, wobei als Phenylsubstituenten infrage kommen: Fluor, Chlor, Brom, Nitro, Cyano, Amino, $C_1$-$C_2$-Alkyl, $C_1$-$C_3$-Alkoxy, $C_1$-$C_2$-Alkylthio, Halogen-($C_1$-$C_2$)-alkyl, Halogen-($C_1$-$C_2$)-alkoxy, Halogen-($C_1$-$C_2$)-alkylthio mit jeweils 1 bis 5 gleichen oder verschiedenen Fluor- und/ oder Chloratomen und Di-($C_1$-$C_2$)-alkylamino, weiterhin für eine unsubstituierte oder einfach bis dreifach, gleich oder verschieden substituierte und gegebenenfalls über eine Methylengruppe gebundene heterocyclische fünf- bzw. sechsgliedrige Gruppierung aus der Reihe Furyl, Thienyl, Pyrrolyl, Pyrazolyl, Imidazolyl, 1,2,3- oder 1,2,4-Triazolyl, Oxazolyl, Isoxazolyl, 1,2,4- oder 1,3,4-Oxadiazolyl, Thiazolyl, Isothiazolyl, 1,2,3-, 1,2,4-, 1,2,5- oder 1,3,4-Thiadiazolyl, Pyridyl, Pyridazinyl, Pyrimidinyl und Pyrazinyl stehen, wobei als Substituenten für den Heterocyclus jeweils in Frage kommen: Fluor, Chlor, Brom, Nitro, Cyano, Amino, $C_1$-$C_2$-Alkyl, $C_1$-$C_2$-Alkoxy oder $C_1$-$C_2$-Alkylthio, Halogen-($C_1$-$C_2$)-alkyl, Halogen-($C_1$-$C_2$)-alkoxy, Halogen-($C_1$-$C_2$)-alkylthio mit jeweils 1 bis 5 gleichen oder verschiedenen Fluor- und/oder Chloratomen und Di-($C_1$-$C_2$)-alkylamino,

weiterhin für Fluor, Chlor oder Brom stehen,

wobei mindestens zwei der Reste $R^{3'}$, $R^{4'}$, $R^{5'}$ und $R^{6'}$ für Wasserstoff stehen,

$R^{7'}$ für jeweils geradkettiges oder verzweigtes Alkyl oder Alkoxy mit 1 bis 4 Kohlenstoffatomen steht,

$R^{8'}$ für Hydroxy, geradkettiges oder verzweigtes Hydroxyalkyloxy mit 1 bis 6 Kohlenstoffatomen, geradkettiges oder verzweigtes Halogenalkyloxy mit 1 bis 6 Kohlenstoffatomen und 1 bis 13 gleichen oder verschiedenen Halogenatomen, unsubstituiertes oder einfach bis dreifach, gleich oder verschieden durch Fluor, Chlor, Brom substituiertes Cycloalkyloxy mit 3 bis 6 Kohlenstoffatomen, jeweils geradkettiges oder verzweigtes Alkoxy oder Alkylthio mit 1 bis 4 Kohlenstoffatomen, geradkettiges oder verzweigtes Alkoxyalkyloxy mit jeweils 1 bis 4 Kohlenstoffatomen im Alkoxy- oder Alkylteil, jeweils im Phenylteil unsubstituiertes oder einfach bis dreifach, gleich oder verschieden substituiertes Phenyloxy, Phenylthio, Phenylalkyl oder Phenylalkyloxy mit jeweils gegebenenfalls 1 bis 6 Kohlenstoffatomen im Alkylteil steht, wobei als Phenylsubstituenten die oben aufgeführten Phenylsubstituenten in Frage kommen

oder für eine Gruppe -O-Z-$NR^{11'}R^{12'}$, -$NHR^{10'}$, -$NR^{11'}R^{12'}$ oder -OM steht,

$R^{9'}$ für Formyl, Cyano oder für die Gruppe

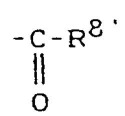

steht,

$R^{10'}$ für Wasserstoff, geradkettiges oder verzweigtes Alkyl mit 1 bis 4 Kohlenstoffatomen oder unsubstituiertes oder einfach bis dreifach, gleich oder verschieden substituiertes Phenyl steht, wobei als Phenylsubstituenten die oben aufgeführten Phenylsubstituenten in Frage kommen,

$R^{11'}$ für geradkettiges oder verzweigtes Alkyl mit 1 bis 4 Kohlenstoffatomen oder unsubstituiertes oder einfach bis dreifach, gleich oder verschieden substituiertes Phenyl steht, wobei als Phenylsubstituenten die oben aufgeführten Phenylsubstituenten in Frage kommen,

$R^{12'}$ für geradkettiges oder verzweigtes Alkyl mit 1 bis 4 Kohlenstoffatomen oder

unsubstituiertes oder einfach bis dreifach, gleich oder verschieden substituiertes Phenyl steht, wobei als Phenylsubstituenten die oben aufgeführten Phenylsubstituenten in Frage kommen,

M für Wasserstoff oder für ein Äquivalent eines entsprechenden Alkalimetall-, Erdalkalimetall- oder Ammoniumkations steht und

Z für eine geradkettige oder verzweigte Alkylkette mit 1 bis 6 Kohlenstoffatomen steht

oder

$R^{2'}$ und $R^{3'}$ gemeinsam für einen der Reste

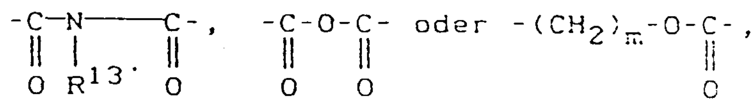

verbrückt über die Positionen 6 und 5, stehen,

$R^{13'}$ für Wasserstoff, geradkettiges oder verzweigtes Alkyl mit 1 bis 6 Kohlenstoffatomen oder unsubstituiertes oder einfach bis dreifach, gleich oder verschieden substituiertes Phenyl steht, wobei als Phenylsubstituenten die oben aufgeführten Phenylsubstituenten in Frage kommen und

m für eine Zahl 1 oder 2 steht,

oder

$R^{4'}$ und $R^{5'}$ gemeinsam für eine Alkylkette mit 3- oder 4-Kohlenstoffatomen steht, die über die Positionen 4 und 3 verbunden ist,

sowie deren Saüreadditions-Salze und Metallsalz-Komplexe.

**5.** Verfahren gemäß Anspruch 2 in welchem die Verbindung der Formel (Ia) das Hydrochlorid des cis-Diastereomeren der 2-Amino-cyclohex-3-en-carbonsäure ist.

**6.** Verfahren gemäß Anspruch 2 in welchem die Verbindung der Formel (Ia) die Hydrochloride der Enantiomeren der 2-Amino-cyclohex-3-en-carbonsäure vom Schmelzpunkt 210 - 213,5° C und 208 - 210° C sind.

**7.** Verfahren gemäß Anspruch 2 in welchem die Verbindung der Formel (Ia) die Isomere 2-Amino-Cyclohex-3-en-carbonsäure vom Schmelzpunkt 98° C ist.

**8.** Verfahren gemäß Anspruch 2 in welchem die Verbindung der Formel (Ia) das Hydrochlorid des Isomeren des 2-Amino-cyclohex-3-en-carbonsäureethylester vom Schmelzpunkt 139 - 148° C ist.

**9.** Schädlingsbekämpfungsmittel, gekennzeichnet durch einen Gehalt an mindestens einem 2-Cyclohexen-1-yl-amin-Derivat der Formel (I) nach Anspruch 1 und unter Verwendung von oberflächenaktiven Mitteln.

**10.** Verfahren zur Bekämpfung von Schädlingen, dadurch gekennzeichnet, daß man 2-Cyclohexen-1-yl-amin-Derivate der Formel (I) nach Anspruch 1 auf Schädlinge und/oder ihren Lebensraum einwirken läßt.

**11.** Verfahren zur Herstellung von Schädlingsbekämpfungsmitteln, dadurch gekennzeichnet, daß man 2-Cyclohexan-1-yl-amim-Derivate der Formel (I) nach Anspruch 1 mit Streckmitteln und/oder oberflächenaktiven Mitteln vermischt.

**12.** Verfahren zur Herstellung der 2-Cyclohexan-Derivate der Formel (IIb)

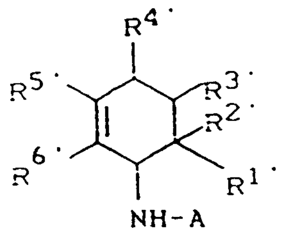

(IIb)

in welcher

$R^{1'}$, $R^{2'}$, $R^{3'}$, $R^{4'}$, $R^{5'}$ und $R^{6'}$ die in Anspruch 2 angegebene Bedeutung haben und

A für eine Aminoschutzgruppe steht,

ausgenommen die Verbindungen und ihre Enantiomeren und Isomeren: 6-Formyl-5-{[(phenylmethoxy)-carbonyl]-amino}-3-cyclohexen-1-carbonsäuremethylester, 6-[3-Oxo-1-propenyl)-5-{[(phenylmethoxy)-carbonyl]-amino}-3-cyclohexen-1-carbonsäuremethylester , 3-Cyclohexen-2-[(trichloracetyl)-amino]-1-carbonsäuremethylester, 2,2,2-Trichlor-N-(6-formyl-2-cyclohexen-1-yl)-acetamid, (6-Formyl-5-methyl-2-cyclohexen-1-yl)-carbaminsäureethylester, 2-[(Ethoxy-carbonyl)-amino]-6-methyl-3-cyclohexen-1-carbonsäuremethylester, 2-[(Ethoxycarbonyl)-amino]-5-methyl-3-cyclohexen-1-carbonsäuremethylester, 2-[(Ethoxycarbonyl)-amino]-3-cyclohexen-1-carbonsäuremethylester, 3-{2-[(Ethoxycarbonyl)-amino] -6-methyl-3-cyclohexan-1-yl}-2-propensäureethylester, (6-Formyl-5-propyl-2-cyclohexen-1-yl)-carbaminsäurephenylmethylester, (6-Formyl-5-methyl-2-cyclohexen-1-yl)-carbaminsäurephenylmethylester, 2-[-(Phenoxycarbonyl)-amino]-3-cyclohexen-1-carbonsäuremethylester, 3-⟨6-Methyl-2-{[(phenylmethoxy)-carbonyl]-amino}-3-cyclohexen-1-yl⟩-2-propensäureethylester, ⟨6-Formyl-5-pentyl-2-cyclohexen-1-yl)-carbaminsäurephenylmethylester Phenyl-6-carbomethoxy-2-cyclohexen-1-yl-thiocarbamat, N-(6-carbomethoxy-2-cyclohexan-1-yl)-1-pyrrolidin-carboxamid, 2-[(p-Toluolsulfonyl)-amino]-3-cyclohexen-1 -1-carbonsäuremethylester, -carbonsäure-t-butylester, -carbonsäure-benzylester und -carbonsäure-2,4,6-trimethylphenylester und {6-Formyl-5-[2-(methoxymethoxy)-ethyl]-2-cyclohexen-1-yl}-carbaminsäurephenylmethylester, dadurch gekennzeichnet, daß man Dienophile der Formel (III)

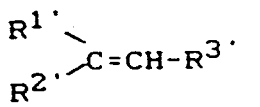

(III)

in welcher

$R^{1'}$, $R^{2'}$ und $R^{3'}$ die oben angegebene Bedeutung haben

(B/a)mit N-Acyl-1-amino-1,3-butadien-Derivaten der Formel (IVa)

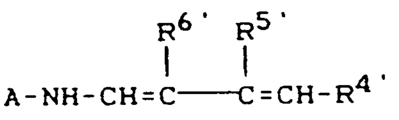

(IVa)

in welcher

$R^{4'}$, $R^{5'}$, $R^{6'}$ und A die oben angegebene Bedeutung haben,

gegebenenfalls in Gegenwart eines Verdünnungsmittels, gegebenenfalls in Gegenwart eines Katalysators, gegebenenfalls in Gegenwart eines Inertgases und gegebenenfalls unter Druck, cyclisiert oder

(B/b)mit substituierten Butadienen der Formel (IVb)

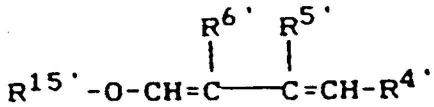

(IVb)

in welcher
$R^{4'}$, $R^{5'}$ und $R^{6'}$ die oben angegebene Bedeutung haben und
$R^{15'}$ für den Acetyl- oder Trimethylsilylrest steht,
zunächst in einer ersten Stufe gegebenenfalls in Gegenwart eines Verdünnungsmittels, gegebenenfalls in Gegenwart eines Katalysators, gegebenenfalls in Gegenwart eines Inertgases und gegebenenfalls unter Druck, cyclisiert
und die so erhältlichen 2-Cyclohexen-Derivate der Formel (IIc)

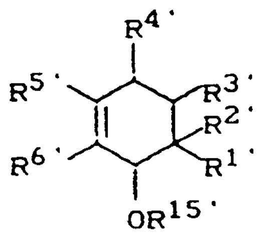

(IIc)

in welcher
$R^{1'}$, $R^{2'}$, $R^{3'}$, $R^{4'}$, $R^{5'}$ und $R^{6'}$ die oben angegebene Bedeutung haben und
$R^{15'}$ für den Acetyl- oder Trimethylsilylrest steht
in einer zweiten Stufe mit 4,4'-Dimethoxybenzhydrylamin (DMB) der Formel (V)

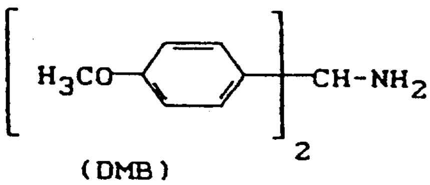

(DMB) (V)

gegebenenfalls in Gegenwart eines Verdünnungsmittels bei Temperaturen von 0 °C bis zur Siedetemperatur des jeweils verwendeten Verdünnungsmittels und in Gegenwart eines Katalysators umsetzt
oder
(B/c)die nach Verfahren (A) intermediär auftretenden Isocyanate der Formel (IIa)

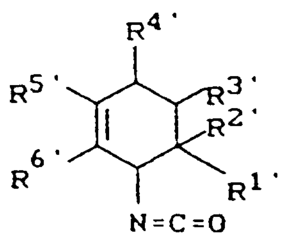

(IIa)

in welcher
$R^{1'}$, $R^{2'}$, $R^{3'}$, $R^{4'}$, $R^{5'}$ und $R^{6'}$ die oben angegebene Bedeutung haben,

mit Alkoholen der Formel (VII)

$R^{16'}$-OH (VII)

in welcher

$R^{16'}$ für jeweils geradkettiges oder verzweigtes, gegebenenfalls durch Halogen substituiertes Alkyl, Alkenyl, Alkinyl oder Alkoxyalkyl mit 1 bis 12 Kohlenstoffatomen (vorzugsweise 1 bis 6 Kohlenstoffatomen), unsubstituiertes oder substituiertes Phenyl oder Benzyl steht,

umsetzt,

oder

(B/d)man erhält die substituierten 2-Cyclohexen-Derivate der Formel (IIb), wenn man die nach Verfahren (B/a), (B/b) oder (B/c) erhältlichen 2-Cyclohexen-Derivate der Formel (IId)

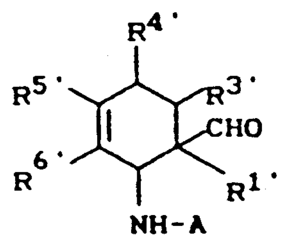

(IId)

in welcher

$R^{1'}$, $R^{3'}$, $R^{4'}$, $R^{5'}$, $R^{6'}$ und A die oben angegebene Bedeutung haben,

mit einem komplexen Metallhydrid, wie beispielsweise Natriumborhydrid, in einem geeigneten Lösungsmittel bei Temperaturen von 0 °C bis 20 °C reduziert und die so erhältlichen 2-Cyclohexen-1-yl-amin-alkohole der Formel (IIe)

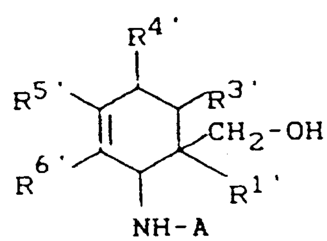

(IIe)

in welcher

$R^{1'}$, $R^{3'}$, $R^{4'}$, $R^{5'}$, $R^{6'}$ und A die oben angegebene Bedeutung haben,

durch weitere Umsetzungen an der Hydroxy-Gruppe in Ester und Ether oder Carbamoylchloriden Acyl- oder Carbamoyl-Derivate der Verbindungen der Formel (IIb) überführt,

oder

(B/e)man erhält die substituierten 2-Cyclohexen-Derivate der Formel (IIb), wenn man die nach Verfahren (B/a), (B/b) oder (B/c) erhältlichen 2-Cyclohexen-Derivate der Formel (IId)

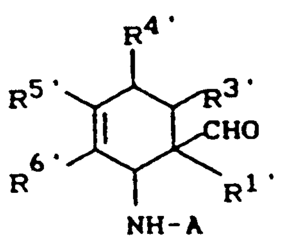

(IId)

in welcher

$R^{1'}$, $R^{3'}$, $R^{4'}$, $R^{5'}$, $R^{6'}$ und A die oben angegebene Bedeutung haben,
mit Alkanphosphonsäure-Derivaten der Formel (VIII)

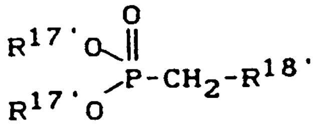 (VIII)

in welcher
$R^{17'}$ für Methyl oder Ethyl steht und
$R^{18'}$ für die Cyano- oder für die Alkoxycarbonylgruppe steht
gegebenenfalls in Gegenwart eines Verdünnungsmittels, gegebenenfalls in Gegenwart einer Base und gegebenenfalls in Gegenwart eines Inertgases, umsetzt.

**Claims**
**Claims for the following Contracting States : BE, CH, DE, FR, GB, IT, LI, NL**

**1.** Use of substituted 2-cyclohexen-1-yl-amine derivatives of the formula (I)

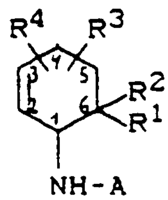 (I)

in which
$R^1$ represents hydrogen, alkyl or halogen,
$R^2$ represents formyl, hydroxyalkyl, cyano or nitro, or represents one of the radicals $-NHR^5$, $-NR^6R^7$,

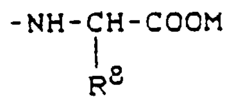,

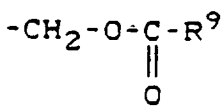, 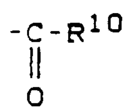, 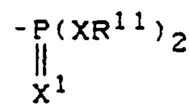, 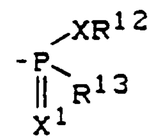,

$-S(O)_nR^{14}$ or $-CH=CH-R^{15}$,
$R^3$ and $R^4$ are identical or different and in each case represent hydrogen, alkyl, alkenyl, alkinyl, alkoxy, alkenyloxy or alkinyloxy, or represent unsubstituted or substituted aryl, or represent unsubstituted or substituted aralkyl, or represent unsubstituted or substituted heteroaryl, or represent unsubstituted or substituted heterocyclylalkyl, alkoxyalkyloxy or halogen, or represent one of the radicals $-NH-R^5$, $-NR^6R^7$ or $-S(O)_n-R^{14}$,
or
$R^2$ and $R^3$ together represent one of the radicals

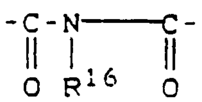, 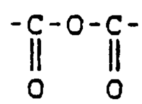 or 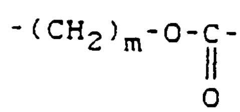,

which are bridged via the 6- and 5-positions,

or

$R^3$ and $R^4$ together represent an alkyl chain which has 3 or 4 carbon atoms and which is linked via the 4- and 3-positions,

$R^5$ represents hydrogen, alkyl or unsubstituted or substituted aryl,

$R^6$ represents alkyl or unsubstituted or substituted aryl,

$R^7$ represents alkyl or unsubstituted or substituted aryl,

$R^8$ represents hydrogen, alkyl, unsubstituted or substituted aryl or unsubstituted or substituted aralkyl,

$R^9$ represents alkyl or alkoxy,

$R^{10}$ represents hydroxyl, hydroxyalkyloxy, halogenoalkyloxy, alkoxy, alkoxyalkyloxy, unsubstituted or substituted cycloalkyloxy, unsubstituted or substituted aralkyloxy, unsubstituted or substituted aryloxy, unsubstituted or substituted aralkyl, alkylthio or unsubstituted or substituted arylthio, or represents a group -OM, -$NHR^5$, -$NR^6R^7$ or -O-Z-$NR^5R^6$,

$R^{11}$ represents hydrogen or alkyl,

$R^{12}$ represents hydrogen or alkyl,

$R^{13}$ represents alkyl,

$R^{14}$ represents alkyl, alkoxy or unsubstituted or substituted aryl, or represents the group -OH,

$R^{15}$ represents formyl or cyano, or represents the group

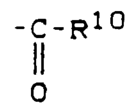

$R^{16}$ represents hydrogen, alkyl or unsubstituted or substituted aryl,

M represents hydrogen or represents an equivalent of a corresponding alkali metal cation, alkaline earth metal cation or ammonium cation,

n represents a number 0, 1 or 2,

X and $X^1$ are identical or different and represent oxygen or sulphur,

m represents a number 1 or 2,

A represents hydrogen or an amino-protecting group and

Z represents a straight-chain or branched alkyl chain,

and their acid addition salts and metal salt complexes for combating pests.

**2.** Substituted 2-cyclohexen-1-yl-amine derivatives of the formula (Ia)

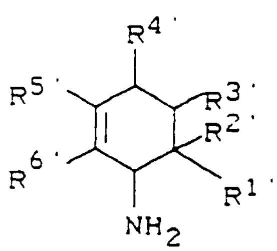

(Ia)

in which

$R^{1'}$ represents hydrogen, alkyl or halogen,

$R^{2'}$ represents formyl, hydroxyalkyl, cyano or nitro, or represents one of the radicals

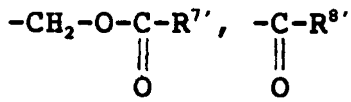

or $-CH=CH-R^{9'}$,

$R^{3'}$, $R^{4'}$, $R^{5'}$ and $R^{6'}$ are identical or different and in each case represent hydrogen, alkyl, alkenyl, alkinyl, alkoxy, alkenyloxy and alkinyloxy, or represent unsubstituted or substituted aryl, or represent unsubstituted or substituted aralkyl, or represent unsubstituted or substituted heteroaryl, or represent unsubstituted or substituted heterocyclylalkyl, or represent alkoxyalkyloxy, or represent halogen,
where at least two of the radicals $R^{3'}$, $R^{4'}$, $R^{5'}$ or $R^{6'}$ represent hydrogen,

$R^{7'}$ represents alkyl or alkoxy,

$R^{8'}$ represents hydroxyl, hydroxyalkyloxy, halogenoalkyloxy, alkoxy or alkoxyalkyloxy, unsubstituted or substituted cycloalkyloxy, unsubstituted or substituted aralkyloxy, unsubstituted or substituted aryloxy, unsubstituted or substituted aralkyl, alkylthio or unsubstituted or substituted arylthio, or represents a group $-O-Z-NR^{11'}R^{12'}$, $-NHR^{10'}$, $-NR^{11'}R^{12'}$ or $-OM$,

$R^{9'}$ represents formyl or cyano, or represents the group

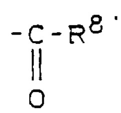

$R^{10'}$ represents hydrogen, alkyl or unsubstituted or substituted aryl,

$R^{11'}$ and $R^{12'}$ are identical or different and in each case represent alkyl or unsubstituted or substituted aryl,

Z represents a straight-chain or branched alkyl chain and

M represents hydrogen, or represents an equivalent of a corresponding alkali metal cation, alkaline earth metal cation or ammonium cation

or

$R^{2'}$ and $R^{3'}$ together represent one of the radicals

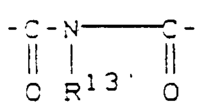 , 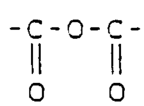 or 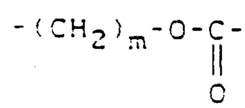 ,

bridged via the 6- and 5-positions,

where

$R^{13'}$ represents hydrogen, alkyl or unsubstituted or substituted aryl and

m represents a number 1 or 2,

or

$R^{4'}$ and $R^{5'}$ together represent an alkyl chain which has 3 or 4 carbon atoms and which is linked via the 4- and 3-positions,

and their acid addition salts and metal salt complexes, with the exception of the compounds 2-amino-cyclohex-3-enecarboxylic acid and ethyl 2-amino-cyclohex-3-enecarboxylate.

**3.** Substituted 2-cyclohexen-1-yl-amine derivatives of the formula (Ia) according to Claim 2, in which

$R^{1'}$ represents hydrogen, straight-chain or branched alkyl having 1 to 4 carbon atoms, or fluorine, chlorine or bromine,

$R^{2'}$ represents formyl, straight-chain or branched hydroxyalkyl having 1 to 8 carbon atoms in the alkyl moiety, cyano or nitro, or represents one of the radicals

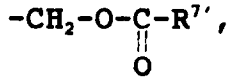, 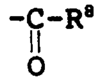

or $-CH=CHR^{9'}$,

$R^{3'}$, $R^{4'}$, $R^{5'}$ and $R^{6'}$ are identical or different and in each case represent hydrogen, in each case straight-chain or branched alkyl or alkoxy having 1 to 8 carbon atoms, in each case straight-chain or branched alkenyl, alkinyl, alkenyloxy or alkinyloxy, in each case having 2 to 8 carbon atoms, or alkoxyalkyloxy, in each case having 1 to 8 carbon atoms in the individual alkyl moieties, or represents aryl or aralkyl, having in each case 6 to 10 carbon atoms in the aryl moiety and where appropriate 1 to 4 carbon atoms in the alkyl moiety, and in each case being unsubstituted or monosubstituted to pentasubstituted in the aryl moiety by identical or different substituents, suitable aryl substituents being: halogen, nitro, cyano, amino, $C_1$-$C_4$-alkyl, $C_1$-$C_4$-alkoxy, $C_1$-$C_4$-alkylthio, halogeno-($C_1$-$C_4$)-alkyl, halogeno-($C_1$-$C_4$)-alkoxy, halogeno-($C_1$-$C_4$)-alkylthio, each having 1 to 9 identical or different halogen atoms, and di-($C_1$-$C_4$)-alkylamino,
furthermore represent a heterocyclic 5- or 6-membered group from the series comprising furyl, thienyl, pyrrolyl, pyrazolyl, imidazolyl, 1,2,3- or 1,2,4-triazolyl, oxazolyl, isoxazolyl, 1,2,4- or 1,3,4-oxadiazolyl, thiazolyl, isothiazolyl, 1,2,3-, 1,2,4-, 1,2,5- or 1,3,4-thiadiazolyl, pyridyl, pyridazinyl, pyrimidinyl and pyrazinyl, which group is unsubstituted or monosubstituted to trisubstituted by identical or different substituents and where appropriate linked via a methylene group, suitable substituents on the heterocycle in each case being: halogen, nitro, cyano, amino, $C_1$-$C_4$-alkyl, $C_1$-$C_4$-alkoxy or $C_1$-$C_4$-alkylthio, halogeno-($C_1$-$C_4$)-alkyl halogeno-($C_1$-$C_4$)-alkoxy, halogeno-($C_1$-$C_4$)-alkylthio, each having 1 to 9 identical or different halogen atoms, and di-($C_1$-$C_4$)-alkylamino, furthermore represent fluorine, chlorine or bromine,
where at least two of the radicals $R^{3'}$, $R^{4'}$, $R^{5'}$ or $R^{6'}$ represent hydrogen,

$R^{7'}$ represents in each case straight-chain or branched alkyl or alkoxy having 1 to 6 carbon atoms,

$R^{8'}$ represents hydroxyl, straight-chain or branched hydroxyalkyloxy having 1 to 8 carbon atoms, straight-chain or branched halogenoalkyloxy having 1 to 8 carbon atoms and 1 to 17 identical or different halogen atoms, cycloalkyloxy having 3 to 6 carbon atoms which is unsubstituted or monosubstituted to polysubstituted by identical or different halogen substituents, in each case straight-chain or branched alkoxy or alkylthio, aralkyl having 1 to 6 carbon atoms, or straight-chain or branched alkoxyalkyloxy, in each case having 1 to 6 carbon atoms in the alkoxy moiety or alkyl moiety, or aryloxy, arylthio, aralkyl or aralkyloxy, in each case having 6 to 10 carbon atoms in the aryl moiety and where appropriate 1 to 8 carbon atoms in the alkyl moiety, and in each case being unsubstituted or monosubstituted to pentasubstituted in the aryl moiety by identical or different substituents, suitable aryl substituents being the aryl substituents mentioned above, or represents a group $-O-Z-NR^{11'}R^{12'}$, $-NHR^{10'}$, $-NR^{11'}R^{12'}$, or -OM,

$R^{9'}$ represents formyl or cyano, or represents the group

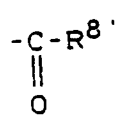

$R^{10'}$ represents hydrogen, straight-chain or branched alkyl having 1 to 6 carbon atoms, or aryl which has 6 to 10 carbon atoms and which is unsubstituted or monosubstituted to pentasubstituted by identical or different substituents,

suitable aryl substituents being the aryl substituents mentioned above,

$R^{11'}$ represents straight-chain or branched alkyl having 1 to 6 carbon atoms, or aryl which has 6 to 10 carbon atoms and which is unsubstituted or monosubstituted to pentasubstituted by identical or different substituents, suitable aryl substituents being the aryl substituents mentioned above,

$R^{12'}$ represents straight-chain or branched alkyl having 1 to 6 carbon atoms, or aryl which has 6 to 10 carbon atoms and which is unsubstituted or monosubstituted to pentasubstituted by identical or different substituents, suitable aryl substituents being the aryl substituents mentioned above,

M represents hydrogen, or represents an equivalent of a corresponding alkali metal cation, alkaline earth metal cation or ammonium cation, and

Z represents a straight-chain or branched alkyl chain having 1 to 8 carbon atoms

or

$R^{2'}$ and $R^{3'}$ together represent one of the radicals

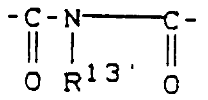, 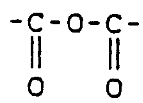 or 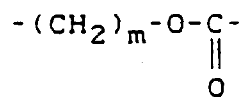 ,

bridged via the 6- and 5-positions,

where

$R^{13'}$ represents hydrogen, straight-chain or branched alkyl having 1 to 6 carbon atoms or aryl which has 6 to 10 carbon atoms and which is unsubstituted or monosubstituted to pentasubstituted by identical or different substituents, suitable aryl substituents being the aryl substituents mentioned above, and

m represents a number 1 or 2,

or

$R^{4'}$ and $R^{5'}$ together represents an alkyl chain which has 3 or 4 carbon atoms and which is linked via the 4- and 3-positions,

and their acid addition salts and metal salt complexes.

**4.** Substituted 2-cyclohexen-1-yl-amine derivatives of the formula (Ia) according to Claim 2, in which

$R^{1'}$ represents hydrogen, straight-chain or branched alkyl having 1 to 4 carbon atoms, or fluorine, chlorine or bromine,

$R^{2'}$ represents formyl, straight-chain or branched hydroxyalkyl having 1 to 4 carbon atoms in the alkyl moiety, cyano or nitro, or represents one of the radicals

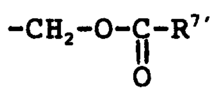, 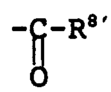

or $-CH=CH-R^{9'}$,

$R^{3'}$, $R^{4'}$, $R^{5'}$ and $R^{6'}$ are identical or different and in each case represent hydrogen, in each case straight-chain or branched alkyl or alkoxy having 1 to 6 carbon atoms, in each case straight-chain or branched alkenyl, alkinyl, alkenyloxy or alkinyloxy, in each case having 2 to 6 carbon atoms, or alkoxyalkyloxy, in each case having 1 to 6 carbon atoms in the individual alkyl moieties, or represent phenyl or phenylalkyl, where appropriate having 1 or 2 carbon atoms in the alkyl moiety, and in each case being unsubstituted or monosubstituted to trisubstituted in the phenyl moiety by identical or different substituents, suitable phenyl substituents being: fluorine, chlorine, bromine, nitro, cyano, amino, $C_1$-$C_2$-alkyl, $C_1$-$C_3$-alkoxy, $C_1$-$C_2$-alkylthio, halogeno-($C_1$-$C_2$)-alkyl, halogeno-($C_1$-$C_2$)-alkoxy and halogeno-($C_1$-$C_2$)-alkylthio, in each case having

1 to 5 identical or different fluorine and/or chlorine atoms, and di-($C_1$-$C_2$)-alkylamino, furthermore represent a heterocyclic five- or six-membered group from the series comprising furyl, thienyl, pyrrolyl, pyrazolyl, imidazolyl, 1,2,3- or 1,2,4-triazolyl, oxazolyl, isoxazolyl, 1,2,4- or 1,3,4-oxadiazolyl, thiazolyl, isothiazolyl, 1,2,3-, 1,2,4-, 1,2,5- or 1,3,4-thiadiazolyl, pyridyl, pyridazinyl, pyrimidinyl and pyrazinyl, which group is unsubstituted or monosubstituted to trisubstituted by identical or different substituents and where appropriate bonded via a methylene group, suitable substituents on the heterocycle in each case being: fluorine, chlorine, bromine, nitro, cyano, amino, $C_1$-$C_2$-alkyl, $C_1$-$C_2$-alkoxy or $C_1$-$C_2$-alkylthio, halogeno-($C_1$-$C_2$)-alkyl, halogeno-($C_1$-$C_2$)-alkoxy and halogeno-($C_1$-$C_2$)-alkylthio, in each case having 1 to 5 identical or different fluorine and/or chlorine atoms, and di-($C_1$-$C_2$)-alkylamino, furthermore represent fluorine, chlorine or bromine,

where at least two of the radicals $R^{3'}$, $R^{4'}$, $R^{5'}$ and $R^{6'}$ represent hydrogen,

$R^{7'}$ represents in each case straight-chain or branched alkyl or alkoxy having 1 to 4 carbon atoms,

$R^{8'}$ represents hydroxyl, straight-chain or branched hydroxyalkyloxy having 1 to 6 carbon atoms, straight-chain or branched halogenoalkyloxy having 1 to 6 carbon atoms and 1 to 13 identical or different halogen atoms, cycloalkyloxy having 3 to 6 carbon atoms which is unsubstituted or monosubstituted to trisubstituted by identical or different substituents from the series consisting of fluorine, chlorine and bromine, in each case straight-chain or branched alkoxy or alkylthio having 1 to 4 carbon atoms, or straight-chain or branched alkoxyalkyloxy, in each case having 1 to 4 carbon atoms in the alkoxy moiety or alkyl moiety, or phenyloxy, phenylthio, phenylalkyl or phenylalkyloxy, where appropriate in each case having 1 to 6 carbon atoms in the alkyl moiety, and in each case unsubstituted or monosubstituted to trisubstituted in the phenyl moiety by identical or different substituents, suitable phenyl substituents being the phenyl substituents mentioned above, or represents a group -O-Z-$NR^{11'}R^{12'}$, -$NHR^{10'}$, -$NR^{11'}R^{12'}$ or -OM,

$R^{9'}$ represents formyl or cyano, or represents the group

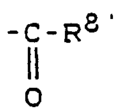

$R^{10'}$ represents hydrogen, straight-chain or branched alkyl having 1 to 4 carbon atoms, or phenyl which is unsubstituted or monosubstituted to trisubstituted by identical or different substituents, suitable phenyl substituents being the phenyl substituents mentioned above,

$R^{11'}$ represents straight-chain or branched alkyl having 1 to 4 carbon atoms, or phenyl which is unsubstituted or monosubstituted to trisubstituted by identical or different substituents, suitable phenyl substituents being the phenyl substituents mentioned above,

$R^{12'}$ represents straight-chain or branched alkyl having 1 to 4 carbon atoms, or phenyl which is unsubstituted or monosubstituted to trisubstituted by identical or different substituents, suitable phenyl substituents being the phenyl substituents mentioned above,

M represents hydrogen, or represents an equivalent of a corresponding alkali metal cation, alkaline earth metal cation or ammonium cation, and

Z represents a straight-chain or branched alkyl chain having 1 to 6 carbon atoms,

or

$R^{2'}$ and $R^{3'}$ together represent one of the radicals

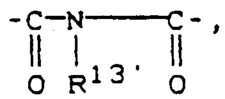, 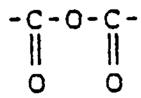 or 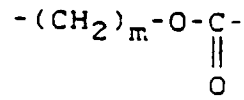,

bridged via the 6- and 5-positions,

$R^{13'}$ represents hydrogen, straight-chain or branched alkyl having 1 to 6 carbon atoms or phenyl which is unsubstituted or monosubstituted to trisubstituted by identical or different substituents, suitable phenyl substituents being the phenyl substituents mentioned above, and

m represents a number 1 or 2,

or

$R^{4'}$ and $R^{5'}$ together represents an alkyl chain which has 3 or 4 carbon atoms and which is linked via the 4- and 3-positions,

and their acid addition salts and metal salt complexes.

**5.** The hydrochloride of the cis-diastereomer of 2-amino-cyclohex-3-ene-carboxylic acid.

**6.** The hydrochlorides of the enantiomers of 2-amino-cyclohex-3-ene-carboxylic acid of melting point 210 - 213.5 °C and 208 - 210 °C.

**7.** The 2-amino-cyclohex-3-ene-carboxylic acid isomer of melting point 98 °C.

**8.** The hydrochloride of the ethyl 2-amino-cyclohex-3-ene-carboxylate-isomer of melting point 139 - 148 °C.

**9.** Process for the preparation of substituted 2-cyclohexen-1-yl-amine derivatives of the formula (Ia)

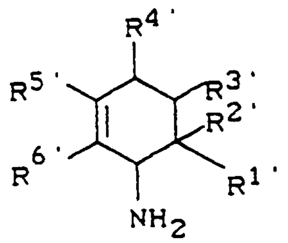

(Ia)

in which

$R^{1'}$ represents hydrogen, alkyl or halogen,

$R^{2'}$ represents formyl, hydroxyalkyl, cyano or nitro, or represents one of the radicals

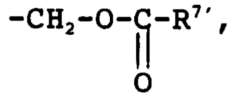, 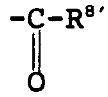

or -CH = CH-$R^{9'}$,

$R^{3'}$, $R^{4'}$, $R^{5'}$ and $R^{6'}$ are identical or different and in each case represent hydrogen, alkyl, alkenyl, alkinyl, alkoxy, alkenyloxy and alkinyloxy, or represent unsubstituted or substituted aryl, or represent unsubstituted or substituted aralkyl, or represent unsubstituted or substituted heteroaryl, or represent unsubstituted or substituted heterocyclylalkyl, or represent alkoxyalkyloxy, or represent halogen,

where at least two of the radicals $R^{3'}$, $R^{4'}$, $R^{5'}$ or $R^{6'}$ represent hydrogen,

$R^{7'}$ represents alkyl or alkoxy,

$R^{8'}$ represents hydroxyl, hydroxyalkyloxy, halogenoalkyloxy, alkoxy or alkoxyalkyloxy, unsubstituted or substituted cycloalkyloxy, unsubstituted or substituted aralkyloxy, unsubstituted or substituted aryloxy, unsubstituted or substituted aralkyl, alkylthio or unsubstituted or substituted arylthio, or represents a group $-O-Z-NR^{11'}R^{12'}$, $-NHR^{10'}$, $-NR^{11'}R^{12'}$ or -OM,

$R^{9'}$ represents formyl or cyano, or represents the group

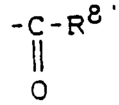

$R^{10'}$ represents hydrogen, alkyl or unsubstituted or substituted aryl,

$R^{11'}$ and $R^{12'}$ are identical or different and in each case represent alkyl or unsubstituted or substituted aryl,

Z represents a straight-chain or branched alkyl chain and

M represents hydrogen, or represents an equivalent of a corresponding alkali metal cation, alkaline earth metal cation or ammonium cation

or

$R^{2'}$ and $R^{3'}$ together represent one of the radicals

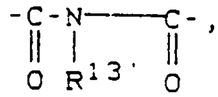 , 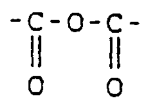 or 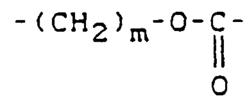 ,

bridged via the 6- and 5-positions,

where

$R^{13'}$ represents hydrogen, alkyl or unsubstituted or substituted aryl and

m represents a number 1 or 2,

or

$R^{4'}$ and $R^{5'}$ together represent an alkyl chain which has 3 or 4 carbon atoms and which is linked via the 4- and 3-positions,

and their acid addition salts and metal salt complexes, with the exception of the compounds 2-amino-cyclohex-3-ene-carboxylic acid and ethyl 2-amino-cyclohex-3-ene-carboxylate, characterized in that

A) 2-cyclohexen-1-yl-carboxylic acid derivatives of the formula (II)

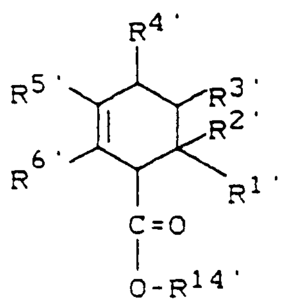

(II)

in which

$R^{1'}$, $R^{2'}$, $R^{3'}$, $R^{4'}$, $R^{5'}$ and $R^{6'}$ have the above-mentioned meaning and

$R^{14'}$ represents hydrogen, methyl or ethyl,

are treated in a generally customary manner with chloroformic acid ester, by the method of Curtius, if appropriate in the presence of a diluent and in the presence of a base at temperatures between -15 °C and +10 °C, and adding an azide to this reaction mixture, if appropriate in the presence of a diluent, at temperatures between -5 °C and +25 °C,

and hydrolyzing the isocyanate, which occurs as an intermediate, of the formula (IIa)

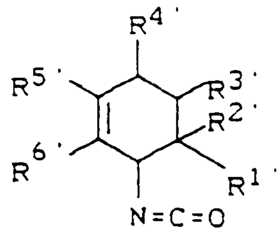

(IIa)

in which

$R^{1'}$, $R^{2'}$, $R^{3'}$, $R^{4'}$, $R^{5'}$ and $R^{6'}$ have the above-mentioned meaning,

with water, if appropriate in the presence of an acid or a base, and, if desired, converting the resulting amines into acid addition salts and metal salt complexes, or

B) the amino-protecting group is eliminated from the 2-cyclohexene derivatives of the formula (IIb)

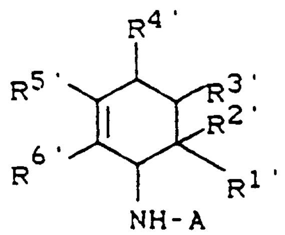

(IIb)

in which

$R^{1'}$, $R^{2'}$, $R^{3'}$, $R^{4'}$, $R^{5'}$ and $R^{6'}$ have the above-mentioned meaning and

A represents an amino-protecting group,

in a known manner by customary methods, if appropriate in the presence of a suitable solvent or diluent or a mixture thereof, with cooling, at room temperature or with heating and, if required, in a sealed vessel, under pressure, in an inert gas atmosphere and/or under anhydrous conditions, and, if desired, converting the resulting products into acid addition salts or metal salt complexes.

**10.** 2-Cyclohexene derivatives of the formula (IIb)

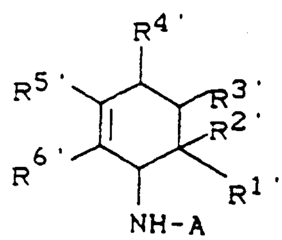

(IIb)

in which

$R^{1'}$, $R^{2'}$, $R^{3'}$, $R^{4'}$, $R^{5'}$ and $R^{6'}$ have the meaning indicated in Claim 2 and

A represents an amino-protecting group,

with the exception of the compounds and the enantiomers and isomers thereof: methyl 6-formyl-5-{[-(phenylmethoxy)-carbonyl]-amino}-3-cyclohexene-1-carboxylate and methyl 6-[3-oxo-1-propenyl)-5-{[-(phenylmethoxy)-carbonyl]-amino}-3-cyclohexene-1-carboxylate, methyl 3-cyclohexene-2-[-(trichloroacetyl)-amino]-1-carbo xylate, 2,2,2-trichloro-N-(6-formyl-2-cyclohexen-1-yl)-acetamide, ethyl (6-formyl-5-methyl-2-cyclohexen-1-yl)-carbamate, methyl 2-[(ethoxy-carbonyl)-amino]-6-methyl-3-cyclohexene-1-carboxylate,methyl 2-[(ethoxycarbonyl)-amino]-5-methyl-3-cyclohexene-1-carboxylate, methyl 2-[(ethoxycarbonyl)-amino]-3-cyclohexene-1-carboxylate, ethyl 3-{2-[(ethoxycarbonyl)-amino]-6-methyl-3-cyclohexen-1-yl}-2-propenoate, phenylmethyl (6-formyl-5-propyl-2-cyclohexen-1-yl)-carbamate, phenylmethyl (6-formyl-5-methyl-2-cyclohexen-1-yl)-carbamate, methyl 2-[(phenoxycarbonyl)-amino]-3-cyclohexene-1-carboxylate and ethyl 3-⟨6-methyl-2-{[(phenylmethoxy)-carbonyl]-amino}-3-cyclohexen-1-yl)-2-propenoate,phenylmethyl (6-formyl-5-pentyl-2-cyclohexen-1-yl)-carbamate,phenyl 6-carbomethoxy-2-cyclohexen-1-yl-thiocarbamate, N-(6-carbomethoxy-2-cyclohexen-1-yl)-1-pyrrolidine-carboxamide, methyl, t-butyl, benzyl and 2,4,6-trimethylphenyl 2-[(p-toluenesulphonyl)-amino]-3-cyclohexen-1-yl-carboxylate and phenylmethyl {6-formyl-5-[2-(methoxymethoxy)-ethyl]-2-cyclohexen-1-yl}-carbamate.

**11.** Pesticides, characterized in that they contain at least one 2-cyclohexen-1-yl-amine derivative of the formula (I) according to Claim 1 and using surface-active agents.

**12.** Method of combating pests, characterized in that 2-cyclohexen-1-yl-amine derivatives of the formula (I) according to Claim 1 are allowed to act on pests and/or their environment.

**13.** Process for the preparation of pesticides, characterized in that 2-cyclohexen-1-yl-amine derivatives of the formula (I) according to Claim 1 are mixed with extenders and/or surface-active agents.

**Claims for the following Contracting State : ES**

**1.** Use of substituted 2-cyclohexen-1-yl-amine derivatives of the formula (I)

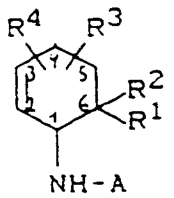

(I)

in which

$R^1$ represents hydrogen, alkyl or halogen,

$R^2$ represents formyl, hydroxyalkyl, cyano or nitro, or represents one of the radicals -$NHR^5$, -$NR^6R^7$,

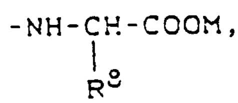

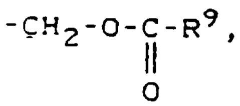 , 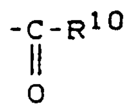 , 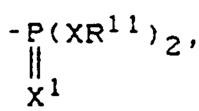 , 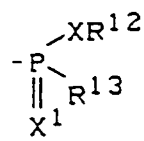 ,

-$S(O)_nR^{14}$ or -CH = CH-$R^{15}$,

$R^3$ and $R^4$ are identical or different and in each case represent hydrogen, alkyl, alkenyl, alkinyl, alkoxy, alkenyloxy or alkinyloxy, or represent unsubstituted or substituted aryl, or represent unsubstituted or substituted aralkyl, or represent unsubstituted or substituted heteroaryl, or represent unsubstituted or substituted heterocyclylalkyl, alkoxyalkyloxy or halogen, or represent one of the radicals -NH-$R^5$, -$NR^6R^7$ or -$S(O)_n$-$R^{14}$,

or

$R^2$ and $R^3$ together represent one of the radicals

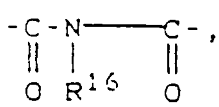 , 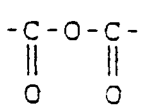 or 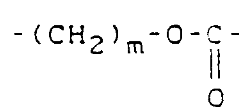 ,

which are bridged via the 6- and 5-positions,

or

$R^3$ and $R^4$ together represent an alkyl chain which has 3 or 4 carbon atoms and which is linked via the 4- and 3-positions,

$R^5$ represents hydrogen, alkyl or unsubstituted or substituted aryl,

$R^6$ represents alkyl or unsubstituted or substituted aryl,

$R^7$ represents alkyl or unsubstituted or substituted aryl,

$R^8$ represents hydrogen, alkyl, unsubstituted or substituted aryl or unsubstituted or substituted aralkyl,

$R^9$ represents alkyl or alkoxy,

$R^{10}$ represents hydroxyl, hydroxyalkyloxy, halogenoalkyloxy, alkoxy, alkoxyalkyloxy, unsubstituted or substituted cycloalkyloxy, unsubstituted or substituted aralkyloxy, unsubstituted or substituted aryloxy, unsubstituted or substituted aralkyl, alkylthio or unsubstituted or substituted arylthio, or represents a group -OM, $-NHR^5$, $-NR^6R^7$ or $-O-Z-NR^5R^6$,

$R^{11}$ represents hydrogen or alkyl,

$R^{12}$ represents hydrogen or alkyl,

$R^{13}$ represents alkyl,

$R^{14}$ represents alkyl, alkoxy or unsubstituted or substituted aryl, or represents the group -OM,

$R^{15}$ represents formyl or cyano, or represents the group

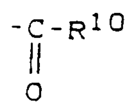

$R^{16}$ represents hydrogen, alkyl or unsubstituted or substituted aryl,

M represents hydrogen or represents an equivalent of a corresponding alkali metal cation, alkaline earth metal cation or ammonium cation,

n represents a number 0, 1 or 2,

X and $X^1$ are identical or different and represent oxygen or sulphur,

m represents a number 1 or 2,

A represents hydrogen or an amino-protecting group and

Z represents a straight-chain or branched alkyl chain,

and their acid addition salts and metal salt complexes for combating pests.

**2.** Process for the preparation of substituted 2-cyclohexen-1-yl-amine derivatives of the formula (Ia)

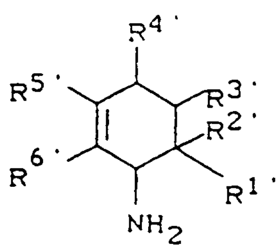

(Ia)

in which

$R^{1'}$ represents hydrogen, alkyl or halogen,

$R^{2'}$ represents formyl, hydroxyalkyl, cyano or nitro, or represents one of the radicals

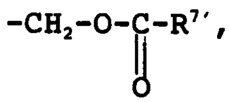, 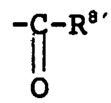

or $-CH=CH-R^{9'}$,

$R^{3'}$, $R^{4'}$, $R^{5'}$ and $R^{6'}$ are identical or different and in each case represent hydrogen, alkyl, alkenyl, alkinyl, alkoxy, alkenyloxy and alkinyloxy, or represent unsubstituted or substituted aryl, or represent unsubstituted or substituted aralkyl, or represent unsubstituted or substituted heteroaryl, or represent unsubstituted or substituted heterocyclylalkyl, or represent alkoxyalkyloxy, or represent halogen,
where at least two of the radicals $R^{3'}$, $R^{4'}$, $R^{5'}$ or $R^{6'}$ represent hydrogen,

$R^{7'}$ represents alkyl or alkoxy,

$R^{8'}$ represents hydroxyl, hydroxyalkyloxy, halogeno-alkyloxy, alkoxy or alkoxyalkyloxy, unsubstituted or substituted cycloalkyloxy, unsubstituted or substituted aralkyloxy, unsubstituted or substituted aryloxy, unsubstituted or substituted aralkyl, alkylthio or unsubstituted or substituted arylthio, or represents a group $-O-Z-NR^{11'}R^{12'}$, $-NHR^{10'}$, $-NR^{11'}R^{12'}$ or $-OM$,

$R^{9'}$ represents formyl or cyano, or represents the group

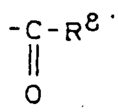

$R^{10'}$ represents hydrogen, alkyl or unsubstituted or substituted aryl,

$R^{11'}$ and $R^{12'}$ are identical or different and in each case represent alkyl or unsubstituted or substituted aryl,

Z represents a straight-chain or branched alkyl chain and

M represents hydrogen, or represents an equivalent of a corresponding alkali metal cation, alkaline earth metal cation or ammonium cation

or

$R^{2'}$ and $R^{3'}$ together represent one of the radicals

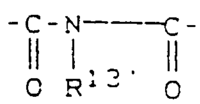, 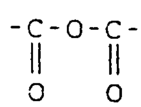 or 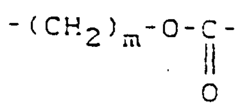,

bridged via the 6- and 5-positions,

where

$R^{13'}$ represents hydrogen, alkyl or unsubstituted or substituted aryl and

m represents a number 1 or 2,

or

$R^{4'}$ and $R^{5'}$ together represent an alkyl chain which has 3 or 4 carbon atoms and which is linked via the 4- and 3-positions,

and their acid addition salts and metal salt complexes, with the exception of the compounds 2-amino-cyclohex-3-ene-carboxylic acid and ethyl 2-amino-cyclohex-3-ene-carboxylate, characterized in that

A) 2-cyclohexen-1-yl-carboxylic acid derivatives of the formula (II)

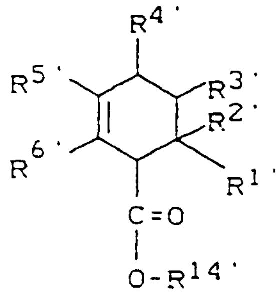

(II)

in which
$R^{1'}$, $R^{2'}$, $R^{3'}$, $R^{4'}$, $R^{5'}$ and $R^{6'}$ have the above-mentioned meaning and
$R^{14'}$ represents hydrogen, methyl or ethyl,
are treated in a generally customary manner with chloroformic acid ester, by the method of Curtius, if appropriate in the presence of a diluent and in the presence of a base at temperatures between -15°C and +10°C, and adding an azide to this reaction mixture, if appropriate in the presence of a diluent, at temperatures between -5°C and +25°C,
and hydrolyzing the isocyanate, which occurs as an intermediate, of the formula (IIa)

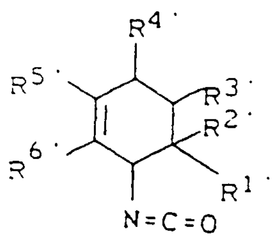

(IIa)

in which
$R^{1'}$, $R^{2'}$, $R^{3'}$, $R^{4'}$, $R^{5'}$ and $R^{6'}$ have the above-mentioned meaning,
with water, if appropriate in the presence of an acid or a base, and, if desired, converting the resulting amines into acid addition salts and metal salt complexes, or
B) the amino-protecting group is eliminated from the 2-cyclohexene derivatives of the formula (IIb)

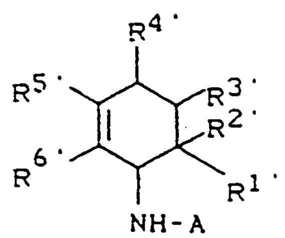

(IIb)

in which
$R^{1'}$, $R^{2'}$, $R^{3'}$, $R^{4'}$, $R^{5'}$ and $R^{6'}$ have the above-mentioned meaning and
A represents an amino-protecting group,
in a known manner by customary methods, if appropriate in the presence of a suitable solvent or diluent or a mixture thereof, with cooling, at room temperature or with heating and, if required, in a sealed vessel, under pressure, in an inert gas atmosphere and/or under anhydrous conditions, and, if desired, converting the resulting products into acid addition salts or metal salt complexes.

**3.** Process according to Claim 2, in which

$R^{1'}$ represents hydrogen, straight-chain or branched alkyl having 1 to 6 carbon atoms, or fluorine, chlorine or bromine,

$R^{2'}$ represents formyl, straight-chain or branched hydroxyalkyl having 1 to 8 carbon atoms in the alkyl moiety, cyano or nitro, or represents one of the radicals

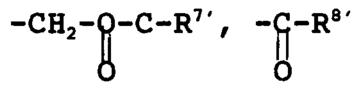

or $-CH=CH-R^{9'}$

$R^{3'}$, $R^{4'}$, $R^{5'}$ and $R^{6'}$ are identical or different and in each case represent hydrogen, in each case straight-chain or branched alkyl or alkoxy having 1 to 8 carbon atoms, in each case straight-chain or branched alkenyl, alkinyl, alkenyloxy or alkinyloxy, in each case having 2 to 8 carbon atoms, or alkoxyalkyloxy, in each case having 1 to 8 carbon atoms in the individual alkyl moieties, or represents aryl or aralkyl, having in each case 6 to 10 carbon atoms in the aryl moiety and where appropriate 1 to 4 carbon atoms in the alkyl moiety, and in each case being unsubstituted or mono-substituted to pentasubstituted in the aryl moiety by identical or different substituents, suitable aryl substituents being: halogen, nitro, cyano, amino, $C_1$-$C_4$-alkyl, $C_1$-$C_4$-alkoxy, $C_1$-$C_4$-alkylthio, halogeno-($C_1$-$C_4$)-alkyl, halogeno-($C_1$-$C_4$)-alkoxy, halogeno-($C_1$-$C_4$)-alkylthio, each having 1 to 9 identical or different halogen atoms, and di-($C_1$-$C_4$)-alkylamino,
furthermore represent a heterocyclic 5- or 6-membered group from the series comprising furyl, thienyl, pyrrolyl, pyrazolyl, imidazolyl, 1,2,3- or 1,2,4-triazolyl, oxazolyl, isoxazolyl, 1,2,4- or 1,3,4-oxadiazolyl, thiazolyl, isothiazolyl, 1,2,3-, 1,2,4-, 1,2,5- or 1,3,4-thiadiazolyl, pyridyl, pyridazinyl, pyrimidinyl and pyrazinyl, which group is unsubstituted or monosubstituted to trisubstituted by identical or different substituents and where appropriate linked via a methylene group, suitable substituents on the heterocycle in each case being: halogen, nitro, cyano, amino, $C_1$-$C_4$-alkyl, $C_1$-$C_4$-alkoxy or $C_1$-$C_4$-alkylthio, halogeno-($C_1$-$C_4$)-alkyl, halogeno-($C_1$-$C_4$)-alkoxy, halogeno-($C_1$-$C_4$)-alkylthio, each having 1 to 9 identical or different halogen atoms, and di-($C_1$-$C_4$)-alkylamino, furthermore represent fluorine, chlorine or bromine,
where at least two of the radicals $R^{3'}$, $R^{4'}$, $R^{5'}$ or $R^{6'}$ represent hydrogen,

$R^{7'}$ represents in each case straight-chain or branched alkyl or alkoxy having 1 to 6 carbon atoms,

$R^{8'}$ represents hydroxyl, straight-chain or branched hydroxyalkyloxy having 1 to 8 carbon atoms, straight-chain or branched halogenoalkyloxy having 1 to 8 carbon atoms and 1 to 17 identical or different halogen atoms, cycloalkyloxy having 3 to 6 carbon atoms which is unsubstituted or monosubstituted to polysubstituted by identical or different halogen substituents, in each case straight-chain or branched alkoxy or alkylthio, aralkyl having 1 to 6 carbon atoms, or straight-chain or branched alkoxyalkyloxy, in each case having 1 to 6 carbon atoms in the alkoxy moiety or alkyl moiety, or aryloxy, arylthio, aralkyl or aralkyloxy, in each case having 6 to 10 carbon atoms in the aryl moiety and where appropriate 1 to 8 carbon atoms in the alkyl moiety, and in each case being unsubstituted or monosubstituted to pentasubstituted in the aryl moiety by identical or different substituents, suitable aryl substituents being the aryl substituents mentioned above, or represents a group $-O-Z-NR^{11'}R^{12'}$, $-NHR^{10'}$, $-NR^{11'}R^{12'}$ or -OM,

$R^{9'}$ represents formyl or cyano, or represents the group

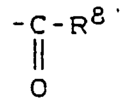

$R^{10'}$ represents hydrogen, straight-chain or branched alkyl having 1 to 6 carbon atoms, or aryl which has 6 to 10 carbon atoms and which is unsubstituted or monosubstituted to pentasubstituted by identical or different substituents, suitable aryl substituents being the aryl substituents mentioned above,

$R^{11'}$ represents straight-chain or branched alkyl having 1 to 6 carbon atoms, or aryl which has 6 to 10 carbon atoms and which is unsubstituted or monosubstituted to pentasubstituted by identical or different substituents, suitable aryl substituents being the aryl substituents mentioned above,

$R^{12'}$ represents straight-chain or branched alkyl having 1 to 6 carbon atoms, or aryl which has 6 to 10 carbon atoms and which is unsubstituted or monosubstituted to pentasubstituted by identical or different substituents, suitable aryl substituents being the aryl substituents mentioned above,

M represents hydrogen, or represents an equivalent of a corresponding alkali metal cation, alkaline earth metal cation or ammonium cation, and

Z represents a straight-chain or branched alkyl chain having 1 to 8 carbon atoms

or

$R^{2'}$ and $R^{3'}$ together represent one of the radicals

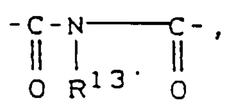, 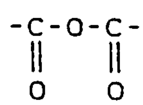 or 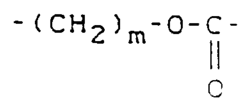,

bridged via the 6- and 5-positions,

where

$R^{13'}$ represents hydrogen, straight-chain or branched alkyl having 1 to 6 carbon atoms or aryl which has 6 to 10 carbon atoms and which is unsubstituted or monosubstituted to pentasubstituted by identical or different substituents, suitable aryl substituents being the aryl substituents mentioned above, and

m represents a number 1 or 2,

or

$R^{4'}$ and $R^{5'}$ together represents an alkyl chain which has 3 or 4 carbon atoms and which is linked via the 4- and 3-positions,

and their acid addition salts and metal salt complexes.

**4.** Process according to Claim 2, in which

$R^{1'}$ represents hydrogen, straight-chain or branched alkyl having 1 to 4 carbon atoms, or fluorine, chlorine or bromine,

$R^{2'}$ represents formyl, straight-chain or branched hydroxyalkyl having 1 to 4 carbon atoms in the alkyl moiety, cyano or nitro, or represents one of the radicals

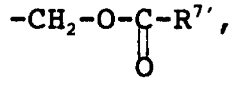, 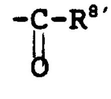

or $-CH=CH-R^{9'}$,

$R^{3'}$, $R^{4'}$, $R^{5'}$ and $R^{6'}$ are identical or different and in each case represent hydrogen, in each case straight-chain or branched alkyl or alkoxy having 1 to 6 carbon atoms, in

each case straight-chain or branched alkenyl, alkinyl, alkenyloxy or alkinyloxy, in each case having 2 to 6 carbon atoms, or alkoxyalkyloxy, in each case having 1 to 6 carbon atoms in the individual alkyl moieties, or represent phenyl or phenylalkyl, where appropriate having 1 or 2 carbon atoms in the alkyl moiety, and in each case being unsubstituted or monosubstituted to trisubstituted in the phenyl moiety by identical or different substituents, suitable phenyl substituents being: fluorine, chlorine, bromine, nitro, cyano, amino, $C_1$-$C_2$-alkyl, $C_1$-$C_3$-alkoxy, $C_1$-$C_2$-alkylthio, halogeno-($C_1$-$C_2$)-alkyl, halogeno-($C_1$-$C_2$)-alkoxy and halogeno-($C_1$-$C_2$)-alkylthio, in each case having 1 to 5 identical or different fluorine and/or chlorine atoms, and di-($C_1$-$C_2$)-alkylamino,

furthermore represent a heterocyclic five- or six-membered group from the series comprising furyl, thienyl, pyrrolyl, pyrazolyl, imidazolyl, 1,2,3- or 1,2,4-triazolyl, oxazolyl, isoxazolyl, 1,2,4- or 1,3,4-oxadiazolyl, thiazolyl, isothiazolyl, 1,2,3-, 1,2,4-, 1,2,5- or 1,3,4-thiadiazolyl, pyridyl, pyridazinyl, pyrimidinyl and pyrazinyl, which group is unsubstituted or monosubstituted to trisubstituted by identical or different substituents and where appropriate bonded via a methylene group, suitable substituents on the heterocycle in each case being: fluorine, chlorine, bromine, nitro, cyano, amino, $C_1$-$C_2$-alkyl, $C_1$-$C_2$-alkoxy or $C_1$-$C_2$-alkylthio, halogeno-($C_1$-$C_2$)-alkyl, halogeno-($C_1$-$C_2$)-alkoxy and halogeno-($C_1$-$C_2$)-alkylthio, in each case having 1 to 5 identical or different fluorine and/or chlorine atoms, and di-($C_1$-$C_2$)-alkylamino,

furthermore represent fluorine, chlorine or bromine,

where at least two of the radicals $R^{3'}$, $R^{4'}$, $R^{5'}$ and $R^{6'}$ represent hydrogen,

$R^{7'}$ represents in each case straight-chain or branched alkyl or alkoxy having 1 to 4 carbon atoms,

$R^{8'}$ represents hydroxyl, straight-chain or branched hydroxyalkyloxy having 1 to 6 carbon atoms, straight-chain or branched halogenoalkyloxy having 1 to 6 carbon atoms and 1 to 13 identical or different halogen atoms, cycloalkyloxy having 3 to 6 carbon atoms which is unsubstituted or monosubstituted to trisubstituted by identical or different substituents from the series consisting of fluorine, chlorine and bromine, in each case straight-chain or branched alkoxy or alkylthio having 1 to 4 carbon atoms, or straight-chain or branched alkoxyalkyloxy, in each case having 1 to 4 carbon atoms in the alkoxy moiety or alkyl moiety, or phenyloxy, phenylthio, phenylalkyl or phenylalkyloxy, where appropriate in each case having 1 to 6 carbon atoms in the alkyl moiety, and in each case unsubstituted or monosubstituted to trisubstituted in the phenyl moiety by identical or different substituents, suitable phenyl substituents being the phenyl substituents mentioned above, or represents a group -O-Z-$NR^{11'}R^{12'}$, -$NHR^{10'}$, -$NR^{11'}R^{12'}$ or -OM,

$R^{9'}$ represents formyl or cyano, or represents the group

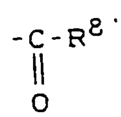

$R^{10'}$ represents hydrogen, straight-chain or branched alkyl having 1 to 4 carbon atoms, or phenyl which is unsubstituted or monosubstituted to trisubstituted by identical or different substituents, suitable phenyl substituents being the phenyl substituents mentioned above,

$R^{11'}$ represents straight-chain or branched alkyl having 1 to 4 carbon atoms, or phenyl which is unsubstituted or monosubstituted to trisubstituted by identical or different substituents, suitable phenyl substituents being the phenyl substituents mentioned above,

$R^{12'}$ represents straight-chain or branched alkyl having 1 to 4 carbon atoms, or phenyl which is unsubstituted or monosubstituted to trisubstituted by identical or different substituents, suitable phenyl substituents being the phenyl substituents mentioned above,

M represents hydrogen, or represents an equivalent of a corresponding alkali metal cation, alkaline earth metal cation or ammonium cation and

Z represents a straight-chain or branched alkyl chain having 1 to 6 carbon atoms

or

$R^{2'}$ and $R^{3'}$ together represent one of the radicals

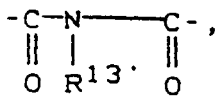, 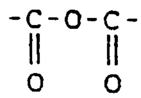 or 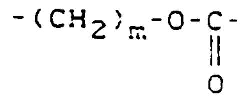 ,

bridged via the 6- and 5-positions,

$R^{13'}$ represents hydrogen, straight-chain or branched alkyl having 1 to 6 carbon atoms or phenyl which is unsubstituted or monosubstituted to trisubstituted by identical or different substituents, suitable phenyl substituents being the phenyl substituents mentioned above, and

m represents a number 1 or 2,

or

$R^{4'}$ and $R^{5'}$ together represents an alkyl chain which has 3 or 4 carbon atoms and which is linked via the 4- and 3-positions,

and their acid addition salts and metal salt complexes.

**5.** Process according to Claim 2, in which the compound of the formula (Ia) is the hydrochloride of the cis-diastereomer of 2-amino-cyclohex-3-ene-carboxylic acid.

**6.** Process according to Claim 2, in which the compounds of the formula (Ia) are the hydrochlorides of the enantiomers of 2-amino-cyclohex-3-ene-carboxylic acid of melting point 210 - 213.5 °C and 208 - 210 °C.

**7.** Process according to Claim 2, in which the compound of the formula (Ia) is the 2-amino-cyclohex-3-ene-carboxylic acid isomer of melting point 98 °C.

**8.** Process according to Claim 2, in which the compound of the formula (Ia) is the hydrochloride of the ethyl 2-amino-cyclohex-3-ene-carboxylate-isomer of melting point 139 - 148 °C.

**9.** Pesticides, characterized in that they contain at least one 2-cyclohexen-1-yl-amine derivative of the formula (I) according to Claim 1 and using surfaceactive agents.

**10.** Method of combating pests, characterized in that 2-cyclohexen-1-yl-amine derivatives of the formula (I) according to Claim 1 are allowed to act on pests and/or their environment.

**11.** Process for the preparation of pesticides, characterized in that 2-cyclohexen-1-yl-amine derivatives of the formula (I) according to Claim 1 are mixed with extenders and/or surface-active agents.

**12.** Process for the preparation of 2-cyclohexene derivatives of the formula (IIb)

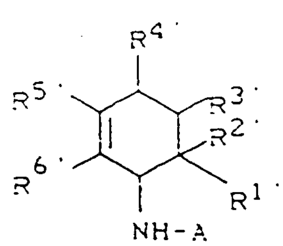

(IIb)

in which

$R^{1'}$, $R^{2'}$, $R^{3'}$, $R^{4'}$, $R^{5'}$ and $R^{6'}$ have the meaning indicated in Claim 2 and

A represents an amino-protecting group,

with the exception of the compounds and the enantiomers and isomers thereof: methyl 6-formyl-5-{[-(phenylmethoxy)-carbonyl]-amino}-3-cyclohexene-1-carboxylate and methyl 6-[3-oxo-1-propenyl)-5-{[-(phenylmethoxy)-carbonyl]-amino}-3-cyclohexene-1-carboxylate, methyl 3-cyclohexene-2-[-(trichloroacetyl)-amino]-1-carboxylate, 2,2,2-trichloro-N-(6-formyl-2-cyclohexen-1-yl)-acetamide, ethyl (6-formyl-5-methyl-2-cyclohexen-1-yl)-carbamate, methyl 2-[(ethoxy-carbonyl)-amino]-6-methyl-3-cyclohexene-1-carboxylate, methyl 2-[(ethoxycarbonyl)-amino]-5-methyl-3-cyclohexene-1-carboxylate, methyl 2-[(ethoxycarbonyl)-amino]-3-cyclohexene-1-carboxylate, ethyl 3-{2-[(ethoxycarbonyl)-amino]-6-methyl-3-cyclohexen-1-yl}-2-propenoate, phenyl methyl (6-formyl-5-propyl-2-cyclohexen-1-yl)-carbamate, phenyl methyl (6-formyl-5-methyl-2-cyclohexen-1-yl)-carbamate, methyl 2-[(phenoxycarbonyl)-amino]-3-cyclohexene-1-carboxylate and ethyl 3-⟨6-methyl-2-{[(phenylmethoxy)-carbonyl]-amino}-3-cyclo hexen-1-yl⟩-2-propenoate, phenyl methyl (6-formyl-5-pentyl-2-cyclohexen-1-yl)-carbamate, phenyl 6-carbomethoxy-2-cyclohexen-1-yl-thiocarbamate, N-(6-carbomethoxy-2-cyclohexen-1-yl)-1-pyrrolidinecarboxamide, methyl, t-butyl, benzyl and 2,4,6-trimethylphenyl 2-[(p-toluenesulphonyl)-amino]-3-cyclohexen-1-yl-carboxylate and phenyl methyl {6-formyl-5-[2-(methoxymethoxy)-ethyl]-2-cyclohexen-1-yl}-carbamate, characterized in that dienophiles of the formula (III) in which

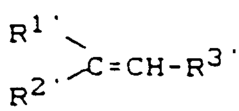

(III)

in which

$R^{1'}$, $R^{2'}$ and $R^{3'}$ have the abovementioned meaning,

(B/a) are cyclized with N-acyl-1-amino-1,3-butadiene derivatives of the formula (IVa)

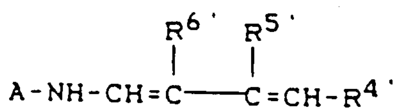

(IVa)

in which

$R^{4'}$, $R^{5'}$, $R^{6'}$ and A have the abovementioned meaning,

if appropriate in the presence of a diluent, if appropriate in the presence of a catalyst, if appropriate in the presence of an inert gas and if appropriate under pressure, or

(B/b) are initially cyclized, in a first step, with substituted butadienes of the formula (IVb)

(IVb)

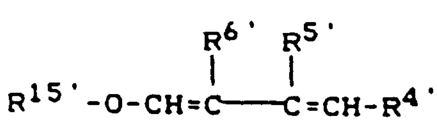

in which

$R^{4'}$, $R^{5'}$ and $R^{6'}$ have the abovementioned meaning and

$R^{15'}$ represents the acetyl or trimethylsilyl radical,

if appropriate in the presence of a diluent, if appropriate in the presence of a catalyst, if appropriate in the presence of an inert gas and if appropriate under pressure,

and the resulting 2-cyclohexene derivatives of the formula (IIc)

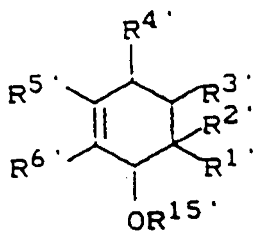

(IIc)

in which

$R^{1'}$, $R^{2}$, $R^{3'}$, $R^{4'}$, $R^{5'}$ and $R^{6'}$ have the above-mentioned meaning and

$R^{15'}$ represents the acetyl or trimethylsilyl radical,

are reacted, in a second step, with 4,4'-dimethoxybenzhydrylamine (DMB) of the formula (V)

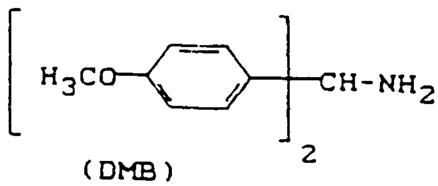

(DMB)

(V)

if appropriate in the presence of a diluent at temperatures from 0 °C to the boiling point of the solvent used in each case and in the presence of a catalyst,

or

(B/c)the isocyanates of the formula (IIa)

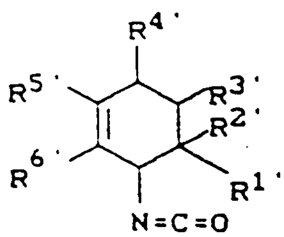

(IIa)

in which

$R^{1'}$, $R^{2'}$, $R^{3'}$, $R^{4'}$, $R^{5'}$ and $R^{6'}$ have the above-mentioned meaning and which occur as intermediates in process (A),

are reacted with alcohols of the formula (VII)

$R^{16'}$-OH (VII)

in which

$R^{16'}$ represents in each case straight-chain or branched alkyl, alkenyl, alkinyl or alkoxyalkyl, each of which has 1 to 12 carbon atoms (preferably 1 to 6 carbon atoms) and each of which is optionally substituted by halogen, or represents in each case unsubstituted or substituted phenyl or benzyl,

or

(B/d)the substituted 2-cyclohexene derivatives of the formula (IIb) are obtained when the 2-cyclohexene derivatives of the formula (IId)

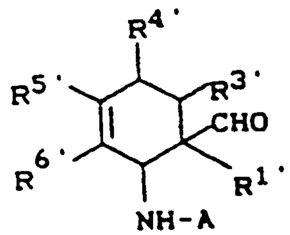

(IId)

in which

$R^{1'}$, $R^{3'}$, $R^{4'}$, $R^{5'}$, $R^{6'}$ and A have the abovementioned meaning and which can be obtained by process (B/a), (B/b) or (B/c),

are reduced using a complex metal hydride, such as, for example, sodium borohydride, in a suitable solvent at temperatures from 0 °C to 20 °C, and the 2-cyclohexen-1-yl-amine alcohols of the formula (IIe)

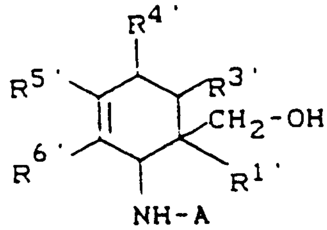

(IIe)

in which

$R^{1'}$, $R^{3'}$, $R^{4'}$, $R^{5'}$, $R^{6'}$ and A have the abovementioned meaning and which can thus be obtained,

are converted by further reactions on the hydroxyl group into esters and ethers or by carbamoyl chlorides into carbamoyl derivatives of the compounds of the formula (IIb),

or

(B/e)the substituted 2-cyclehexene derivatives of the formula (IIb) are obtained when the 2-cyclohexene derivatives of the formula (IId)

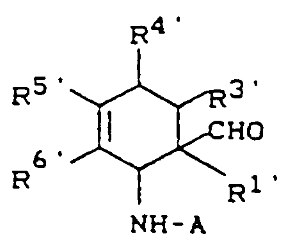

(IId)

in which

$R^{1'}$, $R^{3'}$, $R^{4'}$, $R^{5'}$, $R^{6'}$ and A have the abovementioned meaning and which can be obtained by process (B/a), (B/b) or (B/c),

are reacted with alkanephosphonic acid derivatives of the formula (VIII)

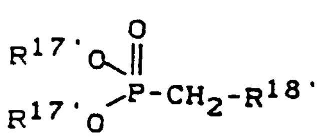

(VIII)

in which

$R^{17'}$ represents methyl or ethyl and

$R^{18'}$ represents the cyano or the alkoxycarbonyl group,

if appropriate in the presence of a diluent, if appropriate in the presence of a base and if appropriate in the presence of an inert gas.

## Revendications

### Revendications pour les Etats contractants suivants : BE, CH, DE, FR, GB, IT, LI, NL

**1.** Utilisation de dérivés de 2-cyclohexène-1-yl-amine de formule (I)

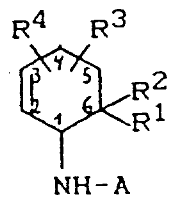

(I)

dans laquelle

$R^1$ représente l'hydrogène, un groupe alkyle ou un halogène,

$R^2$ est un groupe formyle, hydroxyalkyle, cyano, nitro ou représente l'un des restes $-NHR^5$, $-NR^6R^7$,

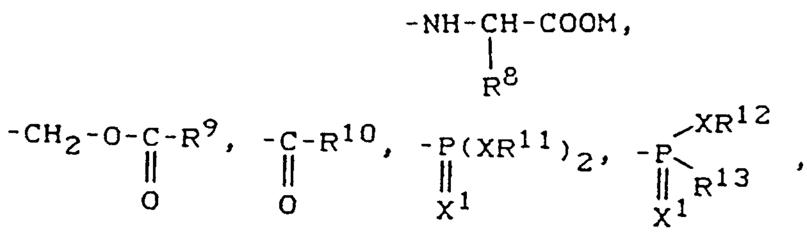

$-S(O)_nR^{14}$ ou $-CH=CH-R^{15}$

$R^3$ et $R^4$ sont identiques ou différents et représentent chacun l'hydrogène, un groupe alkyle, alcényle, alcynyle, alkoxy, alcényloxy, alcynyloxy, un groupe aryle non substitué ou substitué, un groupe aralkyle non substitué ou substitué, un groupe hétéroaryle non substitué ou substitué, un groupe hétérocyclylalkyle non substitué ou substitué, un groupe alkoxyalkyloxy, un halogène ou l'un des restes $-NH-R^5$, $-NR^6R^7$ ou $-S(O)_n-R^{14}$,

ou bien

$R^2$ et $R^3$ représentent ensemble l'un des restes

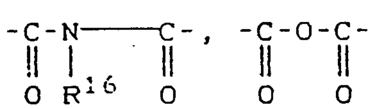

ou

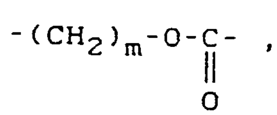

ponté par les positions 6 et 5,

ou bien

$R^3$ et $R^4$ forment conjointement une chaîne alkylique de 3 ou 4 atomes de carbone, qui est liée par les positions 4 et 3,

$R^5$ est l'hydrogène, un groupe alkyle ou un groupe aryle non substitué ou substitué,

$R^6$ est un groupe alkyle ou un groupe aryle non substitué ou substitué,

$R^7$ est un groupe alkyle ou un groupe aryle non substitué ou substitué,

$R^8$ est l'hydrogène, un groupe alkyle, un groupe aryle non substitué ou substitué, ou un groupe aralkyle non substitué ou substitué,

$R^9$ est un groupe alkyle ou alkoxy,

$R^{10}$ est un groupe hydroxy, hydroxyalkyloxy, halogénalkyloxy, alkoxy, alkoxyalkyloxy, un groupe cycloalkyloxy non substitué ou substitué ou un groupe aralkyloxy non substitué ou substitué, un groupe aryloxy non substitué ou substitué, un groupe aralkyle non substitué ou substitué, un groupe alkylthio, un groupe arylthio non substitué ou substitué ou un groupe -OM, $-NHR^5$, $-NR^6R^7$ ou $-O-Z-NR^5R^6$,

$R^{11}$ est de l'hydrogène ou un groupe alkyle,

$R^{12}$ est de l'hydrogène ou un groupe alkyle,

$R^{13}$ est un groupe alkyle,

$R^{14}$ est un groupe alkyle, un groupe alkoxy, un groupe aryle non substitué ou substitué ou le groupe -OM,

$R^{15}$ est un groupe formyle, cyano ou le groupement

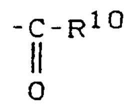

$R^{16}$ est de l'hydrogène, un groupe alkyle ou un groupe aryle non substitué ou substitué,

M est de l'hydrogène ou représente un équivalent d'un cation correspondant de métal alcalin, de métal alcalino-terreux ou d'ammonium,

n représente le nombre 0, 1 ou 2,

X et $X^1$ sont identiques ou différents et représentent l'oxygène ou le soufre,

m est le nombre 1 ou 2,

A est de l'hydrogène ou un groupe protégeant la fonction amino et

Z est une chaîne alkylique linéaire ou ramifiée,

ainsi que leurs sels d'addition d'acides et leurs complexes de sels métalliques, pour combattre des parasites.

**2.** Dérivés substitués de 2-cyclohexène-1-yl-amine de formule (Ia)

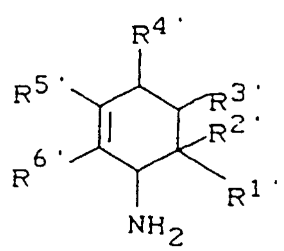

(Ia)

dans laquelle

$R^{1'}$ représente de l'hydrogène, un groupe alkyle ou un halogène,

$R^{2'}$ est un groupe formyle, hydroxyalkyle, cyano, nitro ou l'un des restes

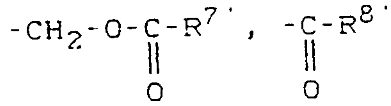

ou $-CH=CH-R^{9'}$

$R^{3'}$, $R^{4'}$, $R^{5'}$ et $R^{6'}$ sont identiques ou différents et représentent chacun de l'hydrogène, un groupe alkyle, alcényle, alcynyle, alkoxy, alcényloxy, alcynyloxy, un groupe aryle non substitué ou substitué, un groupe aralkyle non substitué ou substitué, un groupe hétéroaryle non substitué ou substitué, un groupe hétérocyclylalkyle non substitué ou substitué, un groupe alkoxyalkyloxy ou un halogène,
deux au moins des restes $R^{3'}$, $R^{4'}$, $R^{5'}$ ou $R^{6'}$ représentent de l'hydrogène,

$R^{7'}$ est un groupe alkyle ou alkoxy,

$R^{8'}$ est un groupe hydroxy, hydroxyalkyloxy, halogénalkyloxy, alkoxy, alkoxyalkyloxy, un groupe cycloalkyloxy non substitué ou substitué, un groupe aralkyloxy non substitué ou substitué, un groupe aryloxy non substitué ou substitué, un groupe aralkyle non substitué ou substitué, un groupe alkylthio, un groupe arylthio non substitué ou substitué ou un groupe $-O-Z-NR^{11'}R^{12'}$,$-NHR^{10'}$, $-NR^{11'}R^{12'}$ ou -OM,

$R^{9'}$ est un groupe formyle, cyano ou le groupe

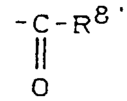

$R^{10'}$ représente de l'hydrogène, un groupe alkyle ou un groupe aryle non substitué ou substitué,

$R^{11'}$ et $R^{12'}$ sont identiques ou différents et représentent chacun un groupe alkyle ou un groupe aryle non substitué ou substitué,

Z est une chaîne alkylique linéaire ou ramifiée et

M représente de l'hydrogène ou un équivalent d'un cation correspondant de métal alcalin, de métal alcalino-terreux ou d'ammonium,

ou bien

$R^{2'}$ et $R^{3'}$ forment conjointement l'un des restes

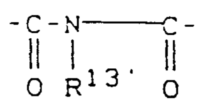 , 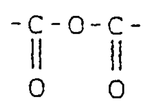 ou 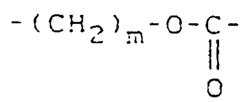 ,

ponté par les positions 6 et 5,

où

$R^{13'}$ représente de l'hydrogène, un groupe alkyle ou un groupe aryle non substitué ou substitué et

m est le nombre 1 ou 2,

ou bien

$R^{4'}$ et $R^{5'}$ forment conjointement une chaîne alkylique de 3 ou 4 atomes de carbone, qui est liée par les positions 4 et 3

ainsi que leurs sels d'addition d'acides et leurs complexes de sels métalliques, excepté les composés acide 2-amino-cyclohex-3-ène-carboxyliques et ester éthylique d'acide 2-aminocyclohex-3-ène-carboxylique.

**3.** Dérivés substitués de 2-cyclohexène-1-yl-amine de formule (Ia) suivant la revendication 2, dans lesquels

$R^{1'}$ représente de l'hydrogène, un groupe alkyle à chaîne droite ou ramifiée ayant 1 à 6 atomes de carbone ou du fluor, du chlore ou du brome,

$R^{2'}$ est un groupe formyle, un groupe hydroxyalkyle à chaîne droite ou ramifiée ayant 1 à 8 atomes de carbone dans la partie alkyle, un groupe cyano, nitro ou l'un des restes

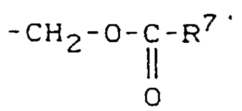 , 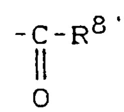

ou $-CH=CH-R^{9'}$

$R^{3'}$, $R^{4'}$, $R^{5'}$ et $R^{6'}$ sont identiques ou différents et représentent chacun de l'hydrogène, chacun un groupe alkyle ou alkoxy à chaîne droite ou ramifiée ayant 1 à 8 atomes de carbone, chacun un groupe alcényle, alcynyle, alcényloxy ou alcynyloxy à chaîne droite ou ramifiée ayant chacun 2 à 8 atomes de carbone, alkoxyalkyloxy ayant chacun 1 à 8 atomes de carbone dans les parties alkyle individuelles, chacun un groupe aryle ou aralkyle non substitué dans la partie aryle ou portant 1 à 5 substituants identiques ou différents et ayant chacun 6 à 10 atomes de carbone dans la partie aryle et le cas échéant 1 à 4 atomes de carbone dans la partie alkyle, en considérant comme substituants de la partie aryle : un halogène, un radical nitro, cyano, amino, alkyle en $C_1$ à $C_4$, alkoxy en $C_1$ à $C_4$, alkylthio en $C_1$ à $C_4$, halogénalkyle en $C_1$ à $C_4$, halogénalkoxy en $C_1$ à $C_4$, halogénalkylthio en $C_1$ à $C_4$ avec chacun 1 à 9 atomes d'halogènes identiques ou différents et di(alkyle en $C_1$ à $C_4$)amino,

en outre, un groupement hétérocyclique pentagonal ou hexagonal non substitué ou portant un à trois substituants identiques ou différents et lié le cas échéant par un groupe méthylène, de la série furyle, thiényle, pyrrolyle, pyrazolyle, imidazolyle, 1,2,3- ou 1,2,4-triazolyle, oxazolyle, isoxazolyle, 1,2,4- ou 1,3,4-oxadiazolyle, thiazolyle, isothiazolyle, 1,2,3-, 1,2,4-, 1,2,5- ou 1,3,4-thiadiazolyle, pyridyle, pyridazinyle, pyrimidinyle et pyrazinyle, en considérant dans chaque cas comme substituants de l'hétérocycle : un halogène, un radical nitro, cyano, amino, alkyle en $C_1$ à $C_4$, alkoxy en $C_1$ à $C_4$ ou alkylthio en $C_1$ à $C_4$, halogénalkyle en $C_1$ à $C_4$, halogénalkoxy en $C_1$ à $C_4$, halogénalkylthio en $C_1$ à $C_4$ ayant dans chaque cas 1 à 9 atomes d'halogènes identiques ou différents, et di(alkyle en $C_1$ à $C_4$)amino, en outre le fluor, le chlore ou le brome,

deux au moins des restes $R^{3'}$, $R^{4'}$, $R^{5'}$ ou $R^{6'}$ représentant de l'hydrogène,

$R^{7'}$ est un groupe alkyle ou alkoxy ayant 1 à 6 atomes de carbone, chacun à chaîne droite ou ramifiée,

$R^{8'}$ est un groupe hydroxy, un groupe hydroxyalkyloxy linéaire ou ramifié ayant 1 à 8 atomes de carbone, un groupe halogénalkyloxy linéaire ou ramifié ayant 1 à 8 atomes de carbone et 1 à 17 atomes d'halogènes identiques ou différents, un groupe cycloalkyloxy non substitué ou portant un à plusieurs substituants halogéno identiques ou différents, et ayant 3 à 6 atomes de carbone, un groupe alkoxy ou alkylthio ayant 1 à 6 atomes de carbone, chacun à chaîne droite ou ramifiée, un groupe alkoxyalkyloxy à chaîne droite ou ramifiée ayant chacun 1 à 6 atomes de carbone dans la partie alkoxy ou alkyle, un groupe aryloxy, arylthio, aralkyle ou aralkyloxy non substitué dans la partie aryle ou portant un à cinq substituants identiques ou différents et ayant chacun 6 à 10 atomes de carbone dans la partie aryle et le cas échéant 1 à 8 atomes de carbone dans la partie alkyle, en considérant comme substituants de la partie aryle les substituants de cette partie indiqués ci-dessus, ou un groupe -O-Z-$NR^{11'}R^{12'}$, -$NHR^{10'}$, -$NR^{11'}R^{12'}$ ou -OM,

$R^{9'}$ est un groupe formyle, cyano ou le groupe

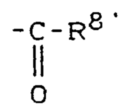

$R^{10'}$ représente l'hydrogène, un groupe alkyle à chaîne droite ou ramifiée ayant 1 à 6 atomes de carbone ou un groupe aryle de 6 à 10 atomes de carbone non substitué ou portant un à cinq substituants identiques ou différents,

en considérant comme substituants du groupe aryle les substituants de ce groupe indiqués ci-dessus,

$R^{11'}$ est un groupe alkyle à chaîne droite ou ramifiée ayant 1 à 6 atomes de carbone ou un groupe aryle de 6 à 10 atomes de carbone non substitué ou portant un à cinq substituants identiques ou différents, en considérant comme substituants du groupe aryle les substituants de ce groupe indiqués ci-dessus,

$R^{12'}$ est un groupe alkyle à chaîne droite ou ramifiée ayant 1 à 6 atomes de carbone ou un groupe aryle de 6 à 10 atomes de carbone non substitué ou portant un à cinq substituants identiques ou différents, en considérant comme substituants du groupe aryle les substituants de ce groupe indiqués ci-dessus,

M représente de l'hydrogène ou un équivalent d'un cation correspondant de métal alcalin, de métal alcalino-terreux ou d'ammonium et

Z est une chaîne alkylique linéaire ou ramifiée ayant 1 à 8 atomes de carbone,

ou bien

$R^{2'}$ et $R^{3'}$ forment conjointement l'un des restes

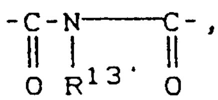, 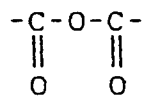 ou 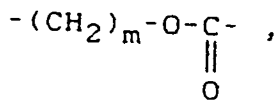,

ponté par les positions 6 et 5,

où

$R^{13'}$ représente de l'hydrogène, un groupe alkyle à chaîne droite ou ramifiée ayant 1 à 6 atomes de carbone ou un groupe aryle de 6 à 10 atomes de carbone non substitué ou portant un à cinq substituants identiques ou différents, en considérant comme substituants du groupe aryle les substituants de ce groupe indiqués ci-dessus et

m est le nombre 1 ou 2,

ou bien

$R^{4'}$ et $R^{5'}$ forment ensemble une chaîne alkylique de 3 ou 4 atomes de carbone, qui est liée par les positions 4 et 3,

ainsi que leurs sels d'addition d'acides et leurs complexes de sels métalliques.

**4.** Dérivés substitués de 2-cyclohexène-1-yl-amine de formule (Ia) suivant la revendication 2, dans lesquels

$R^{1'}$ représente de l'hydrogène, un groupe alkyle à chaîne droite ou ramifiée ayant 1 à 4 atomes de carbone ,ou du fluor, du chlore ou du brome,

$R^{2'}$ est un groupe formyle, un groupe hydroxyalkyle à chaîne droite ou ramifiée ayant 1 à 4 atomes de carbone dans la partie alkyle, un groupe cyano, nitro ou l'un des restes

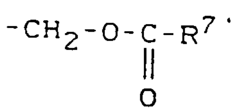, 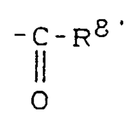

ou $-CH=CH-R^{9'}$

$R^{3'}$, $R^{4'}$, $R^{5'}$ et $R^{6'}$ sont identiques ou différents et représentent chacun de l'hydrogène, chacun un groupe alkyle ou alkoxy de 1 à 6 atomes de carbone à chaîne droite ou ramifiée, chacun un groupe alcényle, alcynyle, alcényloxy ou alcynyloxy linéaire ou ramifié ayant chacun 2 à 6 atomes de carbone, un groupe alkoxyalkyloxy ayant 1 à 6 atomes de carbone dans chacune des parties alkyle individuelles, un groupe phényle ou phénylalkyle chacun non substitué dans la partie phényle ou portant un à trois substituants identiques ou différents, avec le cas échéant 1 ou 2 atomes de carbone dans la partie alkyle, en considérant comme substituants du groupe phényle : le fluor, le chlore, le brome, un radical nitro, cyano, amino, alkyle en $C_1$ ou $C_2$, alkoxy en $C_1$ à $C_3$, alkylthio en $C_1$ ou $C_2$, halogénalkyle en $C_1$ ou $C_2$, halogénalkoxy en $C_1$ ou $C_2$, halogénalkylthio en $C_1$ ou $C_2$ avec chacun 1 à 5 atomes de fluor et/ou de chlore identiques ou différents et di(alkyle en $C_1$ ou $C_2$)amino, en outre un groupement hétérocyclique pentagonal ou hexagonal non substitué ou portant un à trois substituants identiques ou différents et le cas échéant lié par un groupe

méthylène, de la série furyle, thiényle, pyrrolyle, pyrazolyle, imidazolyle, 1,2,3- ou 1,2,4-triazolyle, oxazolyle, isoxazolyle, 1,2,4- ou 1,3,4-oxadiazolyle, thiazolyle, isothiazolyle, 1,2,3-, 1,2,4-, 1,2,5- ou 1,3,4-thiadiazolyle, pyridyle, pyridazinyle, pyrimidinyle et pyrazinyle, en considérant dans chaque cas comme substituants de l'hétérocycle : du fluor, du chlore, du brome, un radical nitro, cyano, amino, alkyle en $C_1$ ou $C_2$, alkoxy en $C_1$ ou $C_2$ ou alkylthio en $C_1$ ou $C_2$, halogénalkyle en $C_1$ ou $C_2$, halogénalkoxy en $C_1$ ou $C_2$, halogénalkylthio en $C_1$ ou $C_2$ avec dans chaque cas 1 à 5 atomes de fluor et/ou de chlore identiques ou différents, et di(alkyle en $C_1$ ou $C_2$)amino,

en outre le fluor, le chlore ou le brome,

deux au moins des restes $R^{3'}$, $R^{4'}$, $R^{5'}$ et $R^{6'}$ représentant de l'hydrogène,

$R^{7'}$ est un groupe alkyle ou un groupe alkoxy, chacun linéaire ou ramifié avec 1 à 4 atomes de carbone,

$R^{8'}$ est un groupe hydroxy, un groupe hydroxyalkyloxy à chaîne droite ou ramifiée ayant 1 à 6 atomes de carbone, un groupe halogénalkyloxy à chaîne droite ou ramifiée ayant 1 à 6 atomes de carbone et 1 à 13 atomes d'halogènes identiques ou différents, un groupe cycloalkyloxy de 3 à 6 atomes de carbone non substitué ou portant un à trois substituants fluoro, chloro, bromo identiques ou différents, un groupe alkoxy ou alkylthio, chacun à chaîne droite ou ramifiée ayant 1 à 4 atomes de carbone, un groupe alkoxyalkyloxy à chaîne droite ou ramifiée ayant 1 à 4 atomes de carbone dans chaque partie alkoxy ou alkyle, un groupe phényloxy, phénylthio, phénylalkyle ou phénylalkyloxy non substitué chacun dans la partie phényle ou portant chacun un à trois substituants identiques ou différents, avec chacun le cas échéant 1 à 6 atomes de carbone dans la partie alkyle, en considérant comme substituants du groupe phényle les substituants de ce groupe indiqués ci-dessus,

ou bien un groupe $-O-Z-NR^{11'}R^{12'}$, $-NHR^{10'}$, $-NR^{11'}R^{12'}$ ou -OM,

$R^{9'}$ est un groupe formyle, cyano ou le groupe

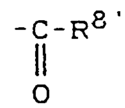

$R^{10'}$ représente de l'hydrogène, un groupe alkyle à chaîne droite ou ramifiée ayant 1 à 4 atomes de carbone ou un groupe phényle non substitué ou portant un à trois substituants identiques ou différents, en considérant comme substituants du groupe phényle les substituants de ce groupe indiqués ci-dessus,

$R^{11'}$ est un groupe alkyle à chaîne droite ou ramifiée ayant 1 à 4 atomes de carbone ou un groupe phényle non substitué ou portant un à trois substituants identiques ou différents, en considérant comme substituants du groupe phényle les substituants de ce groupe indiqués ci-dessus,

$R^{12'}$ est un groupe alkyle à chaîne droite ou ramifiée ayant 1 à 4 atomes de carbone ou un groupe phényle non substitué ou portant un à trois substituants identiques ou différents, en considérant comme substituants du groupe phényle les substituants de ce groupe indiqués ci-dessus,

M représente de l'hydrogène ou un équivalent d'un cation correspondant de métal alcalin, de métal alcalino-terreux ou d'ammonium et

Z est une chaîne alkylique linéaire ou ramifiée ayant 1 à 6 atomes de carbone,

ou bien

$R^{2'}$ et $R^{3'}$ forment ensemble l'un des restes

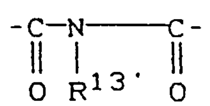 , 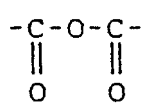 ou 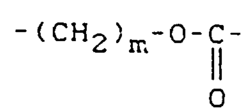 ,

ponté par les positions 6 et 5,

$R^{13'}$ représente de l'hydrogène, un groupe alkyle à chaîne droite ou ramifiée ayant 1 à 6 atomes de carbone et un groupe phényle non substitué ou portant un à trois substituants identiques ou différents, en considérant comme substituants du groupe phényle les substituants de ce groupe indiqués ci-dessus et

m représente le nombre 1 ou 2,

ou bien

$R^{4'}$ et $R^{5'}$ forment conjointement une chaîne alkylique de 3 ou 4 atomes de carbone, qui est liée par les positions 4 et 3,

ainsi que leurs sels d'addition d'acides et leurs complexes de sels métalliques.

**5.** Le chlorhydrate du cis-diastéréoisomère de l'acide 2-amino-cyclohex-3-ène-carboxylique.

**6.** Les chlorhydrates des énantiomères de l'acide 2-amino-cyclohex-3-ène-carboxylique ayant un point de fusion de 210 - 213,5 °C et de 208 - 210 °C.

**7.** L'acide 2-amino-cyclohex-3-ène-carboxylique isomère de point de fusion égal à 98 °C.

**8.** Le chlorhydrate de l'isomère de l'ester éthylique d'acide 2-amino-cyclohex-3-ène-carboxylique ayant un point de fusion de 139 - 148 °C.

**9.** Procédé de production de dérivés substitues de 2-cyclo-hexène-1-yl-amine de formule (Ia)

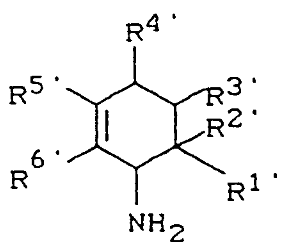

(Ia)

dans laquelle

$R^{1'}$ représente de l'hydrogène, un groupe alkyle ou un halogène,

$R^{2'}$ est un groupe formyle, hydroxyalkyle, cyano, nitro ou l'un des restes

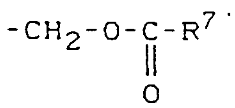 , 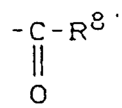

ou $-CH=CH-R^{9'}$

$R^{3'}$, $R^{4'}$, $R^{5'}$ et $R^{6'}$ sont identiques ou différents et représentent chacun de l'hydrogène, un groupe alkyle, alcényle, alcynyle, alkoxy, alcényloxy, alcynyloxy, un groupe aryle non substitué ou substitué, un groupe aralkyle non substitué ou substitué, un groupe hétéroaryle non substitué ou substitué, un groupe hétérocyclylalkyle non substitué ou substitué, un groupe alkoxyalkyloxy ou un halogène, deux au moins des restes $R^{3'}$, $R^{4'}$, $R^{5'}$ ou $R^{6'}$ représentent de l'hydrogène,

$R^{7'}$ est un groupe alkyle ou alkoxy,

$R^{8'}$ est un groupe hydroxy, hydroxyalkyloxy, halogénalkyloxy, alkoxy, alkoxyalkyloxy, un groupe cycloalkyloxy non substitué ou substitué, un groupe aralkyloxy non substitué ou substitué, un groupe aryloxy non substitué ou substitué, un groupe aralkyle non substitué ou substitué, un groupe alkylthio, un groupe arylthio non substitué ou substitué ou un groupe $-O-Z-NR^{11'}R^{12'}$, $-NHR^{10'}$, $-NR^{11'}R^{12'}$ ou -OM,

$R^{9'}$ est un groupe formyle, cyano ou le groupe

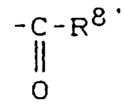

$R^{10'}$ représente de l'hydrogène, un groupe alkyle ou un groupe aryle non substitué ou substitué,

$R^{11'}$ et $R^{12'}$ sont identiques ou différents et représentent chacun un groupe alkyle ou un groupe aryle non substitué ou substitué,

Z est une chaîne alkylique linéaire ou ramifiée et

M représente de l'hydrogène ou un équivalent d'un cation correspondant de métal alcalin, de métal alcalino-terreux ou d'ammonium,

ou bien

$R^{2'}$ et $R^{3'}$ forment conjointement l'un des restes

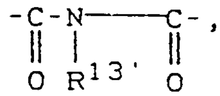 , 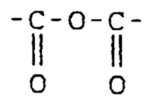 ou 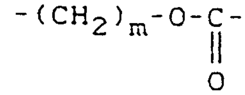 ,

ponté par les positions 6 et 5,

où

$R^{13'}$ représente de l'hydrogène, un groupe alkyle ou un groupe aryle non substitué ou substitué et

m est le nombre 1 ou 2,

ou bien

$R^{4'}$ et $R^{5'}$ forment conjointement une chaîne alkylique de 3 ou 4 atomes de carbone, qui est liée par les positions 4 et 3,

ainsi que de leurs sels d'addition d'acides et de leurs complexes de sels métalliques, excepté les composés acide 2-amino-cyclohex-3-ène-carboxylique et ester éthylique d'acide 2-amino-cyclohex-3-ène-carboxylique,

caractérise en ce que :

A) on ajoute un ester d'acide chloroformique à des derivés d'acide 2-cyclohexène-1-yl-carboxylique de formule

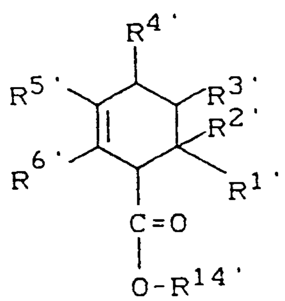

(II)

dans laquelle

$R^{1'}$, $R^{2'}$, $R^{3'}$, $R^{4'}$, $R^{5'}$ et $R^{6'}$ ont la définition indiquée ci-dessus et

$R^{14'}$ représente l'hydrogène, un groupe méthyle ou éthyle,

d'une manière généralement classique, selon Curtius, le cas échéant en présence d'un diluant et en la présence d'une base, à des températures comprises entre -15 °C et +10 °C et on ajoute un azide à ce mélange réactionnel, le cas échéant en présence d'un diluant, à des températures comprises entre -5 °C et +25 °C,

et on hydrolyse l'isocyanate obtenu comme produit intermédiaire de formule (IIa)

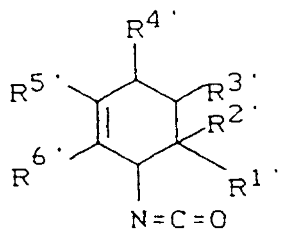

(IIa)

dans laquelle

$R^{1'}$, $R^{2'}$, $R^{3'}$, $R^{4'}$, $R^{5'}$ et $R^{6'}$ ont la définition indiquée ci-dessus,

avec de l'eau, éventuellement en présence d'un acide ou d'une base et on transforme éventuellement les amines ainsi obtenues en sels d'addition d'acides et en complexes de sels métalliques, ou bien

B) on élimine le groupe protégeant la fonction amino des dérivés de 2-cyclohexène de formule (IIb)

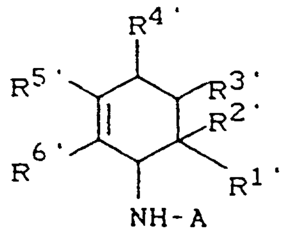

(IIb)

dans laquelle

$R^{1'}$, $R^{2'}$, $R^{3'}$, $R^{4'}$, $R^{5'}$ et $R^{6'}$ ont la définition indiquée ci-dessus et

A représente un groupe protégeant la fonction amino,

d'une manière connue par des procédés classiques, éventuellement en présence d'un solvant ou d'un diluant ou d'un mélange de solvants ou de diluants appropriés, en refroidissant, à la température ambiante ou en chauffant et, si nécessaire, dans un récipient clos, sous pression, en atmosphère de gaz inerte et/ou dans des conditions anhydres et on transforme éventuellement les produits ainsi obtenus en sels d'addition d'acides ou en complexes de sels métalliques.

**10.** Dérivés de 2-cyclohexène de formule (IIb)

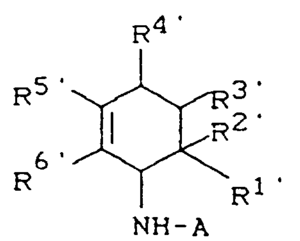

(IIb)

dans laquelle

$R^{1'}$, $R^{2'}$, $R^{3'}$, $R^{4'}$, $R^{5'}$, et $R^{6'}$ ont la définition indiquée dans la revendication 2 et

A est un groupe protégeant la fonction amino, excepté les composés suivants, et leurs énantiomères et isomères : ester méthylique d'acide 6-formyl-5-{[(phénylméthoxy)carbonyl]-amino)-3-cyclohexène-1-carboxylique, ester méthylique d'acide 6-[3-oxo-1-propényl)-5-{[-(phényl-méthoxy)-carbonyl}-amino}-3-cyclohexène-1-carboxylique, ester méthylique d'acide 3-cyclohexène-2-[(trichloracétyl)amino]-1-carboxylique, 2,2,2-trichloro-N-(6-formyl-2-cyclohexène-1-yl)-acétamide, ester éthylique d'acide (6-formyl-5-méthyl-2-cyclohexène-1-yl)-carbamique, ester méthylique d'acide 2-[(éthoxycarbonyl)-amino]-6-méthyl-3-cyclohexène-1-carboxylique, ester méthylique d'acide 2-[-(éthoxycarbonyl)-amino]-5-méthyl-3-cyclohexène-1-carboxylique, ester méthylique d'acide 2-[(éthoxycarbonyl)-amino]-3-cyclohexène-1-carboxylique, ester méthylique d'acide 3-{2-[(éthoxycarbonyl)-amino]-6-méthyl-3-cyclohexène-1-yl}-2-propénoïque, ester phénylméthylique d'acide (6-formyl-5-propyl-2-cyclohexène-1-yl)-carbamique, ester phénylméthylique d'acide (6-formyl-5-méthyl-2-cyclohexène-1-yl)-carbamique, ester méthylique d'acide 2-[(phénoxycarbonyl)-amino]-3-cyclohexène-1-carboxylique, ester éthylique d'acide 3-⟨6-méthyl-2-{[-(phénylméthoxy)-carbonyl]-amino}-3-cyclohexène-1-yl⟩-2-propénoïque, ester phénylméthylique d'acide (6-formyl-5-pentyl-2-cyclohexène-1-yl)-carbamique, {6-carbométhoxy-2-cyclohexène-1-yl-thiocarbamate de phényle, N-(6-carbométhoxy-2-cyclohexène-1-yl)-1-pyrrolidine-carboxamide, ester méthylique, ester tertiobutylique, ester benzylique et ester 2,4,6-triméthylphénylique d'acide 2-[(p-toluènesulfonyl)-amino]-3-cyclohexène-1-carboxylique et ester phénylméthylique d'acide {6-formyl-5-[2-(méthoxyméthoxy)-éthyl}-2-cyclohexène-1-yl}-carbamique.

**11.** Compositions pesticides caractérisées par une teneur en au moins un dérivé de 2-cyclohexène-1-yl-amine de formule (I) suivant la revendication 1 et avec l'utilisation d'agents tensio-actifs.

**12.** Procédé pour combattre des parasites, caractérisé en ce qu'on fait agir des dérivés de 2-cyclohexène-1-yl-amine de formule (I) suivant la revendication 1 sur des parasites et/ou sur leur milieu.

**13.** Procédé de préparation de compositions pesticides, caractérisé en ce qu'on mélange des dérivés de 2-cyclohexène-1-yl-amine de formule (I) suivant la revendication 1 avec des diluants et/ou des agents tensio-actifs.

**Revendications pour l'Etat contractant suivants : ES**

**1.** Utilisation de dérivés de 2-cyclohexène-1-yl-amine de formule (I)

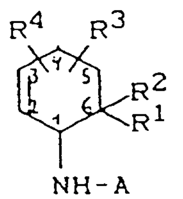

(I)

dans laquelle

$R^1$ représente l'hydrogène, un groupe alkyle ou un halogène,

$R^2$ est un groupe formyle, hydroxyalkyle, cyano, nitro ou représente l'un des restes $-NHR^5$, $-NR^6R^7$,

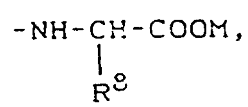

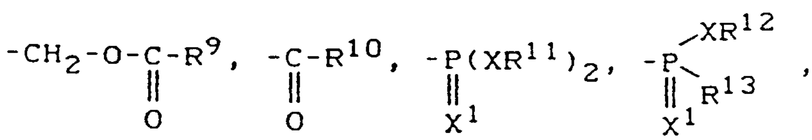

$-S(O)_nR^{14}$ ou $-CH=CH-R^{15}$

$R^3$ et $R^4$ sont identiques ou différents et représentent chacun l'hydrogène, un groupe alkyle, alcényle, alcynyle, alkoxy, alcényloxy, alcynyloxy, un groupe aryle non substitué ou substitué, un groupe aralkyle non substitué ou substitué, un groupe hétéroaryle non substitué ou substitué, un groupe hétérocyclylalkyle non substitué ou substitué, un groupe alkoxyalkyloxy, un halogène ou l'un des restes $-NH-R^5$, $-NR^6R^7$ ou $-S(O)_n-R^{14}$,

ou bien

$R^2$ et $R^3$ représentent ensemble l'un des restes

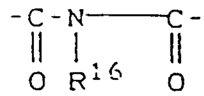 , 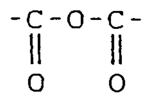 ou 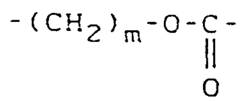 ,

ponté par les positions 6 et 5,

ou bien

$R^3$ et $R^4$ forment conjointement une chaîne alkylique de 3 ou 4 atomes de carbone, qui est liée par les positions 4 et 3,

$R^5$ est l'hydrogène, un groupe alkyle ou un groupe aryle non substitué ou substitué,

$R^6$ est un groupe alkyle ou un groupe aryle non substitué ou substitué,

$R^7$ est un groupe alkyle ou un groupe aryle non substitué ou substitué,

$R^8$ est l'hydrogène, un groupe alkyle, un groupe aryle non substitué ou substitué, ou un groupe aralkyle non substitué ou substitué,

$R^9$ est un groupe alkyle ou alkoxy,

$R^{10}$ est un groupe hydroxy, hydroxyalkyloxy, halogénalkyloxy, alkoxy, alkoxyalkyloxy, un groupe cycloalkyloxy non substitué ou substitué ou un groupe aralkyloxy non substitué ou substitué, un groupe aryloxy non substitué ou substitué, un groupe aralkyle non substitué ou substitué, un groupe alkylthio, un groupe arylthio non substitué ou substitué ou un groupe -OM, $-NHR^5$, $-NR^6R^7$ ou $-O-Z-NR^5R^6$,

$R^{11}$ est de l'hydrogène ou un groupe alkyle,

$R^{12}$ est de l'hydrogène ou un groupe alkyle,

$R^{13}$ est un groupe alkyle,

$R^{14}$ est un groupe alkyle, un groupe alkoxy, un groupe aryle non substitué ou substitué ou le groupe -OM,

$R^{15}$ est un groupe formyle, cyano ou le groupement

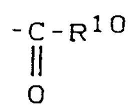

$R^{16}$ est de l'hydrogène, un groupe alkyle ou un groupe aryle non substitué ou substitué,

M est de l'hydrogène ou représente un équivalent d'un cation correspondant de métal alcalin, de métal alcalino-terreux ou d'ammonium,

n représente le nombre 0, 1 ou 2,

X et $X^1$ sont identiques ou différents et représentent l'oxygène ou le soufre,

m est le nombre 1 ou 2,

A est de l'hydrogène ou un groupe protégeant la fonction amino et

Z est une chaîne alkylique linéaire ou ramifiée,

ainsi que leurs sels d'addition d'acides et leurs complexes de sels métalliques, pour combattre des parasites.

**2.** Procédé de production de dérivés substitués de 2-cyclo-hexène-1-yl-amine de formule (Ia)

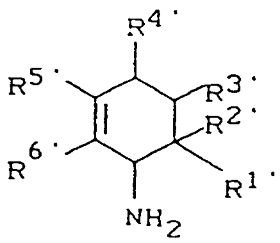

(Ia)

dans laquelle

$R^{1'}$ représente de l'hydrogène, un groupe alkyle ou un halogène,

$R^{2'}$ est un groupe formyle, hydroxyalkyle, cyano, nitro ou l'un des restes

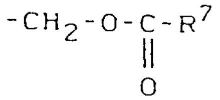 , 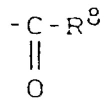

ou $-CH=CH-R^{9'}$

$R^{3'}$, $R^{4'}$, $R^{5'}$, et $R^{6'}$ sont identiques ou différents et représentent chacun de l'hydrogène, un groupe alkyle, alcényle, alcynyle, alkoxy, alcényloxy, alcynyloxy, un groupe aryle non substitué ou substitué, un groupe aralkyle non substitué ou substitué, un groupe hétéroaryle non substitué ou substitué, un groupe hétérocyclylalkyle non substitué ou substitué, un groupe alkoxyalkyloxy ou un halogène, deux au moins des restes $R^{3'}$, $R^{4'}$, $R^{5'}$ ou $R^{6'}$ représentent de l'hydrogène,

$R^{7'}$ est un groupe alkyle ou alkoxy,

$R^{8'}$ est un groupe hydroxy, hydroxyalkyloxy, halogénalkyloxy, alkoxy, alkoxyalkyloxy, un groupe cycloalkyloxy non substitué ou substitué, un groupe aralkyloxy non substitué ou substitué, un groupe aryloxy non substitué ou substitué, un groupe aralkyle non substitué ou substitué, un groupe alkylthio, un groupe arylthio non substitué ou substitué ou un groupe $-O-Z-NR^{11'}R^{12'}$, $-NHR^{10'}$, $-NR^{11'}R^{12'}$, ou -OM,

$R^{9'}$ est un groupe formyle, cyano ou le groupe

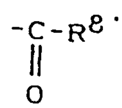

$R^{10'}$ représente de l'hydrogène, un groupe alkyle ou un groupe aryle non substitué ou substitué,

$R^{11'}$ et $R^{12'}$ sont identiques ou différents et représentent chacun un groupe alkyle ou un groupe aryle non substitué ou substitué,

Z est une chaîne alkylique linéaire ou ramifiée et

M représente de l'hydrogène ou un équivalent d'un cation correspondant de métal alcalin, de métal alcalino-terreux ou d'ammonium,

ou bien

$R^{2'}$ et $R^{3'}$ forment conjointement l'un des restes

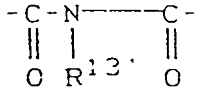 , 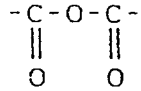 ou 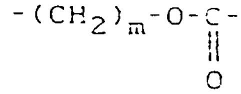 ,

ponté par les positions 6 et 5,

où

$R^{13'}$ représente de l'hydrogène, un groupe alkyle ou un groupe aryle non substitué ou substitué et

m est le nombre 1 ou 2,

ou bien

$R^{4'}$ et $R^{5'}$ forment conjointement une chaîne alkylique de 3 ou 4 atomes de carbone, qui est liée par les positions 4 et 3,

ainsi que de leurs sels d'addition d'acides et de leurs complexes de sels métalliques, excepté les composés acide 2-amino-cyclohex-3-ène-carboxylique et ester éthylique d'acide 2-aminocyclohex-3-ène-carboxylique,

caractérisé en ce que :

A) on ajoute un ester d'acide chloroformique à des dérivés d'acide 2-cyclohexène-1-yl-carboxylique de formule

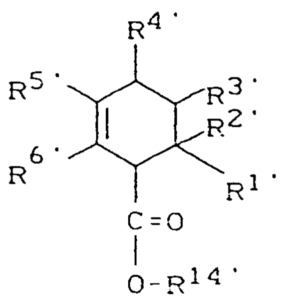

(II)

dans laquelle

$R^{1'}$, $R^{2'}$, $R^{3'}$, $R^{4'}$, $R^{5'}$, et $R^{6'}$ ont la définition indiquée ci-dessus et

$R^{14'}$ représente l'hydrogène, un groupe méthyle ou éthyle,

d'une manière généralement classique, selon Curtius, le cas échéant en présence d'un diluant et en la présence d'une base, à des températures comprises entre -15 °C et +10 °C et on ajoute un azide à ce mélange réactionnel, le cas échéant en présence d'un diluant, à des températures comprises entre -5 °C et +25 °C,

et on hydrolyse l'isocyanate obtenu comme produit intermédiaire de formule (IIa)

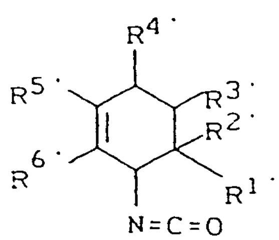

(IIa)

dans laquelle

$R^{1'}$, $R^{2'}$, $R^{3'}$, $R^{4'}$, $R^{5'}$ et $R^{6'}$ ont la définition indiquée ci-dessus,

avec de l'eau, éventuellement en présence d'un acide ou d'une base et on transforme éventuellement les amines ainsi obtenues en sels d'addition d'acides et en complexes de sels métalliques,

ou bien

B) on élimine le groupe protégeant la fonction amino des dérivés de 2-cyclohexène de formule (IIb)

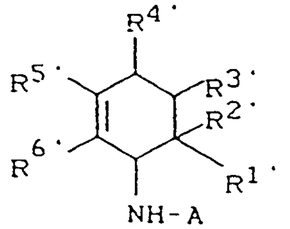

(IIb)

dans laquelle

$R^{1'}$, $R^{2'}$, $R^{3'}$, $R^{4'}$, $R^{5'}$, et $R^{6'}$ ont la définition indiquée ci-dessus et

A représente un groupe protégeant la fonction amino,

d'une manière connue par des procédés classiques, éventuellement en présence d'un solvant ou d'un diluant ou d'un mélange de solvants ou de diluants appropriés, en refroidissant, à la température ambiante ou en chauffant et, si nécessaire, dans un récipient clos, sous pression, en atmosphère de gaz inerte et/ou dans des conditions anhydres et on transforme éventuellement les produits ainsi obtenus en sels d'addition d'acides ou en complexes de sels métalliques.

**3.** Procédé suivant la revendication 2, dans lequel

$R^{1'}$ représente de l'hydrogène, un groupe alkyle à chaîne droite ou ramifiée ayant 1 à 6 atomes de carbone ou du fluor, du chlore ou du brome,

$R^{2'}$ est un groupe formyle, un groupe hydroxyalkyle à chaîne droite ou ramifiée ayant 1 à 8 atomes de carbone dans la partie alkyle, un groupe cyano, nitro ou l'un des restes

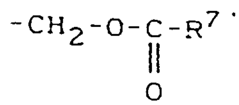 , 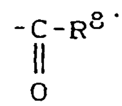

ou $-CH=CH-R^{9'}$

$R^{3'}$, $R^{4'}$, $R^{5'}$ et $R^{6'}$ sont identiques ou différents et représentent chacun de l'hydrogène, chacun un groupe alkyle ou alkoxy à chaîne droite ou ramifiée ayant 1 à 8 atomes de carbone, chacun un groupe alcényle, alcynyle, alcényloxy ou alcynyloxy à chaîne droite ou ramifiée ayant chacun 2 à 8 atomes de carbone, alkoxyalkyloxy ayant chacun 1 à 8 atomes de carbone dans les parties alkyle individuelles, chacun un groupe aryle ou aralkyle non substitué dans la partie aryle ou portant 1 à 5 substituants identiques ou différents et ayant chacun 6 à 10 atomes de carbone dans la partie aryle et le cas échéant 1 à 4 atomes de carbone dans la partie alkyle, en considérant comme substituants de la partie aryle : un halogène, un radical nitro, cyano, amino, alkyle en $C_1$ à $C_4$, alkoxy en $C_1$ à $C_4$, alkylthio en $C_1$ à $C_4$, halogénalkyle en $C_1$ à $C_4$, halogénalkoxy en $C_1$ à $C_4$, halogénalkylthio en $C_1$ à $C_4$ avec chacun 1 à 9 atomes d'halogènes identiques ou différents et di(alkyle en $C_1$ à $C_4$)amino,

en outre, un groupement hétérocyclique pentagonal ou hexagonal non substitué ou portant un à trois substituants identiques ou différents et lié le cas échéant par un groupe méthylène, de la série furyle, thiényle, pyrrolyle, pyrazolyle, imidazolyle, 1,2,3- ou 1,2,4-triazolyle, oxazolyle, isoxazolyle, 1,2,4- ou 1,3,4-oxa-diazolyle, thiazolyle, isothiazolyle, 1,2,3-, 1,2,4-, 1,2,5- ou 1,3,4-thiadiazolyle, pyridyle, pyridazinyle, pyrimidinyle et pyrazinyle, en considérant dans chaque cas comme substituants de l'hétérocycle : un halogène, un radical nitro, cyano, amino, alkyle en $C_1$ à $C_4$, alkoxy en $C_1$ à $C_4$ ou alkylthio en $C_1$ à $C_4$, halogénalkyle en $C_1$ à $C_4$, halogénalkoxy en $C_1$ à $C_4$, halogénalkylthio en $C_1$ à $C_4$ ayant dans chaque cas 1 à 9 atomes d'halogènes

identiques ou différents, et di(alkyle en $C_1$ à $C_4$)amino, en outre le fluor, le chlore ou le brome,

deux au moins des restes $R^{3'}$, $R^{4'}$, $R^{5'}$ ou $R^{6'}$ représentant de l'hydrogène,

$R^{7'}$ est un groupe alkyle ou alkoxy ayant 1 à 6 atomes de carbone, chacun à chaîne droite ou ramifiée,

$R^{8'}$ est un groupe hydroxy, un groupe hydroxyalkyloxy linéaire ou ramifié ayant 1 à 8 atomes de carbone, un groupe halogénalkyloxy linéaire ou ramifié ayant 1 à 8 atomes de carbone et 1 à 17 atomes d'halogénes identiques ou différents, un groupe cycloalkyloxy non substitué ou portant un à plusieurs substituants halogéno identiques ou différents, et ayant 3 à 6 atomes de carbone, un groupe alkoxy ou alkylthio ayant 1 à 6 atomes de carbone, chacun à chaîne droite ou ramifiée, un groupe alkoxyalkyloxy à chaîne droite ou ramifiée ayant chacun 1 à 6 atomes de carbone dans la partie alkoxy ou alkyle, un groupe aryloxy, arylthio, aralkyle ou aralkyloxy non substitué dans la partie aryle ou portant un à cinq substituants identiques ou différents et ayant chacun 6 à 10 atomes de carbone dans la partie aryle et le cas échéant 1 à 8 atomes de carbone dans la partie alkyle, en considérant comme substituants de la partie aryle les substituants de cette partie indiqués ci-dessus, ou un groupe $-O-Z-NR^{11'}R^{12'}$, $-NHR^{10'}$, $-NR^{11'}R^{12'}$, ou -OM,

$R^{9'}$ est un groupe formyle, cyano ou le groupe

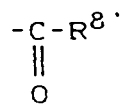

$R^{10'}$ représente l'hydrogène, un groupe alkyle à chaîne droite ou ramifiée ayant 1 à 6 atomes de carbone ou un groupe aryle de 6 à 10 atomes de carbone non substitué ou portant un à cinq substituants identiques ou différents, en considérant comme substituants du groupe aryle les substituants de ce groupe indiqués ci-dessus,

$R^{11'}$ est un groupe alkyle à chaîne droite ou ramifiée ayant 1 à 6 atomes de carbone ou un groupe aryle de 6 à 10 atomes de carbone non substitué ou portant un à cinq substituants identiques ou différents, en considérant comme substituants du groupe aryle les substituants de ce groupe indiqués ci-dessus,

$R^{12'}$ est un groupe alkyle à chaîne droite ou ramifiée ayant 1 à 6 atomes de carbone ou un groupe aryle de 6 à 10 atomes de carbone non substitué ou portant un à cinq substituants identiques ou différents, en considérant comme substituants du groupe aryle les substituants de ce groupe indiqués ci-dessus,

M représente de l'hydrogène ou un équivalent d'un cation correspondant de métal alcalin, de métal alcalino-terreux ou d'ammonium et

Z est une chaîne alkylique linéaire ou ramifiée ayant 1 à 8 atomes de carbone,

ou bien

$R^{2'}$ et $R^{3'}$ forment conjointement l'un des restes

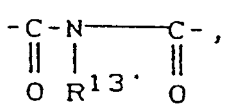 , 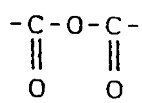 ou 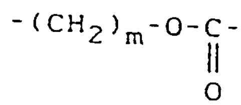 ,

ponté par les positions 6 et 5,

où

$R^{13'}$ représente de l'hydrogène, un groupe alkyle à chaîne droite ou ramifiée ayant 1 à 6 atomes de carbone ou un groupe aryle de 6 à 10 atomes de carbone non substitué ou portant un à cinq substituants identiques ou différents, en considérant comme substituants du groupe aryle les substituants de ce groupe indiqués ci-dessus et

m est le nombre 1 ou 2,

ou bien

$R^{4'}$ et $R^{5'}$ forment ensemble une chaîne alkylique de 3 ou 4 atomes de carbone, qui est liée par les positions 4 et 3,

ainsi que de leurs sels d'addition d'acides et de leurs complexes de sels métalliques.

**4.** Procédé suivant la revendication 2, dans lequel

$R^{1'}$ représente de l'hydrogène, un groupe alkyle à chaîne droite ou ramifiée ayant 1 à 4 atomes de carbone ,ou du fluor, du chlore ou du brome,

$R^{2'}$ est un groupe formyle, un groupe hydroxyalkyle à chaîne droite ou ramifiée ayant 1 à 4 atomes de carbone dans la partie alkyle, un groupe cyano, nitro ou l'un des restes

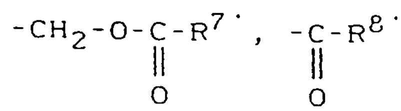

ou $-CH=CH-R^{9'}$

$R^{3'}$, $R^{4'}$, $R^{5'}$ et $R^{6'}$ sont identiques ou différents et représentent chacun de l'hydrogène, chacun un groupe alkyle ou alkoxy de 1 à 6 atomes de carbone à chaîne droite ou ramifiée, chacun un groupe alcényle, alcynyle, alcényloxy ou alcynyloxy linéaire ou ramifié ayant chacun 2 à 6 atomes de carbone, un groupe alkoxyalkyloxy ayant 1 à 6 atomes de carbone dans chacune des parties alkyle individuelles, un groupe phényle ou phénylalkyle chacun non substitué dans la partie phényle ou portant un à trois substituants identiques ou différents, avec le cas échéant 1 ou 2 atomes de carbone dans la partie alkyle, en considérant comme substituants du groupe phényle : le fluor, le chlore, le brome, un radical nitro, cyano, amino, alkyle en $C_1$ ou $C_2$, alkoxy en $C_1$ à $C_3$, alkylthio en $C_1$ ou $C_2$, halogénalkyle en $C_1$ ou $C_2$, halogénalkoxy en $C_1$ ou $C_2$, halogénalkylthio en $C_1$ ou $C_2$ avec chacun 1 à 5 atomes de fluor et/ou de chlore identiques ou différents et di(alkyle en $C_1$ ou $C_2$)amino, en outre un groupement hétérocyclique pentagonal ou hexagonal non substitué ou portant un à trois substituants identiques ou différents et le cas échéant lié par un groupe méthylène, de la série furyle, thiényle, pyrrolyle, pyrazolyle, imidazolyle, 1,2,3- ou 1,2,4-triazolyle, oxazolyle, isoxazolyle, 1,2,4- ou 1,3,4-oxadiazolyle, thiazolyle, isothiazolyle, 1,2,3-, 1,2,4-, 1,2,5- ou 1,3,4-thiadiazolyle, pyridyle, pyridazinyle, pyrimidinyle et pyrazinyle, en considérant dans chaque cas comme substituants de l'hétérocycle : du fluor, du chlore, du brome, un radical nitro, cyano, amino, alkyle en $C_1$ ou $C_2$, alkoxy en $C_1$ ou $C_2$ ou alkylthio en $C_1$ ou $C_2$, halogénalkyle en $C_1$ ou $C_2$, halogénalkoxy en $C_1$ ou $C_2$, halogénalkylthio en $C_1$ ou $C_2$ avec dans chaque cas 1 à 5 atomes de fluor et/ou de chlore identiques ou différents, et di(alkyle en $C_1$ ou $C_2$)amino,
en outre le fluor, le chlore ou le brome,
deux au moins des restes $R^{3'}$, $R^{4'}$, $R^{5'}$ et $R^{6'}$ représentant de l'hydrogène,

$R^{7'}$ est un groupe alkyle ou un groupe alkoxy, chacun linéaire ou ramifié avec 1 à 4 atomes de carbone,

$R^{8'}$ est un groupe hydroxy, un groupe hydroxyalkyloxy à chaîne droite ou ramifiée ayant 1 à 6 atomes de carbone, un groupe halogénalkyloxy à chaîne droite ou ramifiée ayant 1 à 6 atomes de carbone et 1 à 13 atomes d'halogènes identiques ou différents, un groupe cycloalkyloxy de 3 à 6 atomes de carbone non substitué ou portant un à trois substituants fluoro, chloro, bromo identiques ou différents, un groupe alkoxy ou alkylthio, chacun à chaîne droite ou ramifiée ayant 1 à 4 atomes de carbone, un groupe alkoxyalkyloxy à chaîne droite ou ramifiée ayant 1 à 4 atomes de carbone dans chaque partie alkoxy ou alkyle, un groupe phényloxy, phénylthio, phénylalkyle ou phénylalkyloxy non substitué chacun dans la partie alkyle ou portant chacun un à trois

substituants identiques ou différents, avec chacun le cas échéant 1 à 6 atomes de carbone dans la partie alkyle, en considérant comme substituants du groupe phényle les substituants de ce groupe indiqués ci-dessus, ou bien un groupe -O-Z-$NR^{11'}R^{12'}$, -$NHR^{10'}$, -$NR^{11'}R^{12'}$, ou -OM,

$R^{9'}$ est un groupe formyle, cyano ou le groupe

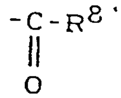

$R^{10'}$ représente de l'hydrogène, un groupe alkyle à chaîne droite ou ramifiée ayant 1 à 4 atomes de carbone ou un groupe phényle non substitué ou portant un à trois substituants identiques ou différents, en considérant comme substituants du groupe phényle les substituants de ce groupe indiqués ci-dessus,

$R^{11'}$ est un groupe alkyle à chaîne droite ou ramifiée ayant 1 à 4 atomes de carbone ou un groupe phényle non substitué ou portant un à trois substituants identiques ou différents, en considérant comme substituants du groupe phényle les substituants de ce groupe indiqués ci-dessus,

$R^{12'}$ est un groupe alkyle à chaîne droite ou ramifiée ayant 1 à 4 atomes de carbone ou un groupe phényle non substitué ou portant un à trois substituants identiques ou différents, en considérant comme substituants du groupe phényle les substituants de ce groupe indiqués ci-dessus,

M représente de l'hydrogène ou un équivalent d'un cation correspondant de métal alcalin, de métal alcalino-terreux ou d'ammonium et

Z est une chaîne alkylique linéaire ou ramifiée ayant 1 à 6 atomes de carbone,

ou bien

$R^{2'}$ et $R^{3'}$ forment ensemble l'un des restes

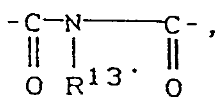 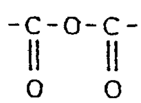 ou 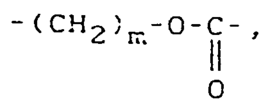

ponté par les positions 6 et 5,

$R^{13'}$ représente de l'hydrogène, un groupe alkyle à chaîne droite ou ramifiée ayant 1 à 6 atomes de carbone et un groupe phényle non substitué ou portant un à trois substituants identiques ou différents, en considérant comme substituants du groupe phényle les substituants de ce groupe indiqués ci-dessus et

m représente le nombre 1 ou 2,

ou bien

$R^{4'}$ et $R^{5'}$ forment conjointement une chaîne alkylique de 3 ou 4 atomes de carbone, qui est liée par les positions 4 et 3,

ainsi que leurs sels d'addition d'acides et leurs complexes de sels métalliques.

**5.** Procédé suivant la revendication 2, dans lequel le composé de formule (Ia) est le chlorhydrate du cis-diastéréoisomère de l'acide 2-amino-cyclohex-3-ène-carboxylique.

**6.** Procédé suivant la revendication 2, dans lequel le composé de formule (Ia) comprend les chlorhydrates des énantiomètres de l'acide 2-amino-cyclohex-3-ène carboxylique ayant un point de fusion de 210 - 213,5 °C et de 208 - 210 °C.

**7.** Procédé suivant la revendication 2, dans lequel le composé de formule (Ia) est l'acide 2-amino-cyclohex-3-ène carboxylique ayant un point de fusion de 98 °C.

8. Procédé suivant la revendication 2, dans lequel le composé de formule (Ia) est le chlorhydrate de l'isomère de l'ester éthylique d'acide 2-amino-cyclohex-3-ène-carboxylique ayant un point de fusion de 139 - 148 °C.

9. Compositions pesticides caractérisées par une teneur en au moins un dérivé de 2-cyclohexène-1-yl-amine de formule (I) suivant la revendication 1 et avec utilisation d'agents tensio-actifs.

10. Procédé pour combattre des parasites, caractérisé en ce qu'on fait agir des dérivés de 2-cyclohexène-1-yl-amine de formule (I) suivant la revendication 1 sur les parasites et/ou sur leur milieu.

11. Procédé de préparation de compositions pesticides, caractérisé en ce qu'on mélange des dérivés de 2-cyclohexène-1-yl-amine de formule (I) suivant la revendication 1 avec des diluants et/ou des agents tensio-actifs.

12. Procédé de préparation de dérivés de 2-cyclohexène de formule (IIb)

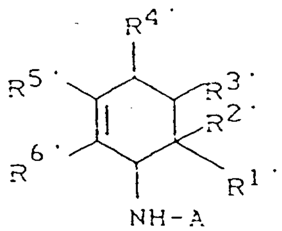

(IIb)

dans laquelle

| | |
|---|---|
| $R^{1'}$, $R^{2'}$, $R^{3'}$, $R^{4'}$, $R^{5'}$ et $R^{6'}$ | ont la définition indiquée dans la revendication 2 et |
| A | est un groupe protégeant la fonction amino, excepté les composés suivants, et leurs énantiomères et isomères : ester méthylique d'acide 6-formyl-5-{[(phénylméthoxy)carbonyl]-amino}-3-cyclohexène-1-carboxylique, ester méthylique d'acide 6-[3-oxo-1-propényl)-5-{[-(phénylméthoxy)-carbonyl}-amino}-3-cyclohexène-1-carboxylique, ester méthylique d'acide 3-cyclohexène-2-[(trichloracétyl)amino]-1-carboxylique, 2,2,2-trichloro-N-(6-formyl-2-cyclohexène-1-yl)-acétamide, ester éthylique d'acide (6-formyl-5-méthyl-2-cyclohexène-1-yl)-carbamique, ester méthylique d'acide 2-[(éthoxycarbonyl)-amino]-6-méthyl-3-cyclohexène-1-carboxylique, ester méthylique d'acide 2-[-(éthoxycarbonyl)-amino]-5-méthyl-3-cyclohexène-1-carboxylique, ester méthylique d'acide 2-[(éthoxycarbonyl)-amino]-3-cyclohexène-1-carboxylique, ester méthylique d'acide 3-{2-[(éthoxycarbonyl)-amino]-6-méthyl-3-cyclohexène-1-yl}-2-propénoïque, ester phénylméthylique d'acide (6-formyl-5-propyl-2-cyclohexène-1-yl)-carbamique, ester phénylméthylique d'acide (6-formyl-5-méthyl-2-cyclohexène-1-yl)-carbamique, ester méthylique d'acide 2-[(phénoxycarbonyl)-amino]-3-cyclohexène-1-carboxylique, ester éthylique d'acide 3-(6-méthyl-2-{[-(phénylméthoxy)-carbonyl]-amino}-3-cyclohexène-1-yl)-2-propénoïque, ester phénylméthylique d'acide (6-formyl-5-pentyl-2-cyclohexène-1-yl)-carbamique, {6-carbométhoxy-2-cyclohexène-1-yl-thiocarbamate de phényle, N-(6-carbométhoxy-2-cyclohexène-1-yl)-1-pyrrolidinecarboxamide, ester méthylique, ester tertiobutylique, ester benzylique et ester 2,4,6-triméthylphénylique d'acide 2-[(p-toluènesulfonyl)-amino]-3-cyclohexène-1-carboxylique et ester phénylméthylique d'acide {6-formyl-5-[2-(méthoxyméthoxy)-éthyl}-2-cyclohexène-1-yl}- carbamique, |

caractérisé en ce que des diénophiles de formule (III)

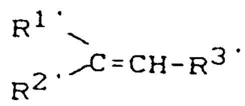

(III)

dans laquelle

$R^{1'}$, $R^{2'}$ et $R^{3'}$ ont la définition indiquée ci-dessus

(B/a) sont cyclisés avec des dérivés de N-acyl-1-amino-1,3-butadiène de formule (IVa)

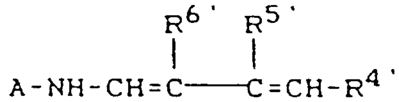

(IVa)

dans laquelle

$R^{4'}$, $R^{5'}$, $R^{6'}$ et A ont la définition indiquée ci-dessus,

le cas échéant en présence d'un diluant, en la présence éventuelle d'un catalyseur, en la présence éventuelle d'un gaz inerte et le cas échéant sous pression,

ou bien

(B/b) sont cyclisés avec des butadiènes substitués de formule (IVb)

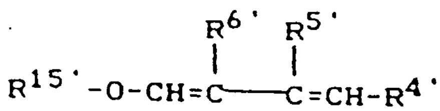

(IVb)

dans laquelle

$R^{4'}$, $R^{5'}$ et $R^{6'}$ ont la définition indiquée ci-dessus et

$R^{15'}$ est le reste acétyle ou triméthylsilyle,

tout d'abord dans une première étape, éventuellement en présence d'un diluant, en la présence éventuelle d'un catalyseur, le cas échéant en présence d'un gaz inerte et, éventuellement, sous pression,

et les dérivés de 2-cyclohexène ainsi obtenus de formule (IIc)

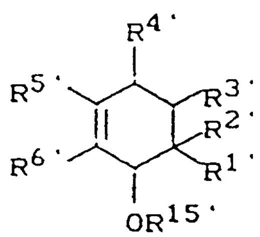

(IIc)

dans laquelle

$R^{1'}$, $R^{2'}$, $R^{3'}$, $R^{4'}$, $R^{5'}$ et $R^{6'}$ ont la définition indiquée ci-dessus et

$R^{15'}$ est un reste acétyle ou triméthylsilyle

sont amenés à réagir dans une seconde étape avec la 4,4'-diméthoxybenzhydrylamine (DMB) de formule (V)

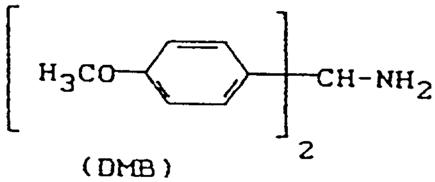

(V)

(DMB)

éventuellement en présence d'un diluant, à des températures allant de 0°C à la température d'ébullition du diluant utilisé dans chaque cas et en présence d'un catalyseur,

ou bien

(B/c) les isocyanates obtenus comme produits intermédiaires dans le procédé (A), de formule (IIa)

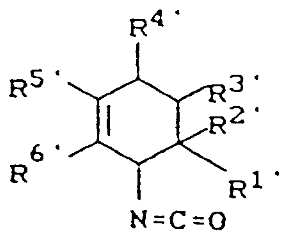

(IIa)

dans laquelle

$R^{1'}$, $R^{2'}$, $R^{3'}$, $R^{4'}$, $R^{5'}$ et $R^{6'}$ ont la définition indiquée ci-dessus,

sont amenés à réagir avec des alcools de formule (VII)

$R^{16'}$-OH (VII)

dans laquelle

$R^{16'}$ est un groupe alkyle, alcényle, alcynyle ou alkoxyalkyle, chacun à chaîne droite ou ramifiée, éventuellement substitué par un halogène, ayant 1 à 12 atomes de carbone (de préférence 1 à 6 atomes de carbone), un groupe phényle ou un groupe benzyle non substitué ou substitué,

ou bien

(B/d) on obtient les dérivés de 2-cyclohexène substitués de formule (IIb) en réduisant les dérivés de 2-cyclohexène obtenus par le procédé (B/a), (B/b) ou (B/c), de formule (IId)

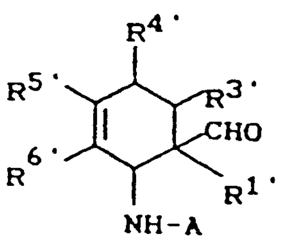

(IId)

dans laquelle

$R^{1'}$, $R^{3'}$, $R^{4'}$, $R^{5'}$, $R^{6'}$ et A ont la définition indiquée ci-dessus,

à des températures de 0 à 20°C et on transforme les 2-cyclohexène-1-yl-amino-alcools ainsi obtenus de formule (IIe)

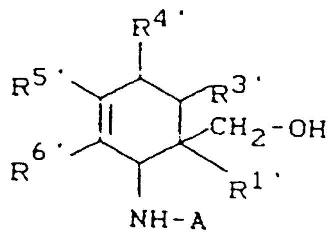

(IIe)

dans laquelle

$R^{1'}$, $R^{3'}$, $R^{4'}$, $R^{5'}$, $R^{6'}$ et A ont la définition indiquée ci-dessus

par d'autres réactions portant sur le groupe hydroxy en esters et éthers ou avec des chlorures de carbamoyle en dérivés acyliques ou carbamoyliques des composés de formule (IIb), ou bien

(B/e) on obtient les dérivés substitués de 2-cyclohexène de formule (IIb) en faisant réagir les dérivés de 2-cyclohexène obtenus par le procédé (B/a), (B/b) ou (B/c), de formule (IId)

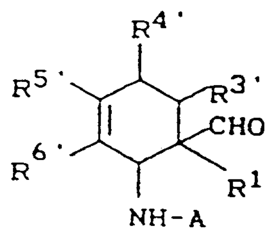

(IId)

dans laquelle

$R^{1'}$, $R^{3'}$, $R^{4'}$, $R^{5'}$, $R^{6'}$ et A ont la définition indiquée ci-dessus,

avec des dérivés d'acides alcane-phosphoniques de formule (VIII)

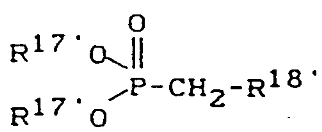

(VIII)

dans laquelle

$R^{17'}$ est un groupe méthyle ou éthyle et

$R^{18'}$ est un groupe cyano ou un groupe alkoxycarbonyle,

le cas échéant en présence d'un diluant, en la présence éventuelle d'une base et, le cas échéant, en présence d'un gaz inerte.